(19) Europäisches Patentamt
European Patent Office
Office européen des brevets

(11) **EP 4 782 543 A1**

(12) **EUROPEAN PATENT APPLICATION**
published in accordance with Art. 153(4) EPC

(43) Date of publication:
**29.07.2026 Bulletin 2026/31**

(21) Application number: **24867336.0**

(22) Date of filing: **10.09.2024**

(51) International Patent Classification (IPC):
*C12N 15/74* (2006.01)   *C12N 15/61* (2006.01)
*C12N 15/113* (2010.01)   *C12N 1/21* (2006.01)
*A61K 48/00* (2006.01)   *A61K 35/74* (2015.01)
*A61P 35/00* (2006.01)

(52) Cooperative Patent Classification (CPC):
**A61K 35/74; A61K 48/00; A61P 35/00; C12N 9/90;
C12N 15/113; C12N 15/74;** C12R 2001/42

(86) International application number:
**PCT/CN2024/117998**

(87) International publication number:
**WO 2025/060924 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.09.2023   CN 202311210601**

(71) Applicant: **Shaoxing Salvectors Biotechnology
Ltd.
Shaoxing, Zhejiang 312080 (CN)**

(72) Inventor: **YU, Bin
Hong Kong (HK)**

(74) Representative: **Gille Hrabal
Partnerschaftsgesellschaft mbB
Patentanwälte
Brucknerstraße 20
40593 Düsseldorf (DE)**

Remarks:
The complete document including Reference
Table(s) and the Sequence Listing(s) can be
downloaded from the EPO website

(54) **HYPOXIA-SPECIFIC GENE EXPRESSION CASSETTE, BACTERIA CONTAINING SAME, AND USE**

(57)   Provided are a hypoxia-specific gene expression cassette, Gram-negative bacteria containing the expression cassette, and a use of the Gram-negative bacteria in preparation of a drug for treating tumors and in treatment of tumors. By reasonably designing a forward hypoxia promoter and a reverse hyperoxia promoter, and coordinating with an appropriate survival-essential gene, a more efficient and safer hypoxia-specific gene expression cassette is obtained. The Gram-negative bacteria modified by the hypoxia-specific gene expression cassette obtained by design have a low overall mutation rate, and in a mouse in-vivo tumor model, more bacteria are distributed in a tumor and have a higher removal speed in normal organs.

Figure 4

## Description

This application claims the priority of a prior application filed with the China National Intellectual Property Administration on 19 September 2023, Chinese patent application No. 2023112106010, entitled "Hypoxia-Specific Gene Expression Cassette, Bacteria Containing Same, and Use". The entire content of this prior application is incorporated herein by reference.

Technical Field

This invention relates to hypoxia-specific gene expression cassette, bacteria containing same, and use, and belongs to the field of genetic engineering and biological therapy.

Background Art

Among all malignant tumors, solid tumors account for approximately 90%, such as sarcoma, melanoma, breast cancer, lung cancer, colon cancer, prostate cancer, etc.

The tumor microenvironment of solid tumors has common characteristics, including abnormal tumor vasculature, excessive connective tissue, immunosuppression, acidic environment and hypoxic regions. Moreover, the aberrant microenvironment of solid tumors serves as a natural barrier that hinders the penetration of conventional therapeutic agents, making it difficult for chemotherapeutic drugs and antibodybased therapeutics to diffuse within the tumor microenvironment. Additionally, the lack of oxygen free radicals makes tumors resistant to chemotherapy and radiotherapy.

Due to the hypoxic environment within solid tumors, facultative anaerobic bacteria and obligate anaerobic bacteria can invade tumors and inhibit their growth, and therefore can be used as therapeutic agents or carriers with great potential.

As early as 1868, the German physician W. Busch first reported that, in some tumor patients who were simultaneously infected with bacteria (*Streptococcus pneumoniae*), tumor growth in the body was inhibited and even eliminated completely. In the subsequent thirty years, the American physician Coley and another German physician, Fehleisen, respectively reported the phenomenon that bacterial infection could suppress tumors. These early studies were controversial because their results were difficult to reproduce and the virulence of the bacteria was difficult to control. However, subsequent rigorous animal experiments demonstrated that bacterial infection can indeed reduce tumor size, and that the host immune system is also activated during treatment. In 1975, Carswell first reported that endotoxin (lipopolysaccharide) of Gram-negative bacteria could stimulate the immune system to release tumor necrosis factor TNF-$\alpha$ and could lead to tumor cell death. In addition, some bacterial vaccines have also been shown to stimulate the immune system to thereby treat tumors. Among them, Bacillus Calmette-Guérin (BCG) is one of the earliest biological preparations used in clinical tumor therapy. BCG is a live attenuated bacterial suspension prepared by serially passaging highly virulent *Mycobacterium bovis* for 230 generations, and is originally intended for the prevention of tuberculosis. Extensive experiments and clinical practices have confirmed that BCG is one of the most effective means for treating bladder cancer.

In recent years, with the rapid development of molecular biology and genetic engineering technologies, it has been found in the research that some facultative or obligate anaerobic bacteria can target, colonize and proliferate within solid tumors and induce tumor regression, such as obligate anaerobic *Clostridium* and probiotic *Bifidobacterium*, etc. Among them, the Gram-negative facultative anaerobe *Salmonella enterica* has the broadest application prospects.

Studies have shown that, after attenuation of *Salmonella typhimurium* using different methods, its colonization capacity in tumor tissues can reach 1,000-10,000 times than that in normal tissues. Methods for attenuating the strains include:

1. *aroA* gene-deficient strain SL7207. The *aroA* mutant cannot synthesize para-aminobenzoic acid (PABA) and aromatic amino acids by itself, and the low PABA levels in the host environment thereby reduces its toxicity *in vivo*.

2. *purI* and *msbB* genes-deficient strain VNP20009. Mutation of *purI* prevents the synthesis of adenosine monophosphate, thereby reducing virulence *in vivo*; mutation of *msbB* prevents the synthesis of lipid A in the lipopolysaccharide component of the cell wall, thereby avoiding bacterial septicemia caused by excessive secretion of TNF-$\alpha$. In early animal experiments, this strain was able to inhibit tumor growth, and Phase I clinical trials demonstrated its safety when administered intravenously as live bacteria.

3. Leucine and arginine auxotrophic strains A1 and A1-R. A1, obtained by Zhao et al. through chemical mutagenesis and screening, can inhibit the growth of tumor cells both *in vivo* and *in vitro*. To enhance its tumor-targeting ability, Zhao et al. further isolated A1 within the tumor to obtain the A1-R strain with stronger affinity for tumor tissues. In addition, *Salmonella* not only inherently can inhibit tumor growth, but can also conveniently carry various DNA vectors. For example, *Escherichia coli* cytosine deaminase (CD), mitomycin C and colicin E3 have each been loaded into VNP20009 for therapeutic studies in various tumor models.

Although the above-mentioned nutritional deficiency-based attenuation methods can significantly reduce the distribution of bacteria in normal organs, such as the liver and spleen, it is difficult to observe a significant decrease over time in the distribution of bacteria in normal organs.

*Salmonella* YB1 is a tumor-targeting strain developed based on the previous research by using chromosomal homologous recombination technology combined with the concepts of synthetic biology. It exhibits tropism for hypoxic regions within tumors and an ability to accumulate in tumors.

Specifically, the anaerobic promoter *pepT* in combination with the reverse oxygen promoter *sodA* is used to control expression of the lethal gene *asd*, thereby enabling survival and growth under hypoxic conditions in tumors and reducing accumulation in normal organs. Compared with conventional oncolytic bacteria, this design improves safety.

YB1 is the first generation of attenuated *Salmonella* designed based on oxygen-regulated promoters by analyzing the oxygen levels within tumors. Mechanistically, it is already significantly superior to conventional auxotrophic strains. However, due to the inherent propensity of bacteria to mutate, it is necessary to pay attention to the mutational behavior of the bacteria under various stress conditions. In 2011, Dr. Yu Bin pointed out that YB1 is very prone to evolution in the absence of diaminopimelic acid (DAP), thereby escaping oxygen regulation. Studies revealed that the mutation frequency of YB1 with respect to oxygen regulation is as high as $10^{-5}$-$10^{-6}$ per bacterium, whereas the mutation frequency of *E. coli*, which belongs to the same Enterobacteriaceae family as *Salmonella*, is $10^{-9}$-$10^{-10}$ per bacterial generation. Other studies have shown that the natural mutation frequency of *Salmonella* is $10^{-8}$ per year, and the division time of *Salmonella* is 20-30 minutes per generation; when converted, the mutation frequency of YB1 is also 10,000-fold higher than the natural mutation frequency of *Salmonella*. Therefore, the substantial increase in the mutation frequency of YB1 is an abnormal phenomenon. This abnormally high mutation frequency diminishes the advantages of hypoxia regulation, may affect its safety, and may introduce new risks to tumor therapy. The possible reason may be related to the unreasonable design of its regulatory genetic elements, including the *pepT* promoter, the *asd* gene and the *sodA* promoter. Therefore, new regulatory schemes are highly important.

According to a 2012 study on YB1, although the distribution of YB1 in normal organs of mice shows a decreasing trend, it is difficult to achieve complete clearance within a short period of time. Even 11 days after injection, there remains a distribution of $10^3$-$10^4$ per gram of tissue, and it takes as long as 26 days to completely clear YB1 from most normal organs of mice, with a small amount remaining in the liver. The reason may be related to the high evolutionary rate caused by design defects inherent to YB1.

In summary, there is an urgent need in the field for a new generation of live bacterial strains that exhibit efficient tumor targeting, can be rapidly cleared from normal organs, and at the same time can efficiently inhibit tumor growth.

Summary of the Invention

In the present invention, unless otherwise specified, the scientific and technical terms used herein shall have the meanings commonly understood by those skilled in the art to which this invention pertains. Furthermore, the relevant terminology and laboratory procedures related to protein and nucleic acid chemistry, molecular biology, cell and tissue culture, microbiology, and immunology used in the present invention are all terms and conventional procedures widely used in the respective fields.

To address the above-mentioned defects existing in the prior art, the present invention provides a hypoxia-specific gene expression cassette, comprising:

a) a forward hypoxia promoter, which is a promoter comprising FNR binding site, wherein the forward hypoxia promoter is capable of being induced for expression under hypoxia;
b) a survival-essential gene; and
c) a reverse hyperoxia promoter, which is a promoter comprising FNR and ArcA binding sites, wherein

the reverse hyperoxia promoter is capable of functioning under oxygen concentration conditions of normal organs; wherein the survival-essential gene is a gene encoding alanine racemase.

On the other hand, the present invention provides a hypoxia-specific gene expression cassette, comprising:

a) a forward hypoxia promoter, which is a promoter comprising FNR binding site, wherein the forward hypoxia promoter is capable of being induced for expression under hypoxia;
b) a survival-essential gene; and
c) a reverse hyperoxia promoter, which is a promoter comprising two ArcA binding sites, wherein the reverse hyperoxia promoter is capable of functioning under oxygen concentration conditions of normal organs;

wherein the survival-essential gene is a gene encoding alanine racemase.

The terms "polypeptide", "peptide", and "protein" are used interchangeably herein and refer to a polymer of amino acid residues. The term applies to amino acid polymers in which one or more amino acid residues are artificial chemical analogs of a corresponding natural amino acid, as well as to polymers of natural amino acids. The terms "polypeptide", "peptide", "amino acid sequence", and "protein" may also include modified forms, including but not limited to glycosylation, lipidation, sulfation, γ-carboxylation of glutamic acid residues, hydroxylation, and ADP-ribosylation.

In the present invention, "polynucleotide" refers to a macromolecule formed by multiple nucleotides linked via phosphodiester bonds, wherein the nucleotides include ribonucleotides and deoxyribonucleotides. The sequence of the polynucleotide of the present invention can be codon-optimized for different host cells (such as *E. coli*) to improve polypeptide expression. Methods for codon optimization are known in the art.

"Sequence identity" between two polypeptide sequences or two polynucleotide sequences refers to the percentage of identical amino acids or nucleotides between said sequences. Methods for assessing the level of sequence identity between polypeptide or polynucleotide sequences are known in the art. Sequence identity can be assessed using various known sequence analysis software. For example, sequence identity can be evaluated using the online alignment tools provided by EMBL-EBI (https://www.ebi.ac.uk/Tools/psa/). Sequence identity between two sequences can be assessed using the Needleman-Wunsch algorithm with default parameters.

To enable bacteria to survive only under low oxygen conditions, three requirements need to be met:

1) Control over the strength of the upstream promoter and basal leaky expression.

If the oxygen sensor exhibits partial leaky expression without binding to the upstream promoter, it would result in the inability to regulate oxygen, resulting in bacterial survival under all oxygen concentrations; or if the strength of the upstream promoter is too weak to initiate the expression of the downstream gene, the bacteria will fail to survive under any oxygen concentration;

2) Selection of a suitable survival-essential gene.

Through the selection of the survival-essential gene, it can be ensured that the bacteria die when the survival-essential gene is not expressed, while bacterial survival can be rapidly ensured when expression is induced under hypoxia;

3) Upon transition between hypoxia and hyperoxia, the upstream promoter can rapidly initiate transcription to rapidly achieve the synthesis and expression of the survival-essential gene, ensuring that the modified bacteria can survive, whereas under high oxygen conditions, the expression of the survival-essential gene will not be initiated, thereby resulting in bacterial death.

Therefore, in the present invention, a "hypoxia-specific gene expression cassette" refers to a segment of DNA capable of initiating the expression of an essential gene under hypoxic conditions, comprising an essential gene under the control of a hypoxia-inducible promoter, and, if necessary, further comprising other regulatory elements required for the expression of the essential gene.

In the present invention, "essential gene" refers to a gene that plays a decisive role in the growth and/or survival of bacteria; once a bacterium lacks this gene or its functional expression product, it cannot survive, divide, and/or grow normally. A typical example of lacking an essential gene or its functional expression product is an auxotrophic strain, which cannot survive, divide, and/or grow normally under *in vitro* culture conditions or in an *in vivo* environment in the absence of a specific exogenous supplement. Essential genes usually exist as a single copy on the bacterial chromosome.

Accordingly, the requirements for the survival-essential gene are as follows:

(1) it is an essential gene for bacterial replication, and its absence results in rapid death of the bacteria;

(2) the product of this gene does not exist in the ordinary environment or the human body, which ensures that the bacteria will not escape control in the human body environment, and facilitates the cultivation and preparation of the bacteria in an ordinary culture environment by adding the corresponding expression product of the gene;

(3) this gene needs to be rapidly initiated under the control of a hypoxia promoter, and can rapidly synthesize the product to achieve the regulatory function of the host bacteria.

In the present invention, the survival-essential gene is a gene encoding alanine racemase.

For Gram-negative bacteria such as *Escherichia coli* and *Salmonella*, the cell wall is an indispensable component. The core component of the cell wall is peptidoglycan. During the synthesis of peptidoglycan, bacteria require D-alanine as an important constituent part. If D-alanine is absent, the bacteria will be unable to synthesize the cell wall, thereby resulting in lysis.

Amino acids existing in nature are typically L-form amino acids; therefore, two genes exist in Gram-negative bacteria: the *alr* gene and *dadX* gene, which synthesize alanine racemase and are responsible for converting L-alanine into D-alanine to meet the requirements of cell wall synthesis.

Studies have found that if both the *alr* gene and the *dadX* gene are mutated simultaneously, a lethal mutation of *Salmonella* can be achieved, and this mutation can be compensated by additionally supplementing D-alanine in the culture medium.

In our previous studies, *Salmonella* YB1 employed the *asd* gene as the essential gene for regulation. Compared with *Salmonella* YB1, the present invention selects the *alr* gene and the *dadX* gene as survival-essential genes. The *alr* gene and the *dadX* gene are functionally homologous genes. Therefore, in the present invention, the gene for alanine racemase can be the *alr* gene from *Salmonella* (SEQ ID NO: 17) and the *Salmonella* Alr protein sequence (SEQ ID NO: 18), or the *dadX* gene from *Salmonella* (SEQ ID NO: 19) and the *Salmonella* DadX protein sequence (SEQ ID NO: 20); or the *alr* gene from *Escherichia coli* (SEQ ID NO: 21) and the *Escherichia coli* Alr protein sequence (SEQ ID NO: 22), or the *dadX* gene from *Escherichia coli* (SEQ ID NO: 23) and the *Escherichia coli* DadX protein sequence (SEQ ID NO: 24); or the *alr* gene from *Shigella* (SEQ ID NO: 25) and the *Shigella* Alr protein sequence (SEQ ID NO: 26), or the *dadX* gene from *Shigella* (SEQ ID NO: 27) and the *Shigella* DadX protein sequence (SEQ ID NO: 28); or the *alr* gene from *Klebsiella* (SEQ ID NO: 29) and the *Klebsiella* Alr protein sequence (SEQ ID NO: 30), or the *dadX* gene from *Klebsiella* (SEQ ID NO: 31) and the *Klebsiella* DadX protein sequence (SEQ ID NO: 32); or the *alr* gene from *Yersinia* (SEQ ID NO: 33) and the *Yersinia* Alr protein sequence (SEQ ID NO: 34), or the *dadX* gene from *Yersinia* (SEQ ID NO: 35) and the *Yersinia* DadX protein sequence (SEQ ID NO: 36); or the *alr* gene from *Haemophilus* (SEQ ID NO: 37) and the *Haemophilus* Alr protein sequence (SEQ ID NO: 38); or the *alr* gene from *Pseudomonas* (SEQ ID NO: 39) and the *Pseudomonas* Alr protein sequence (SEQ ID NO: 40), or the *dadX* gene from *Pseudomonas* (SEQ ID NO: 41) and the *Pseudomonas* DadX protein sequence (SEQ ID NO: 42).

In the present invention, the alanine racemase *alr* gene and *dadX* gene comprise nucleotide sequences having greater than or equal to 81%, preferably greater than or equal to 82%, more preferably greater than or equal to 83%, greater than or equal to 84%, greater than or equal to 85%, greater than or equal to 86%, greater than or equal to 87%, greater than or equal to 88%, greater than or equal to 89%, greater than or equal to 90%, greater than or equal to 91%, greater than or equal to 92%, greater than or equal to 93%, greater than or equal to 94%, greater than or equal to 95%, greater than or equal to 96%, greater than or equal to 97%, greater than or equal to 98%, or most preferably greater than or equal to 99% sequence identity to the specific sequences described above.

In the present invention, an *alr* gene knockout bacterium was first constructed, followed by modification of another gene, *dadX*, resulting in a double-deficient strain lacking both *alr* and *dadX* genes after editing. Meanwhile, the forward hypoxia promoter *pepT* and the reverse hyperoxia promoter *sodA* of YB1 were used to regulate an additional *alr* gene (SWT006). According to the experimental results (SWT006 verification experiment), selecting the *alr* gene and the *dadX* gene as the regulatory means for the essential gene offers higher controllability than the essential gene *asd* selected for the previous *Salmonella* YB1.

The forward hypoxia promoter described in the present invention is a hypoxia-condition promoter regulated by FNR; the reverse hyperoxia promoter described in the present invention is an antisense promoter negatively regulated by FNR and/or ArcA. The fumarate and nitrate reduction gene *fnr* is an important gene regulating the aerobic and anaerobic growth of *Salmonella*. This complex regulatory system has been extensively studied in *Escherichia coli* and *Salmonella*. The DNA-binding protein FNR encoded by the *fnr* gene senses changes in oxygen and controls the expression of different genes, achieving a switch at the overall metabolic level. Therefore, DNA binding sequences such as FNR and ArcA become important modes for controlling downstream gene expression.

FNR possesses an oxygen-sensitive [4Fe-4S]$^{2+}$ domain, which can directly sense oxygen and regulate site-specific DNA binding. In contrast, ArcA senses signals from the aerobic respiratory chain. Thus, two distinct mechanisms exist in Gram-negative facultative anaerobic bacteria such as *Salmonella* and *Escherichia coli* for sensing changes in oxygen concentration. When regulating gene expression under hypoxia, FNR can act through two modes: activation and repression.

To this end, the present invention screened out forward hypoxia promoters *yhbU*, *ynfK*, *yecH*, *cydA*, *focA*, and *tdcA*, for which FNR activates downstream gene expression under hypoxic conditions, as well as reverse hyperoxia promoters *ydcI*, *cyoA*, *phoH*, *argT*, *mqo*, and *lldP*, for which FNR and/or ArcA repress downstream gene expression under hypoxic conditions. Moreover, promoters from different sources were screened to serve as the forward hypoxia promoters and reverse hyperoxia promoters applied in the present invention.

Wherein, the forward hypoxia promoters applied in the present invention are as follows:

(1) *Salmonella yhbU* promoter (yhbU-S) (SEQ ID NO: 1), wherein the FNR binding site is "CTGCCTTAAATCAA";
(2) *Escherichia coli ynfK* promoter (ynfK-E) (SEQ ID NO: 2), wherein the FNR binding site is "TTGCGCTATCTCAA″;
(3) *Salmonella tdcA* promoter (tdcA-S) (SEQ ID NO: 3), wherein the FNR binding site is "TTGATTGAAATCAG";
(4) *Escherichia coli tdcA* promoter (tdcA-E) (SEQ ID NO: 4), wherein the FNR binding site is "TTGACAAAAATCAG";
(5) *Escherichia coli yecH* promoter (yecH-E) (SEQ ID NO: 5), wherein the FNR binding site is "TTCATAAGCGGCAA";
(6) *Salmonella ynfK* promoter (ynfK-S) (SEQ ID NO: 6), wherein the FNR binding site is "TTGCGCTAACTCAA";
(7) *Escherichia coli cydA* promoter (cydA-E) (SEQ ID NO: 7), wherein the FNR binding site is "TTGATATTTATCAA";
(8) *Escherichia coli focA* promoter (focA-E) (SEQ ID NO: 8), wherein the FNR binding site is "ATGATCTATATCAA".

Wherein, the reverse hyperoxia promoters applied in the present invention are as follows:

(1) *Salmonella cyoA* promoter (cyoA-S) (SEQ ID NO: 9), wherein the FNR binding site is "TTTATTGATAATAA", and the ArcA binding site is "GTTAAGTA";

(2) *Salmonella phoH* promoter (phoH-S) (SEQ ID NO: 10), wherein the FNR binding site is "TTGAGCGCCGTTAA", and the ArcA binding site is "GTTAATTA";

(3) *Salmonella lldP* promoter (lldP-S) (SEQ ID NO: 11), wherein the FNR binding site is "TCCATACACAACAA", and the ArcA binding site is "GTTAACTA";

(4) *Salmonella argT* promoter (argT-S) (SEQ ID NO: 12), wherein dual ArcA binding sites are comprised, the first ArcA binding site is "TTTAATTC", and the second ArcA binding site is "TTTTATTA";

(5) *Salmonella ydcI* promoter (ydcI-S) (SEQ ID NO: 13), wherein the FNR binding site is "GTTATCAAAAACAA", and the ArcA binding site is "GTTAATAA";

(6) *Escherichia coli phoH* promoter (phoH-E) (SEQ ID NO: 14), wherein the FNR binding site is "TTCATCACTGTCAT", and the ArcA binding site is "GTTAAATA";

(7) *Escherichia coli lldP* promoter (lldP-E) (SEQ ID NO: 15), wherein the FNR binding site is "TTCATTGTCATTAT", and the ArcA binding site is "TTTAGTTA";

(8) *Escherichia coli ydcI* promoter (ydcI-E) (SEQ ID NO: 16), wherein the FNR binding site is "GTTATCATAATCAA", and the ArcA binding site is "GTTAATAA";

(9) *Escherichia coli mqo* promoter (mqo) (SEQ ID NO: 62), wherein dual ArcA binding sites are comprised, the first ArcA binding site is "ATTATTTA", and the second ArcA binding site is "GTTACTTA".

Through analysis of the aforementioned forward hypoxia promoters and reverse hyperoxia promoters, we found that the FNR binding sites conform to the pattern of "TTGATNNNNATCAA", and wherein any base in the TTGAT and ATCAA sequences of the conserved binding site can be substituted, provided that the total number of substitutions does not exceed 3, and 3 consecutive adjacent bases cannot be substituted; and the ArcA conforms to the pattern of its core region "GTTAATTA", and wherein any base in the GTTAATTA sequence of the conserved binding site can be substituted, provided that the total number of substitutions does not exceed 2.

Thus, in a preferred embodiment of the present invention, the FNR binding site of the forward hypoxia promoter or reverse hyperoxia promoter conforms to the pattern of TTGATNNNNATCAA, wherein N is any base selected from A, T, C, and G, and wherein any base in the TTGAT and ATCAA sequences of the conserved binding site can be substituted, provided that the total number of substitutions does not exceed 3, and 3 consecutive adjacent bases cannot be substituted; the ArcA binding site of the reverse hyperoxia promoter conforms to the pattern of GTTAATTA, wherein any base can be substituted, provided that the total number of substitutions does not exceed 2.

In a more preferred embodiment of the present invention, the forward hypoxia promoter is selected from *yhbU*, *ynfK*, *tdcA*, *yecH*, or *focA*; the survival-essential gene is selected from *alr* or *dadX*; and the reverse hyperoxia promoter is selected from *cyoA*, *ydcI*, *phoH*, *argT*, *mqo*, or *lldP*.

In a further preferred embodiment of the present invention, the expression cassette consists of the forward hypoxia promoter *yhbU*, the survival-essential gene *alr*, and the reverse hyperoxia promoter *cyoA*.

In a further preferred embodiment of the present invention, the expression cassette consists of the forward hypoxia promoter *yhbU*, the survival-essential gene *alr*, and the reverse hyperoxia promoter *ydcI*.

In a further preferred embodiment of the present invention, the expression cassette consists of the forward hypoxia promoter *yhbU*, the survival-essential gene *alr*, and the reverse hyperoxia promoter *phoH*.

In a further preferred embodiment of the present invention, the expression cassette consists of the forward hypoxia promoter *yhbU*, the survival-essential gene *alr*, and the reverse hyperoxia promoter *argT*.

In a further preferred embodiment of the present invention, the expression cassette consists of the forward hypoxia promoter *yhbU*, the survival-essential gene *alr*, and the reverse hyperoxia promoter *mqo*.

In a further preferred embodiment of the present invention, the expression cassette consists of the forward hypoxia promoter *ynfK*, the survival-essential gene *alr*, and the reverse hyperoxia promoter *cyoA*.

In a further preferred embodiment of the present invention, the expression cassette consists of the forward hypoxia promoter *ynfK*, the survival-essential gene *alr*, and the reverse hyperoxia promoter *phoH*.

In a further preferred embodiment of the present invention, the expression cassette consists of the forward hypoxia promoter *ynfK*, the survival-essential gene *alr*, and the reverse hyperoxia promoter *argT*.

In a further preferred embodiment of the present invention, the expression cassette consists of the forward hypoxia promoter *ynfK*, the survival-essential gene *alr*, and the reverse hyperoxia promoter *ydcI*.

In a further preferred embodiment of the present invention, the expression cassette consists of the forward hypoxia promoter *ynfK*, the survival-essential gene *alr*, and the reverse hyperoxia promoter *mqo*.

In a further preferred embodiment of the present invention, the expression cassette consists of the forward hypoxia promoter *tdcA*, the survival-essential gene *alr*, and the reverse hyperoxia promoter *ydcI*.

In a further preferred embodiment of the present invention, the expression cassette consists of the forward hypoxia promoter *tdcA*, the survival-essential gene *alr*, and the reverse hyperoxia promoter *cyoA*.

In a further preferred embodiment of the present invention, the expression cassette consists of the forward hypoxia promoter *yecH*, the survival-essential gene *alr*, and the reverse hyperoxia promoter *cyoA*.

In a further preferred embodiment of the present invention, the expression cassette consists of the forward hypoxia promoter *yecH*, the survival-essential gene *alr*, and the reverse hyperoxia promoter *lldP*.

In a further preferred embodiment of the present invention, the expression cassette consists of the forward hypoxia promoter *yecH*, the survival-essential gene *alr*, and the reverse hyperoxia promoter *argT*.

In a further preferred embodiment of the present invention, the expression cassette consists of the forward hypoxia promoter *yecH*, the survival-essential gene *alr*, and the reverse hyperoxia promoter *phoH*.

In a further preferred embodiment of the present invention, the expression cassette consists of the forward hypoxia promoter *yecH*, the survival-essential gene *alr*, and the reverse hyperoxia promoter *mqo*.

In a further preferred embodiment of the present invention, the expression cassette consists of the forward hypoxia promoter *focA*, the survival-essential gene *alr*, and the reverse hyperoxia promoter *lldP*.

In a further preferred embodiment of the present invention, the expression cassette consists of the forward hypoxia promoter *yhbU*, the survival-essential gene *dadX*, and the reverse hyperoxia promoter *cyoA*.

In a further preferred embodiment of the present invention, the expression cassette consists of the forward hypoxia promoter *yhbU*, the survival-essential gene *dadX*, and the reverse hyperoxia promoter *ydcI*.

In a further preferred embodiment of the present invention, the expression cassette consists of the forward hypoxia promoter *yhbU*, the survival-essential gene *dadX*, and the reverse hyperoxia promoter *phoH*.

In a further preferred embodiment of the present invention, the expression cassette consists of the forward hypoxia promoter *yhbU*, the survival-essential gene *dadX*, and the reverse hyperoxia promoter *argT*.

In a further preferred embodiment of the present invention, the expression cassette consists of the forward hypoxia promoter *yhbU*, the survival-essential gene *dadX*, and the reverse hyperoxia promoter *mqo*.

In a further preferred embodiment of the present invention, the expression cassette consists of the forward hypoxia promoter *ynfK*, the survival-essential gene *dadX*, and the reverse hyperoxia promoter *cyoA*.

In a further preferred embodiment of the present invention, the expression cassette consists of the forward hypoxia promoter *ynfK*, the survival-essential gene *dadX*, and the reverse hyperoxia promoter *phoH*.

In a further preferred embodiment of the present invention, the expression cassette consists of the forward hypoxia promoter *ynfK*, the survival-essential gene *dadX*, and the reverse hyperoxia promoter *argT*.

In a further preferred embodiment of the present invention, the expression cassette consists of the forward hypoxia promoter *ynfK*, the survival-essential gene *dadX*, and the reverse hyperoxia promoter *ydcI*.

In a further preferred embodiment of the present invention, the expression cassette consists of the forward hypoxia promoter *ynfK*, the survival-essential gene *dadX*, and the reverse hyperoxia promoter *mqo*.

In a further preferred embodiment of the present invention, the expression cassette consists of the forward hypoxia promoter *tdcA*, the survival-essential gene *dadX*, and the reverse hyperoxia promoter *ydcI*.

In a further preferred embodiment of the present invention, the expression cassette consists of the forward hypoxia promoter *tdcA*, the survival-essential gene *dadX*, and the reverse hyperoxia promoter *cyoA*.

In a further preferred embodiment of the present invention, the expression cassette consists of the forward hypoxia promoter *yecH*, the survival-essential gene *dadX*, and the reverse hyperoxia promoter *cyoA*.

In a further preferred embodiment of the present invention, the expression cassette consists of the forward hypoxia promoter *yecH*, the survival-essential gene *dadX*, and the reverse hyperoxia promoter *lldP*.

In a further preferred embodiment of the present invention, the expression cassette consists of the forward hypoxia promoter *yecH*, the survival-essential gene *dadX*, and the reverse hyperoxia promoter *argT*.

In a further preferred embodiment of the present invention, the expression cassette consists of the forward hypoxia promoter *yecH*, the survival-essential gene *dadX*, and the reverse hyperoxia promoter *phoH*.

In a further preferred embodiment of the present invention, the expression cassette consists of the forward hypoxia promoter *yecH*, the survival-essential gene *dadX*, and the reverse hyperoxia promoter *mqo*.

In a further preferred embodiment of the present invention, the expression cassette consists of the forward hypoxia promoter *focA*, the survival-essential gene *dadX*, and the reverse hyperoxia promoter *lldP*.

The forward hypoxia promoter disclosed in patent CN104471057B is *pepT*. The *pepT* promoter is not completely regulated by FNR, but is a promoter dually regulated by CRP-cAMP and FNR. Therefore, the FNR binding site in the *pepT* promoter is half a CRP binding site and half an FNR binding site. The CRP-FNR binding region sequence of the *pepT* promoter is GTGACCTGACGCAA, wherein the first half, GTGA, conforms to the first half of the CRP conserved binding site GTGANNNNNTCAC, and the latter half, CGCAA, conforms to a part of the FNR conserved region ATCAA, wherein the tenth position A is substituted by C, and the eleventh position T is substituted by G, and thus it does not conform to the rules for the forward hypoxia promoter of the present invention.

The FNR binding region of the reverse hyperoxia promoter *sodA* disclosed in patent CN104471057B, TTGATAATCATTTT, only contains the first half-region of FNR, TTGAT, and the latter half-region contains three consecutive substitutions, with ATCAA being substituted by ATTTT; therefore, it does not comprise a complete FNR binding site. The ArcA binding site comprised therein is TTTAATTA, which has the 'G' at the first position substituted by 'T' when compared to the conserved core ArcA binding site 5'-GTTAATTA-3'. Therefore, the *sodA* promoter only comprises a single ArcA binding site, and its FNR binding site is incomplete, and thus it does not conform to the rules for the reverse hyperoxia promoter in the present invention.

In summary, the present invention has achieved a more efficient and safer hypoxia-specific gene expression cassette by screening appropriate forward hypoxia promoters and reverse hyperoxia promoters, along with suitable survival -essential genes.

In a preferred embodiment of the present invention, the forward hypoxia promoter and/or reverse hyperoxia promoter are promoters derived from Gram-negative bacteria.

In the present invention, the term "Gram-negative bacteria" refers to bacteria that do not retain the initial basic dye stain (e.g., crystal violet) after the known partial procedure of Gram staining. In an exemplary Gram staining, cells are first fixed on a slide by heating and stained with a basic dye (e.g., crystal violet), the basic dye being absorbed by both Gram-negative and Gram-positive bacteria. Then, the slide is treated with a mordant (e.g., Gram's iodine), which binds to the basic dye (e.g., crystal violet) and traps it within the cells. The cells are then washed with acetone or alcohol and counterstained with a second dye of different color (e.g., safranin). Gram-positive organisms retain the initial purple stain, whereas Gram-negative organisms are decolorized by the organic solvent wash and thus show the counterstain. Exemplary Gram-negative bacteria include, but are not limited to species of *Escherichia* spp., *Shigella* spp., *Salmonella* spp., *Campylobacter* spp., *Neisseria* spp., *Haemophilus* spp., *Aeromonas* spp., *Francisella* spp., *Yersinia* spp., *Klebsiella* spp., *Bordetella* spp., *Legionella* spp., *Corynebacterium* spp., *Citrobacter* spp., *Chlamydia* spp., *Brucella* spp., *Pseudomonas* spp., *Helicobacter* spp., and *Vibrio* spp.

Thus, in a more preferred embodiment of the present invention, the Gram-negative bacterium is selected from the group consisting of *Salmonella*, *Escherichia coli*, *Shigella*, *Yersinia*, *Enterobacter cloacae*, *Cronobacter*, *Klebsiella*, *Pantoea*, *Serratia*, *Shimwellia*, *Enterobacter ludwigii*, *Haemophilus*, *Vibrio*, *Pseudomonas*, *Pasteurella*, *Bordetella*, *Bordetella pertussis*, *Acinetobacter baumannii*, *Burkholderia*, *Vibrio vulnificus*, *Bacteroides fragilis*, *Pseudomonas syringae*, *Pseudomonas putida, Legionella, Klebsiella pneumoniae*, *Vibrio parahaemolyticus*, *Vibrio cholerae*, *Yersinia pestis*, *Catarrhalis coccus, Moraxella catarrhalis*, *Campylobacter jejuni*, *Shigella dysenteriae*, *Neisseria gonorrhoeae*, *Haemophilus influenzae*, *Moraxella*, *Neisseria meningitidis*, *Proteus vulgaris*, *Proteus mirabilis*, *Pasteurella haemolytica*, *Salmonella enterica*, *Salmonella bongori*, *Salmonella paratyphi*, *Salmonella typhi*, *Legionella pneumophila*, *Yersinia pestis*, *Shigella sonnei*, *Pseudomonas aeruginosa*, *Yersinia enterocolitica*, *Cryptococcus neoformans*, *Burkholderia cenocepacia*, and *Helicobacter pylori*.

In the present invention, the term "live bacteria" refers to a bacterial strain that is viable, possesses active nutritional metabolic characteristics, and can exercise its own biological functions. Live bacteria may include bacterial biomass generated during the metabolic processes of the strain.

In a further preferred embodiment of the present invention, the forward hypoxia promoter is *yhbU*, which is selected from the group consisting of *Salmonella*, *Escherichia coli*, *Shigella*, *Yersinia*, *Enterobacter cloacae*, *Cronobacter*, *Klebsiella*, *Pantoea*, *Serratia*, and *Shimwellia*.

In a further preferred embodiment of the present invention, the forward hypoxia promoter is *ynfK*, which is selected from the group consisting of *Salmonella*, *Escherichia coli*, *Serratia*, *Shigella*, and *Enterobacter ludwigii*.

In a preferred embodiment of the present invention, the alanine racemase gene *alr* and/or *dadX* is selected from the group consisting of *Salmonella*, *Escherichia coli*, *Shigella*, *Klebsiella*, *Yersinia*, *Haemophilus*, and *Pseudomonas*.

In a preferred embodiment of the present invention, the hypoxia-specific gene expression cassette is regulated by oxygen concentration.

To enable Gram-negative bacteria such as *Salmonella* to survive in hypoxic regions, the 1% oxygen concentration is a very important key point, as it represents pathological hypoxia. This is because an oxygen concentration below 1% is a clear hallmark of a tumor hypoxic region, and regions with an oxygen concentration below 1% do not exist in normal organ tissues. For example, the oxygen concentration in the hypoxic regions of various tumors, such as pancreatic cancer, cervical cancer, and prostate cancer, is lower than 0.7%.

The hypoxia-specific gene expression cassette designed by the present invention enables Gram-negative bacteria such as *Salmonella* to recognize tumor hypoxic regions and proliferate therein through precise oxygen regulation. The objective achieved by the present invention is that when the oxygen concentration is lower than 0.8%, the modified Gram-negative bacteria such as *Salmonella* can survive and proliferate, and achieve suicide lysis in a normal oxygen concentration environment.

Thus, in a more preferred embodiment of the present invention, the forward hypoxia promoter functions when the oxygen concentration is lower than 1%, and does not function when the oxygen concentration is higher than 1%; and/or the reverse hyperoxia promoter functions when the oxygen concentration is higher than 1%, and does not function when the oxygen concentration is lower than 1%.

In a further preferred embodiment of the present invention, the forward hypoxia promoter functions when the oxygen concentration is lower than 0.8%, and does not function when the oxygen concentration is higher than 0.8%; and/or the reverse hyperoxia promoter functions when the oxygen concentration is higher than 0.8%, and does not function when the oxygen concentration is lower than 0.8%.

In a preferred embodiment of the present invention, the hypoxia-specific gene expression cassette is integrated into the chromosome of a host bacterium.

Another aspect of the present invention provides a modified Gram-negative bacterium, which is modified by the hypoxia-specific gene expression cassette described in the present invention.

In a preferred embodiment of the present invention, the Gram-negative bacterium is *Salmonella*, *Escherichia coli*, *Shigella*, *Yersinia*, *Enterobacter cloacae*, *Cronobacter*, *Klebsiella*, *Pantoea*, *Serratia*, *Shimwellia*, *Enterobacter ludwigii*, *Haemophilus*, *Vibrio*, *Pseudomonas*, *Pasteurella*, *Bordetella*, *Bordetella pertussis*, *Acinetobacter baumannii*, *Burkholderia*, *Vibrio vulnificus*, *Bacteroides fragilis*, *Pseudomonas syringae*, *Pseudomonas putida*, *Legionella*, *Klebsiella pneumoniae*, *Vibrio parahaemolyticus*, *Vibrio cholerae*, *Yersinia pestis*, *Catarrhalis coccus, Moraxella catarrhalis*, *Campylobacter jejuni*, *Shigella dysenteriae*, *Neisseria gonorrhoeae*, *Haemophilus influenzae*, *Moraxella*, *Neisseria meningitidis*, *Proteus vulgaris*, *Proteus mirabilis*, *Pasteurella haemolytica*, *Salmonella enterica*, *Salmonella bongori*, *Salmonella paratyphi*, *Salmonella typhi*, *Legionella pneumophila*, *Yersinia pestis*, *Shigella sonnei*, *Pseudomonas aeruginosa*, *Yersinia* enterocolitica, *Cryptococcus neoformans*, *Burkholderia cenocepacia*, and *Helicobacter pylori*.

In a more preferred embodiment of the present invention, the Gram-negative bacterium is *Salmonella typhimurium*.

Another aspect of the present invention provides a use of the hypoxia-specific gene expression cassette according to the present invention or the Gram-negative bacterium according to the present invention in the preparation of a medicament for treating a tumor.

In a preferred embodiment of the present invention, the tumor is a solid tumor.

The term "solid tumor" as used in the present invention refers to an abnormal mass of tissue, which usually does not contain cysts or liquid areas. Solid tumors may be benign (non-cancerous) or malignant (cancerous). Different types of malignant solid tumors are named according to the type of cells from which they form. Examples of malignant solid tumors are sarcomas, carcinomas, and lymphomas. Leukemias (blood cancers) generally do not form malignant solid tumors. Malignant solid tumors include, but are not limited to, abnormal cell masses that may originate from different tissue types, such as liver, colon, colorectal, skin, breast, pancreas, cervix, uterine body, bladder, gallbladder, kidney, larynx, lip, oral cavity, esophagus, ovary, prostate, stomach, testis, thyroid, or lung, etc.; therefore, malignant solid tumors include malignant solid liver tumors, colon tumors, colorectal tumors, skin tumors, breast tumors, pancreatic tumors, cervical tumors, uterine body tumors, bladder tumors, gallbladder tumors, kidney tumors, laryngeal tumors, lip tumors, oral cavity tumors, esophageal tumors, ovarian tumors, prostate tumors, stomach tumors, testicular tumors, thyroid tumors, or lung tumors, etc.

Thus, in a more preferred embodiment of the present invention, the solid tumor is selected from the group consisting of tumors/cancers of the breast, bone, liver, lung, skin, kidney, stomach, pancreas, prostate, lymph (non-Hodgkin's lymphoma, Hodgkin's lymphoma), intestine (colon cancer, rectal cancer), pelvis (cervical cancer, ovarian malignancy, endometrial cancer, ovarian cancer), nervous system, head and neck, and bladder.

In a further preferred embodiment of the present invention, the solid tumor is breast cancer, osteosarcoma, liver cancer, lung cancer, melanoma, kidney cancer, stomach cancer, pancreatic cancer, prostate cancer, colon cancer, ovarian cancer, neuroblastoma, squamous cell carcinoma, and bladder cancer.

In the present invention, for therapeutic purposes, the term "subject" preferably refers to a subject in need of treatment for a target pathological condition such as a tumor. For prevention purposes, the subject is preferably a subject at risk of developing a target pathological condition or predisposed to developing a target pathological condition. The term "subject" includes living organisms, such as prokaryotes and eukaryotes. Examples of subjects include mammals, such as humans, dogs, cows, horses, pigs, sheep, goats, cats, mice, rabbits, hedgehogs, rats, and transgenic non-human animals. In a specific embodiment of the present invention, the subject is a human.

As used in the present invention, "treatment" is a process used to obtain beneficial or desired clinical results. For the purposes of the present invention, beneficial or desired clinical results include, but are not limited to, one or more of the following: reducing the proliferation of neoplastic or cancerous cells (or destroying neoplastic or cancerous cells), inhibiting metastasis of neoplastic cells, shrinking or reducing tumor size, alleviating malignancy, alleviating symptoms caused by malignancy, improving the quality of life of subjects suffering from malignancy, reducing the dose of other drugs required to treat malignancy, delaying the progression of malignancy, curing malignancy, and/or prolonging the survival of patients suffering from malignancy.

As used in the present invention, an "effective amount" or "effective dose" of a bacterium, drug, or pharmaceutical composition is an amount sufficient to achieve any one or more beneficial or desired results. For prophylactic use, beneficial or desired results include eliminating or reducing the risk of disease, reducing the severity of disease, or delaying the onset of disease, including biochemical, histological, and/or behavioral symptoms of the disease, its complications, and intermediate pathological phenotypes presenting during the development of the disease. For therapeutic use, beneficial or desired results include such as alleviating one or more symptoms of a disease (such as tumor), reducing the dose of other drugs required to treat the disease, enhancing the effect of another drug, prolonging the survival of the treated subject, and/or delaying the progression of cancer in patients. For example, compared to untreated subject, an "effective amount" preferably inhibits cell growth or tumor growth by at least about 10%, preferably at least about 20%, more preferably at least about 30%, more preferably at least about 40%, more preferably at least about 50%, more preferably at least about 60%, more preferably at least about 70%, more preferably at least about 80%. The ability to inhibit tumor growth can be evaluated in animal model systems predictive of efficacy against human tumors. Alternatively, it can also be evaluated by examining the ability to inhibit cell growth, which can be determined *in vitro* by assays well known to those skilled in the art. A therapeutically effective amount of a therapeutic compound can reduce tumor size or otherwise alleviating symptoms in a subject. Those skilled in the art can determine such amounts based on factors such as the size of the subject, the severity of the subject's symptoms, and the specific composition or route of administration selected.

In some embodiments of the present invention, the effective amount of bacteria in the bacterial agent, drug, or pharmaceutical composition comprises at least about $10^4$ colony-forming units (cfu), for example, at least about $10^4$, $10^5$, $10^6$, $10^7$, $10^8$, $10^9$, $10^{10}$, $10^{11}$, $10^{12}$, or $10^{13}$ cfu, including any ranges between the specified values, such as $10^4$-$10^8$cfu, $10^4$-$10^9$cfu, $10^4$-$10^{10}$cfu, $10^4$-$10^{11}$cfu, $10^4$-$10^{12}$cfu, $10^4$-$10^{13}$cfu, $10^5$-$10^8$cfu, $10^5$-$10^9$cfu, $10^5$-$10^{10}$cfu, $10^5$-$10^{11}$cfu, $10^5$-$10^{12}$cfu, $10^5$-$10^{13}$cfu, $10^6$-$10^8$cfu, $10^6$-$10^9$cfu, $10^6$-$10^{10}$cfu, $10^6$-$10^{11}$cfu, $10^6$-$10^{12}$cfu, $10^6$-$10^{13}$cfu, $10^7$-$10^8$cfu, $10^7$-$10^9$cfu, $10^7$-$10^{10}$cfu, $10^7$-$10^{11}$cfu, $10^7$-$10^{12}$cfu, $10^7$-$10^{13}$cfu, $10^8$-$10^9$cfu, $10^8$-$10^{10}$cfu, $10^8$-$10^{11}$cfu, $10^8$-$10^{12}$cfu or $10^8$-$10^{13}$cfu.

In some embodiments of the present invention, the volume of the effective dose of the bacteria, drug, or pharmaceutical composition of the present invention is less than or equal to about 3 mL, about 2.5 mL, about 2 mL, about 1.5 mL, about 1 mL, about 0.75 mL, about 0.5 mL, about 0.25 mL, or about 0.1 mL. In some embodiments, the volume of the effective dose of the bacteria, drug, or pharmaceutical composition is about 20 mL, about 19 mL, about 18 mL, about 17 mL, about 16 mL, about 15 mL, about 14 mL, about 13 mL, about 12 mL, about 11 mL, about 10 mL, about 9 mL, about 8 mL, about 7 mL, about 6 mL, about 5 mL, about 4 mL, about 3 mL, about 2 mL, or about 1 mL. Alternatively, the volume of the effective dose of the bacteria, drug, or pharmaceutical composition is about 20.5 mL, about 19.5 mL, about 18.5 mL, about 17.5 mL, about 16.5 mL, about 15.5 mL, about 14.5 mL, about 13.5 mL, about 12.5 mL, about 11.5 mL, about 10.5 mL, about 9.5 mL, about 8.5 mL, about 7.5 mL, about 6.5 mL, about 5.5 mL, about 4.5 mL, about 3.5 mL, about 2.5 mL, about 1.5 mL, or about 0.5 mL. In some embodiments, the volume of the effective dose of the bacteria, drug, or pharmaceutical composition is about 200 mL, about 190 mL, about 180 mL, about 170 mL, about 160 mL, about 150 mL, about 140 mL, about 130 mL, about 120 mL, about 110 mL, about 100 mL, about 90 mL, about 80 mL, about 70 mL, about 60 mL, about 50 mL, about 40 mL, or about 30 mL, as well as ranges between any of the listed values, such as 100 mL-110 mL, 100 mL-120 mL, 90 mL-120 mL, 90 mL-130 mL, etc. Alternatively, the volume of the effective dose of the bacteria, drug, or pharmaceutical composition is about 205 mL, about 195 mL, about 185 mL, about 175 mL, about 165 mL, about 155 mL, about 145 mL, about 135 mL, about 125 mL, about 115 mL, about 105 mL, about 95 mL, about 85 mL, about 75 mL, about 65 mL, about 55 mL, about 45 mL, about 35 mL, or about 25 mL, as well as ranges between any of the listed values, such as 105 mL-115 mL, 105 mL-125 mL, 95 mL-125 mL, 95 mL-135 mL, etc. Alternatively, the volume of the effective dose of the bacteria, drug, or pharmaceutical composition is about 900 microliters, about 800 microliters, about 700 microliters, about 600 microliters, about 500 microliters, about 400 microliters, about 300 microliters, about 200 microliters, or about 100 microliters; alternatively about 950 microliters, about 850 microliters, about 750 microliters, about 650 microliters, about 550 microliters, about 450 microliters, about 350 microliters, about 250 microliters, about 150 microliters, or about 50 microliters.

In some embodiments, the volume of the effective dose of the bacteria, drug, or pharmaceutical composition is less than or equal to about 2.0 mL.

The dosage ranges discussed herein are merely exemplary and do not limit the dosage ranges that may be selected by a physician. The amount of the active ingredient (e.g., the bacteria of the present invention) in the pharmaceutical composition of the present invention may vary depending on factors such as the disease state, age, gender, and weight of the individual, including the presence or absence of a tumor, the type of tumor being treated, the severity of the tumor, the activity or viability of the bacteria, drug, or pharmaceutical composition, the route of administration, the duration of treatment, the drugs used in combination with the bacteria, drug, or pharmaceutical composition (if any), the diet and general health of the subject, and similar factors well known in the art. The dosage regimen may be adjusted to provide the optimum therapeutic response. For example, it may be administered as a single dose, several divided doses may be administered over time, or the dose may be proportionally reduced or increased as indicated by the exigencies of the therapeutic situation.

As used herein, "pharmaceutically acceptable carrier" includes any material which, when combined with the active ingredient, allows the ingredient to retain its biological activity and does not react with the subject's immune system, including but not limited to disintegrants, binders, fillers, buffers, tonicity agents, stabilizers, antioxidants, surfactants, and lubricants. Preferably, the carrier is suitable for intravenous, intramuscular, subcutaneous, parenteral, spinal, or epidermal administration (e.g., by injection or infusion). For example, depending on the route of administration, the bacteria of the present invention may be coated in a material to protect the bacteria from acidic and other natural conditions that could inactivate them. Pharmaceutically acceptable carriers include physiological saline, PBS buffer, sterile aqueous solutions or dispersions, and powders for the preparation of injectable solutions or dispersions temporarily. The use of such media and agents for pharmaceutically active substances is well known in the art. Conventional media or agents except those incompatible with the active compound may be present in the pharmaceutical compositions of the present invention.

Accordingly, the present invention provides a pharmaceutical composition comprising an effective amount of the hypoxia-specific gene expression cassette of the present invention or the modified bacteria of the present invention. In one embodiment, the modified bacterium is a live bacterium.

In a preferred embodiment of the present invention, the pharmaceutical composition further comprises a pharmaceutically acceptable carrier.

In a more preferred embodiment of the present invention, the pharmaceutically acceptable carrier is selected from disintegrants, binders, fillers, buffers, tonicity agents, stabilizers, antioxidants, surfactants, and lubricants.

In a preferred embodiment of the present invention, the pharmaceutical composition is used for treating solid tumors. The bacteria, drug, or pharmaceutical composition of the present invention is administered via the following routes: intravenous injection, intratumoral injection, intramuscular injection, subcutaneous injection, intraperitoneal injection, intracerebral administration, gastrointestinal administration, topical administration, buccal administration, nasal administration, rectal administration, or vaginal administration.

In a preferred embodiment of the present invention, the bacteria, drug, or pharmaceutical composition of the present invention can be formulated into a form suitable for administration via the following routes: intravenous injection, intratumoral injection, intramuscular injection, subcutaneous injection, intraperitoneal injection, intracerebral administration, gastrointestinal administration, topical administration, buccal administration, nasal administration, rectal administration, or vaginal administration.

The dosage forms of the drug or pharmaceutical composition of the present invention may be in the form of solutions, emulsions, lyophilized formulation, or suspensions; for oral administration, the dosage form may be in the form of tablets or capsules; for intranasal administration, the dosage form may be in the form of powders, nasal drops, or aerosols; for topical administration, the dosage form may be aqueous solutions, suspensions, ointments, creams, or gels; for rectal or vaginal administration, the dosage form may be suppositories, enemas, or delivered as part of an endoscopic or colonoscopic procedure.

The bacteria, drug, or pharmaceutical composition of the present invention can be manufactured by methods well known in the art, such as microbial growth in a fermenter, followed by centrifugal concentration and washing, filtration or dialysis, conventional granulation, mixing, dissolving, encapsulation, lyophilization, or emulsification processes, and other methods. The bacteria, drug, or pharmaceutical composition of the present invention can be produced in various forms, including granules, precipitates or particulates, powders (including freeze-dried, rotary-dried, or spray-dried powders), amorphous powders, injectables, emulsions, elixirs, suspensions, or solutions. The formulation may optionally contain stabilizers, pH adjusters, surfactants, bioavailability modifiers, and combinations thereof.

The bacteria, drug, or pharmaceutical composition of the present invention can be administered alone or in combination with other compounds or compositions in the presence of a carrier. In a preferred embodiment of the present invention, the bacteria, drug, or pharmaceutical composition can be administered in combination with other malignant tumor therapies (including but not limited to radiotherapy, chemotherapy, and surgery). The bacteria, drug, or pharmaceutical composition can be used as an adjuvant in therapy in such cases.

In another aspect, the present invention provides a method for treating a tumor, comprising administering to a subject the hypoxia-specific gene expression cassette described in the present invention, the Gram-negative bacteria described in the present invention, or the pharmaceutical composition described in the present invention.

In one embodiment, the hypoxia-specific gene expression cassette, Gram-negative bacteria, or pharmaceutical composition is used for treating a tumor. In one embodiment, the hypoxia-specific gene expression cassette, Gram-negative bacteria, or pharmaceutical composition is used for inducing an anti-tumor specific immune response in a subject having a tumor. In one embodiment, the hypoxia-specific gene expression cassette, Gram-negative bacteria, or pharmaceutical composition is used for inducing anti-tumor immune memory in a subject having a tumor. In one embodiment, the hypoxia-specific gene expression cassette, Gram-negative bacteria, or pharmaceutical composition is used for preventing or treating metastasis or recurrence of a tumor. In one embodiment, the hypoxia-specific gene expression cassette, Gram-negative bacterium, or pharmaceutical composition is used for treating a tumor that has developed resistance to prior anti-tumor treatments or where treatment has failed.

In one embodiment of the present invention, the tumor is a tumor of the nervous system, respiratory system, digestive system, urinary system, reproductive system, hematopoietic system, lymphatic system, endocrine system, or skin and mucous membranes. In one embodiment, the tumor is a sarcoma or carcinoma. In one embodiment, the tumor is a solid tumor.

In a further preferred embodiment of the present invention, the solid tumor is selected from tumors/cancers of the breast, bone, liver, lung, skin, kidney, stomach, pancreas, prostate, lymph (non-Hodgkin's lymphoma, Hodgkin's lymphoma), intestine (colon cancer, rectal cancer), pelvis (cervical cancer, ovarian malignancy, endometrial cancer, ovarian cancer), nervous system, head and neck cancer, and bladder, etc.

In a more preferred embodiment of the present invention, the solid tumor is breast cancer, osteosarcoma, liver cancer, lung cancer, melanoma, kidney cancer, gastric cancer, pancreatic cancer, prostate cancer, colon cancer, ovarian cancer, neuroblastoma, squamous cell carcinoma, and bladder cancer.

The method for treating a tumor provided by the present invention comprises administering an effective amount of the hypoxia-specific gene expression cassette, Gram-negative bacteria, or pharmaceutical composition of the present invention to a subject having a tumor.

In an embodiment of the present invention, the hypoxia-specific gene expression cassette, Gram-negative bacteria, or pharmaceutical composition of the present invention may be administered via the following routes: intravenous injection, intratumoral injection, intramuscular injection, subcutaneous injection, intraperitoneal injection, intracerebral administration, gastrointestinal administration, topical administration, buccal administration, nasal administration, rectal administration, or vaginal administration.

In some embodiments of the present invention, the hypoxia-specific gene expression cassette, Gram-negative bacteria, or pharmaceutical composition of the present invention may be administered at intervals of, for example, about 1 minute, 2, 3, 4, 5, 6, 7, 8, 9, 10, 15, 20, 25, 30, 35, 40, 45, 50, 55 minutes, 1 hour, 2, 3, 4, 5, 6, 7, 8, 9, 10, 11, 12, 13, 14, 15, 16, 17, 18, 19, 20, 21, 22, 23 hours, 1 day, 2, 3, 4, 5, 6, 7, 8, 9, or 10 days, including ranges between any two of the listed values, for example, 1 minute-10 minutes, 1 minute-30 minutes, 1 minute-1 hour, 1 minute-2 hours, 1 minute-4 hours, 1 minute-12 hours, 1 minute-18 hours, 1 minute-1 day, 10 minutes-30 minutes, 10 minutes-1 hour, 10 minutes-2 hours, 10 minutes-4 hours, 10 minutes-12 hours, 10 minutes-18 hours, 10 minutes-1 day, 30 minutes-1 hour, 30 minutes-2 hours, 30 minutes-4 hours, 30 minutes-12 hours, 30 minutes-18 hours, 30 minutes-1 day, 30 minutes-2 days, 1 hour-2 hours, 1 hour-4 hours, 1 hour-12 hours, 1 hour-18 hours, 1 hour-1 day, 4 hours-12 hours, 4 hours-18 hours, 4 hours-1 day, 1 day-2 days, 1 day-3 days, 1 day-4 days, 1 day-5 days, 1 day-7 days, 1 day-10 days, 2 days-3 days, 2 days-4 days, 2 days-5 days, 2 days-7 days, 2 days-10 days, or 5 days-10 days. In some embodiments, the hypoxia-specific gene expression cassette, Gram-negative bacteria, or pharmaceutical composition is administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. In some embodiments, the hypoxia-specific gene expression cassette, Gram-negative bacteria, or pharmaceutical composition is formulated in a form that, for example, can be administered at intervals of about 1 minute, 2, 3, 4, 5, 6, 7, 8, 9, 10, 15, 20, 25, 30, 35, 40, 45, 50, 55 minutes, 1 hour, 2, 3, 4, 5, 6, 7, 8, 9, 10, 11, 12, 13, 14, 15, 16, 17, 18, 19, 20, 21, 22, 23 hours, 1 day, 2, 3, 4, 5, 6, 7, 8, 9, or 10 days, including ranges between any two of the listed values, for example, 1 minute-10 minutes, 1 minute-30 minutes, 1 minute-1 hour, 1 minute-2 hours, 1 minute-4 hours, 1 minute-12 hours, 1 minute-18 hours, 1 minute-1 day, 10 minutes-30 minutes, 10 minutes-1 hour, 10 minutes-2 hours, 10 minutes-4 hours, 10 minutes-12 hours, 10 minutes-18 hours, 10 minutes-1 day, 30 minutes-1 hour, 30 minutes-2 hours, 30 minutes-4 hours, 30 minutes-12 hours, 30 minutes-18 hours, 30 minutes-1 day, 30 minutes-2 days, 1 hour-2 hours, 1 hour-4 hours, 1 hour-12 hours, 1 hour-18 hours, 1 hour-1 day, 4 hours-12 hours, 4 hours-18 hours, 4 hours-1 day, 1 day-2 days, 1 day-3 days, 1 day-4 days, 1 day-5 days, 1 day-7 days, 1 day-10 days, 2 days-3 days, 2 days-4 days, 2 days-5 days, 2 days-7 days, 2 days-10 days, or 5 days-10 days. In some embodiments, the bacterium, drug, or pharmaceutical composition is formulated in a form to be administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. The dosage regimen may depend on the pattern of pharmacokinetic decay expected by the medical practitioner. The progress of the therapy can be monitored by conventional techniques and assays. The administration regimen may change over time.

The present invention also provides a method for inducing an anti-tumor specific immune response in a subject having a tumor, comprising administering to the subject having a tumor an effective amount of the hypoxia-specific gene expression cassette, Gram-negative bacteria, or pharmaceutical composition of the present invention.

The present invention also provides a method for inducing anti-tumor immune memory in a subject having a tumor, comprising administering to the subject having a tumor an effective amount of the hypoxia-specific gene expression cassette, Gram-negative bacteria, or pharmaceutical composition of the present invention.

The present invention also provides a method for preventing or treating metastasis or recurrence of a tumor, comprising administering to a subject having a tumor an effective amount of the hypoxia-specific gene expression cassette, Gram-negative bacteria, or pharmaceutical composition of the present invention.

The present invention also provides a method for preventing or treating metastasis or recurrence of a tumor, comprising administering an effective amount of the hypoxia-specific gene expression cassette, Gram-negative bacteria, or pharmaceutical composition of the present invention to a subject having tumor metastasis or recurrence or a subject at high risk of tumor metastasis or recurrence.

The present invention also provides a method for treating a tumor that has developed resistance to prior anti-tumor treatments or where treatment has failed, comprising administering an effective amount of the hypoxia-specific gene expression cassette, Gram-negative bacteria, or pharmaceutical composition of the present invention to a subject having a tumor that has developed resistance to prior anti-tumor treatments or where treatment has failed.

By adopting the above technical solutions, the present invention achieves the following beneficial effects:

1. By rationally designing a forward hypoxia promoter and a reverse hyperoxia promoter, in combination with an appropriate survival-essential gene, the present invention obtains a more efficient and safer hypoxia-specific gene expression cassette.

2. The present invention selects *alr* or *dadX* as the survival-essential gene; the controllability of *alr* and *dadX* as survival-essential gene is higher than that of the essential gene *asd* selected for *Salmonella* YB1.

3. The hypoxia-specific gene expression cassette designed by the present invention enables the modified bacteria to identify hypoxic regions of the tumor and proliferate in the hypoxic regions of the tumor through precise regulation of oxygen concentration. When the oxygen concentration is lower than 0.8%, the modified bacteria engineered by the present invention can still survive and proliferate, and realizes suicidal lysis in a normal oxygen concentration environment.

4. The bacteria modified with the hypoxia-specific gene expression cassette designed according to the present invention exhibit a low overall mutation rate; in *in vivo* mouse tumor models, the modified bacteria show increased accumulation within tumor tissues and faster clearance from normal organs.

Brief Description of the Drawings

Figure 1 Verification diagram of positive hypoxia promoter clone library identified by agarose gel electrophoresis.

(A) Forward hypoxia promoters *yhbU-S*, *tdcA-S*, *ynfK-S*, *ynfk-E*, *tdcA-E*, *yecH-E*, *cydA-E*, and *focA-E*;
(B) Forward hypoxia promoter *yhbU-E*.

Figure 2 Amplification diagram of verification of essential gene library identified by agarose gel electrophoresis.
Figure 3 Verification diagram of reverse hyperoxia promoter clone library identified by agarose gel electrophoresis.

(A) Reverse hyperoxia promoters *cyoA-S, phoH-S, lldP-S, argT-S, ydcl-S, phoH-E, lldP-E, ydcl-E*;
(B) Reverse hyperoxia promoter *mqo*.

Figure 4 Schematic diagram of a hypoxia-specific gene expression cassette.
Figure 5 Schematic diagram of lambda RED recombinase and CRE recombinase systems.
Figure 6 Identification diagram of the *aroA* gene knockout in *Salmonella* by experiments.

(A) Verification diagram of PCR-amplified product of the *aroA* gene knockout fragment identified by agarose gel electrophoresis;
(B) Verification diagram of PCR-amplification product of target fragment in strain SWT003 identified by agarose gel electrophoresis.

Figure 7 Identification diagram of the *alr* gene knockout in *Salmonella* by experiments.

(A) Verification diagram of PCR-amplified product of the *alr* gene knockout fragment identified by agarose gel electrophoresis;
(B) Verification diagram of PCR-amplification product of target fragment in strain SWT004 identified by agarose gel electrophoresis.

Figure 8 Schematic diagram of constructing plasmid pSWT004 for constructing YBl like *Salmonella* strain SWT005.
Figure 9 Verification diagram of the enzymatic digestion products of plasmid pSWT004 identified by agarose gel electrophoresis.
Figure 10 Verification diagram of PCR-amplified Products of the *cm-pepT-asd-sodA* fragment identified by Agarose gel Electrophoresis.
Figure 11 Verification diagram of PCR-amplified products for identification of *Salmonella* SWT005 by agarose gel electrophoresis.

Figure 12 Results diagram of oxygen adaptability validation tests for YB1-like *Salmonella* SWT005.

(A) Anaerobic culture on LB plates without D-alanine;
(B) Aerobic culture on LB plates without D-alanine.

Figure 13 Verification diagram of PCR-amplified products of plasmid pSWT005 identified by agarose gel electrophoresis.
Figure 14 Verification diagram of PCR-amplified products of strain SWT006 identified by agarose gel electrophoresis.
Figure 15 Results diagram of oxygen adaptability validation tests for strain SWT006.

(A) Anaerobic culture on LB plates without D-alanine;
(B) Aerobic culture on LB plates without D-alanine.

Figure 16 Verification diagram of strain library with insertion of hypoxia-specific gene expression cassette (*alr* as an essential gene) identified by agarose gel electrophoresis.

(A)SWT1001, SWT1002, SWT1003, SWT1004, SWT1005, SWT1006, SWT1007, SWT1008;
(B)SWT1009, SWT1010, SWT1011, SWT1012, SWT1013, SWT1014, SWT1015, SWT1016;
(C)SWT1017, SWT1018, SWT1019, SWT1020, SWT1021, SWT1022, SWT1023, SWT1024;
(D)SWT1025, SWT1026, SWT1027, SWT1028, SWT1029, SWT1030, SWT1031, SWT1032;
(E)SWT1033, SWT1034, SWT1035, SWT1036, SWT1037, SWT1038, SWT1039, SWT1040;
(F)SWT1041, SWT1042, SWT1043, SWT1044, SWT1045, SWT1046, SWT1047, SWT1048;
(G)SWT1049, SWT1050, SWT1051, SWT1052, SWT1053, SWT1054, SWT1055, SWT1056;
(H)SWT1057, SWT1058, SWT1059, SWT1060, SWT1061, SWT1062, SWT1063, SWT1064;
(I)SWT1065, SWT1066, SWT1067, SWT1068, SWT1069, SWT1070, SWT1071, SWT1072;
(J)SWT1073, SWT1074, SWT1075.

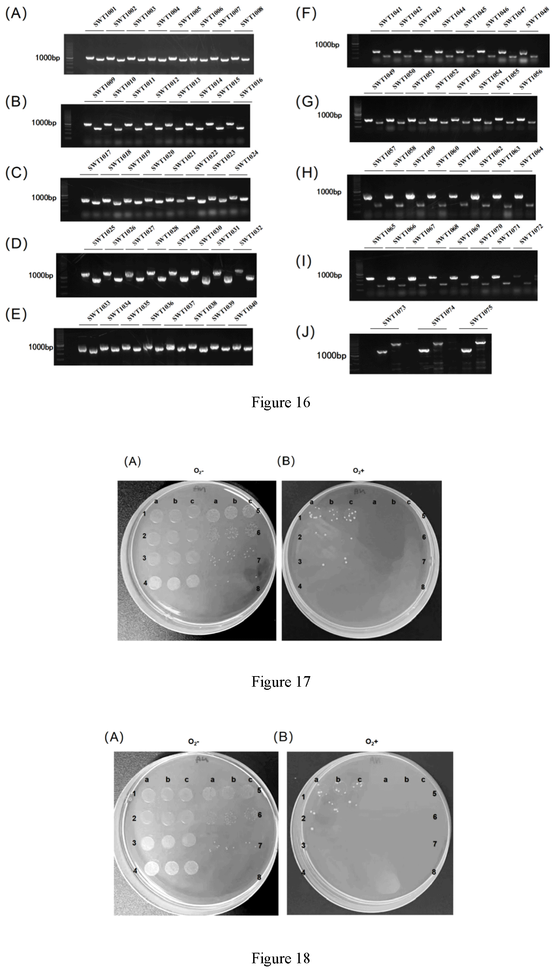

Figure 17 Results diagram of oxygen adaptability validation tests for *Salmonella* strain SWT1001.

(A) Anaerobic culture on LB plates without D-alanine;
(B) Aerobic culture on LB plates without D-alanine.

Figure 18 Results diagram of oxygen adaptability validation tests for *Salmonella* strain SWT1002.

(A) Anaerobic culture on LB plates without D-alanine;
(B) Aerobic culture on LB plates without D-alanine.

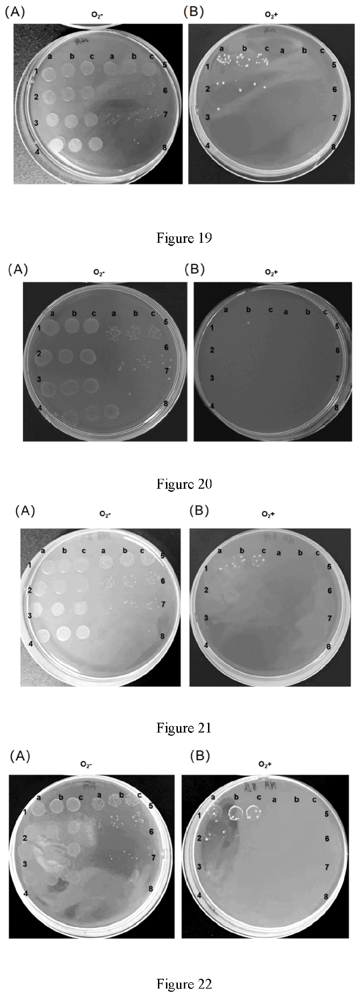

Figure 19 Results diagram of oxygen adaptability validation tests for *Salmonella* strain SWT1004.

(A) Anaerobic culture on LB plates without D-alanine;
(B) Aerobic culture on LB plates without D-alanine.

Figure 20 Results diagram of oxygen adaptability validation tests for *Salmonella* strain SWT1005.

(A) Anaerobic culture on LB plates without D-alanine;
(B) Aerobic culture on LB plates without D-alanine.

Figure 21 Results diagram of oxygen adaptability validation tests for *Salmonella* strain SWT1009.

(A) Anaerobic culture on LB plates without D-alanine;
(B) Aerobic culture on LB plates without D-alanine.

Figure 22 Results diagram of oxygen adaptability validation tests for *Salmonella* strain SWT1010.

(A) Anaerobic culture on LB plates without D-alanine;
(B) Aerobic culture on LB plates without D-alanine.

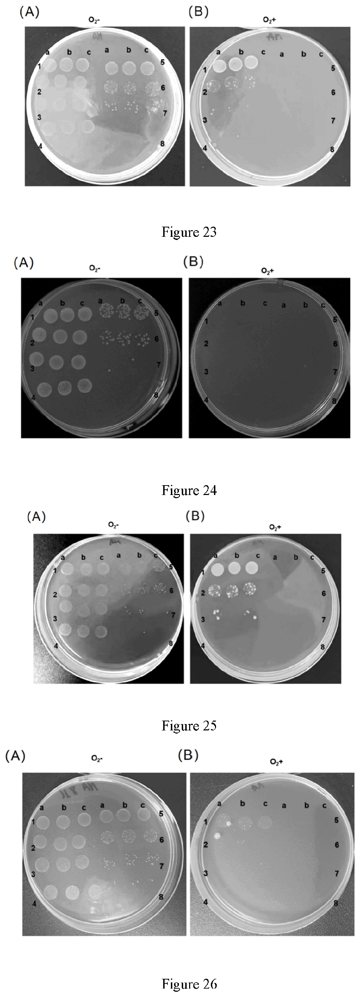

Figure 23 Results diagram of oxygen adaptability validation tests for *Salmonella* strain SWT1012.

(A) Anaerobic culture on LB plates without D-alanine;
(B) Aerobic culture on LB plates without D-alanine.

Figure 24 Results diagram of oxygen adaptability validation tests for *Salmonella* strain SWT1013.

(A) Anaerobic culture on LB plates without D-alanine;
(B) Aerobic culture on LB plates without D-alanine.

Figure 25 Results diagram of oxygen adaptability validation tests for *Salmonella* strain SWT1021.

(A) Anaerobic culture on LB plates without D-alanine;
(B) Aerobic culture on LB plates without D-alanine.

Figure 26 Results diagram of oxygen adaptability validation tests for *Salmonella* strain SWT1025.

(A) Anaerobic culture on LB plates without D-alanine;
(B) Aerobic culture on LB plates without D-alanine.

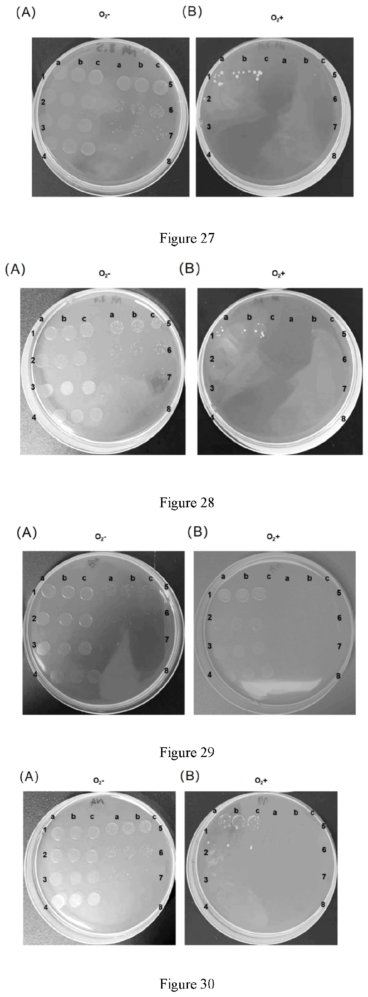

Figure 27 Results diagram of oxygen adaptability validation tests for *Salmonella* strain SWT1033.

(A) Anaerobic culture on LB plates without D-alanine;
(B) Aerobic culture on LB plates without D-alanine.

Figure 28 Results diagram of oxygen adaptability validation tests for *Salmonella* strain SWT1035.

(A) Anaerobic culture on LB plates without D-alanine;
(B) Aerobic culture on LB plates without D-alanine.

Figure 29 Results diagram of oxygen adaptability validation tests for *Salmonella* strain SWT1036.

(A) Anaerobic culture on LB plates without D-alanine;
(B) Aerobic culture on LB plates without D-alanine.

Figure 30 Results diagram of oxygen adaptability validation tests for *Salmonella* strain SWT1038.

(A) Anaerobic culture on LB plates without D-alanine;
(B) Aerobic culture on LB plates without D-alanine.

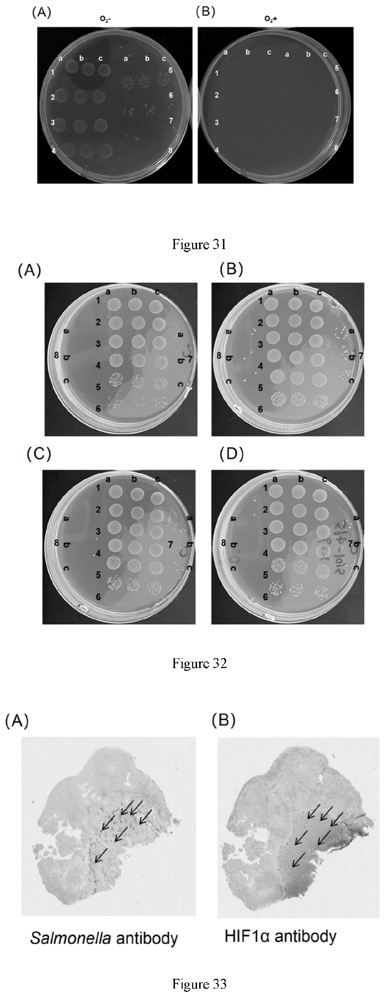

Figure 31 Results diagram of oxygen adaptability validation tests for *Salmonella* strain SWT1063.

(A) Anaerobic culture on LB plates without D-alanine;
(B) Aerobic culture on LB plates without D-alanine.

Figure 32 Results diagram of oxygen adaptability validation tests for various *Salmonella* strains under oxygen concentrations below 0.8%.

(A)SWT1001 cultured on LB plates;
(B)SWT1005 cultured on LB plates;
(C)SWT1009 cultured on LB plates;
(D)SWT1013 cultured on LB plates.

Figure 33 Distribution diagram of *Salmonella* containing a hypoxia-specific gene expression cassette within hypoxic regions of tumors identified by the Immunohistochemical method.

(A)Tumor sections stained with anti-Salmonella antibody, the brown areas indicated by arrows represent positive regions stained with antibody;
(B)Tumor sections stained with anti-HIF1$\alpha$ antibody; the brown areas indicated by arrows represent positive regions stained with antibody.

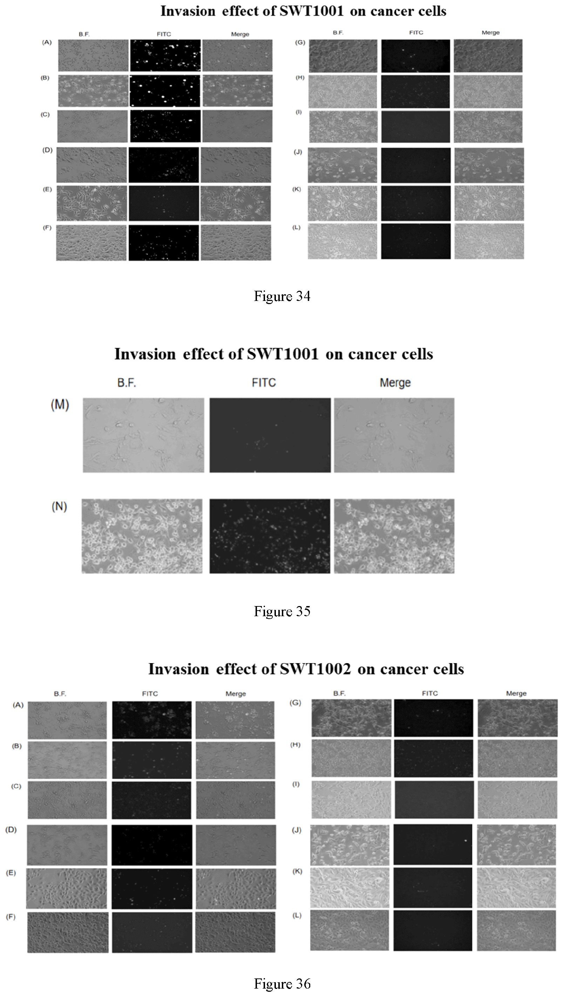

Figure 34 Depicting diagram of the invasive effects of strain SWT1001 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

Figure 35 Supplementary diagram of Figure 34 shows the invasive effects of strain SWT1001 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 36 Depicting diagram of the invasive effects of strain SWT1002 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

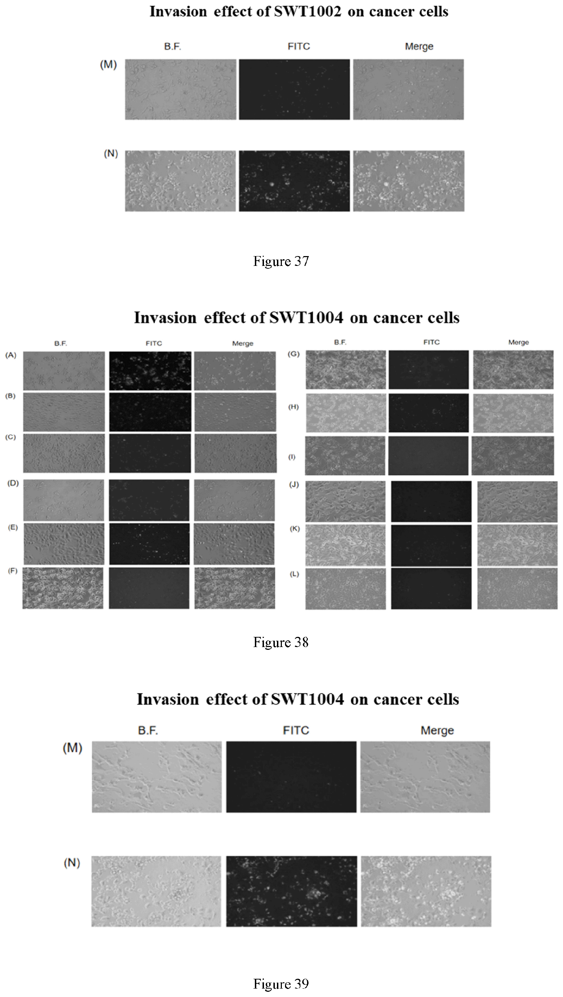

Figure 37 Supplementary diagram of Figure 36 shows the invasive effects of strain SWT1002 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 38 Depicting diagram of the invasive effects of strain SWT1004 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;

(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

Figure 39 Supplementary diagram of Figure 38 shows the invasive effects of strain SWT1004 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

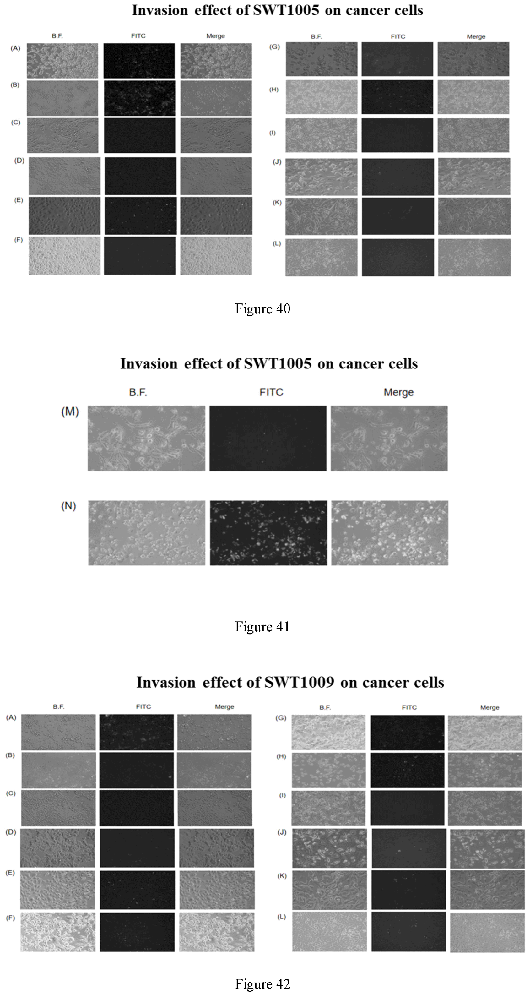

Figure 40 Depicting diagram of the invasive effects of strain SWT1005 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

Figure 41 Supplementary diagram of Figure 40 shows the invasive effects of strain SWT1005 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 42 Depicting diagram of the invasive effects of strain SWT1009 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

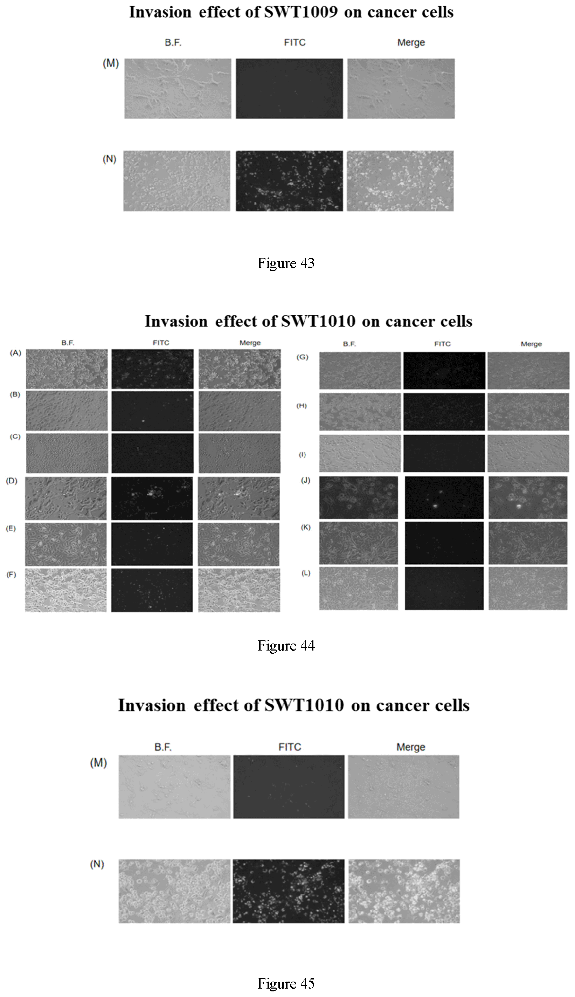

Figure 43 Supplementary diagram of Figure 42 shows the invasive effects of strain SWT1009 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 44 Depicting diagram of the invasive effects of strain SWT1010 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

Figure 45 Supplementary diagram of Figure 44 shows the invasive effects of strain SWT1010 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

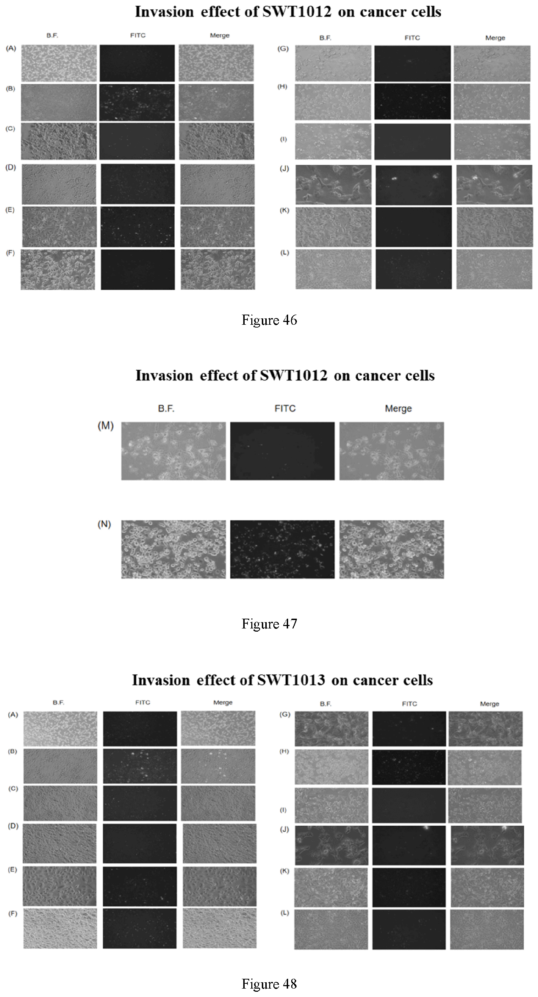

Figure 46 Depicting diagram of the invasive effects of strain SWT1012 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

Figure 47 Supplementary diagram of Figure 46 shows the invasive effects of strain SWT1012 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 48 Depicting diagram of the invasive effects of strain SWT1013 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;

(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

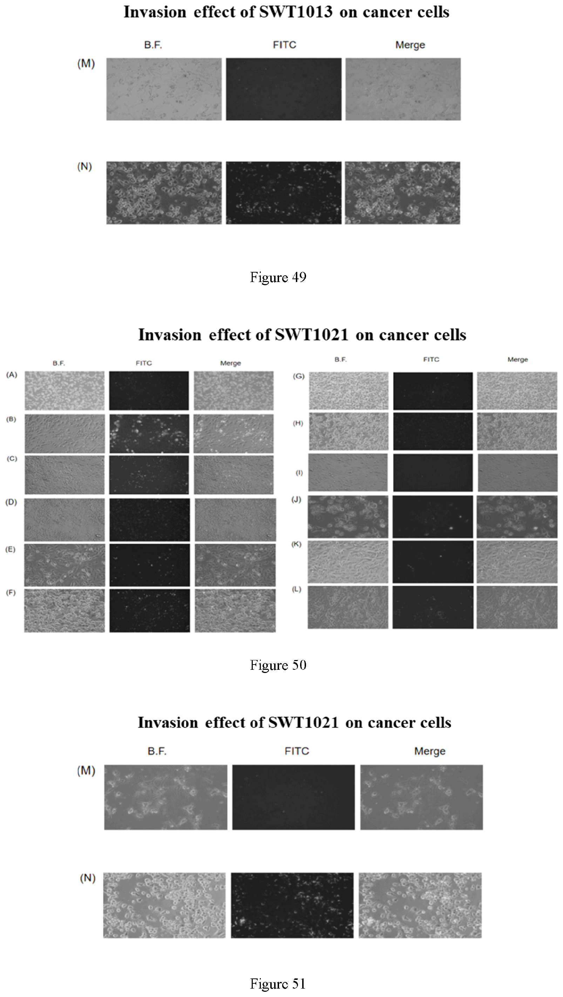

Figure 49 Supplementary diagram of Figure 48 shows the invasive effects of strain SWT1013 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 50 Depicting diagram of the invasive effects of strain SWT1021 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

Figure 51 Supplementary diagram of Figure 50 shows the invasive effects of strain SWT1021 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

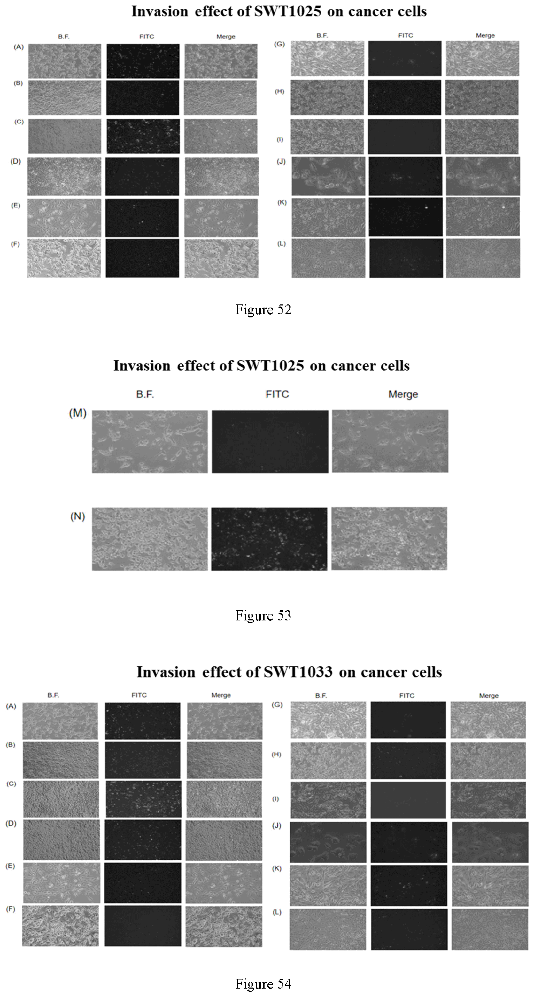

Figure 52 Depicting diagram of the invasive effects of strain SWT1025 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

Figure 53 Supplementary diagram of Figure 52 shows the invasive effects of strain SWT1025 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 54 Depicting diagram of the invasive effects of strain SWT1033 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

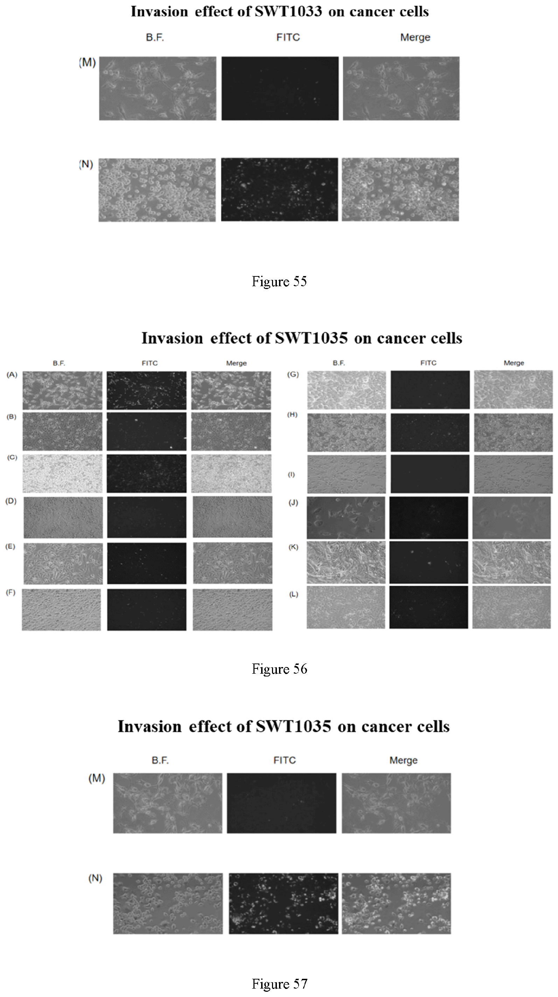

Figure 55 Supplementary diagram of Figure 54 shows the invasive effects of strain SWT1033 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 56 Depicting diagram of the invasive effects of strain SWT1035 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

Figure 57 Supplementary diagram of Figure 56 shows the invasive effects of strain SWT1035 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

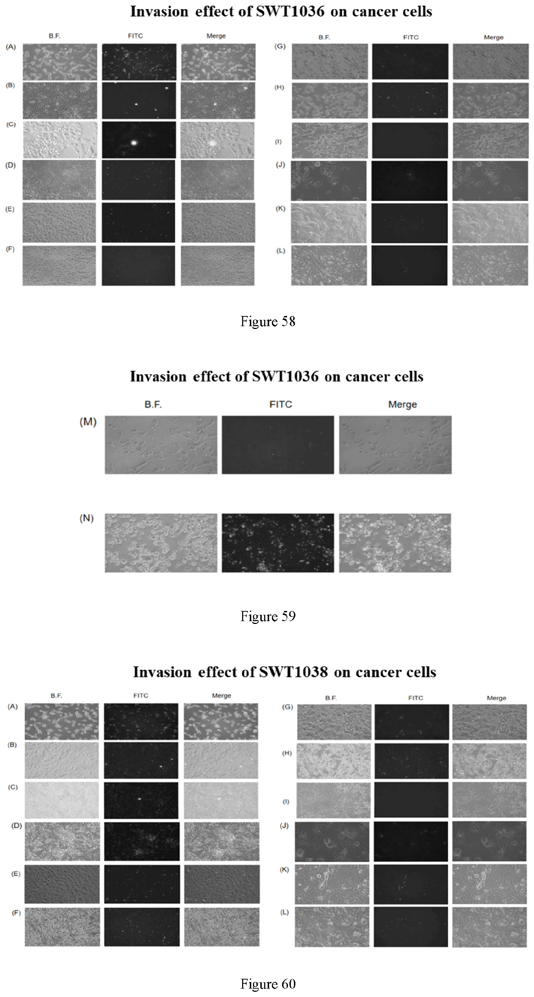

Figure 58 Depicting diagram of the invasive effects of strain SWT1036 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

Figure 59 Supplementary diagram of Figure 58 shows the invasive effects of strain SWT1036 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 60 Depicting diagram of the invasive effects of strain SWT1038 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;
(K)Invasive effect on Pan02 cell line;
(L)Invasive effect on ID8 cell line.

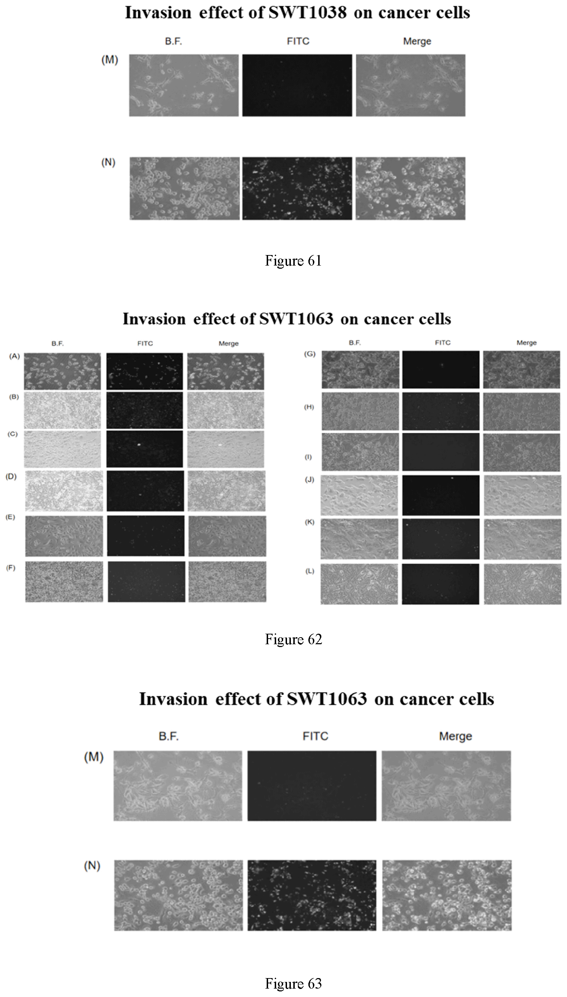

Figure 61 Supplementary diagram of Figure 60 shows the invasive effects of strain SWT1038 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

Figure 62 Depicting diagram of the invasive effects of strain SWT1063 on various cancer cell lines, where B.F. represents bright field, the bright spots shown in FITC indicate fluorescent signals of Salmonella after invasion, and Merge represents the overlay of B.F. and FITC images.

(A)Invasive effect on CT26 cell line;
(B)Invasive effect on EMT6 cell line;
(C)Invasive effect on MB49 cell line;
(D)Invasive effect on RM-1 cell line;
(E)Invasive effect on A549 cell line;
(F)Invasive effect on K7M2 cell line;
(G)Invasive effect on B16F10 cell line;
(H)Invasive effect on MFC cell line;
(I)Invasive effect on SCC7 cell line;
(J)Invasive effect on Hepa1-6 cell line;

(K)Invasive effect on Pan02 cell line;
(L)Invasive effect on ID8 cell line.

Figure 63 Supplementary diagram of Figure 62 shows the invasive effects of strain SWT1063 on different cancer cell lines, where B.F. represents bright field, the bright spots shown in FITC indicate fluorescent signals of Salmonella after invasion, and Merge represents the overlay of B.F. and FITC images.

(M)Invasive effect on Renca cell line;
(N)Invasive effect on Neuro-2a cell line.

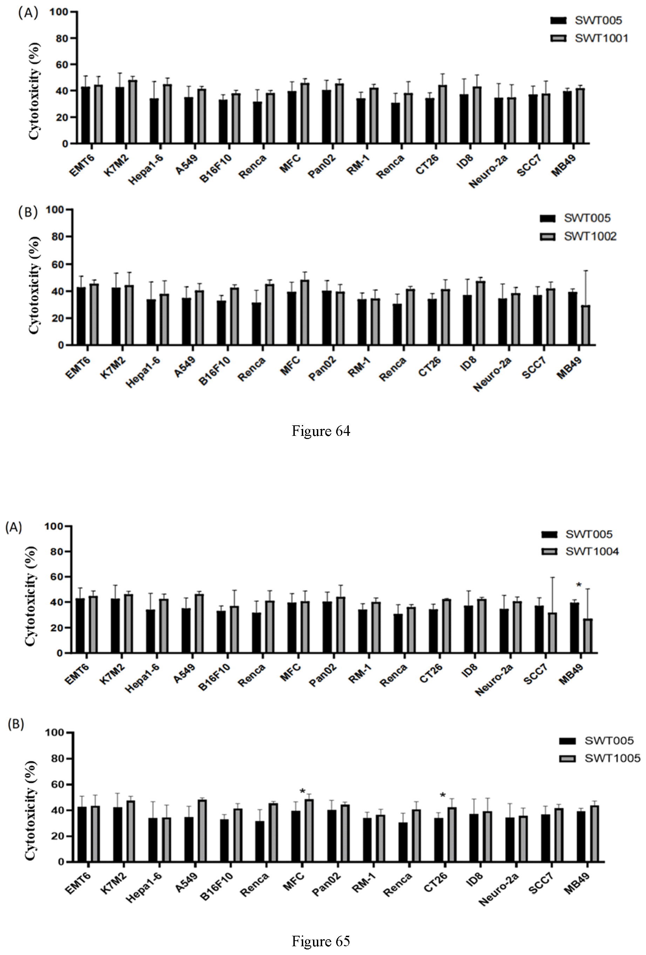

Figure 64 Killing effect diagram of strain SWT005 compared with strains SWT1001 and SWT1002 on EMT6, K7M2, Hepa1-6, A549, B16F10, Renca, MFC, Pan02, RM-1, Renca, CT26, ID8, Neuro-2a, SCC7, and MB49 cells.

(A)Comparison of the killing effects of strain SWT005 and strain SWT1001 on different cancer cell lines;
(B)Comparison of the killing effects of strain SWT005 and strain SWT1002 on different cancer cell lines.

Figure 65 Killing effect diagram of strain SWT005 compared with strains SWT1004 and SWT1005 on EMT6, K7M2, Hepa1-6, A549, B16F10, Renca, MFC, Pan02, RM-1, Renca, CT26, ID8, Neuro-2a, SCC7, and MB49 cells.

(A)Comparison of the killing effects of strain SWT005 and strain SWT1004 on different cancer cell lines;
(B)Comparison of the killing effects of strain SWT005 and strain SWT1005 on different cancer cell lines.

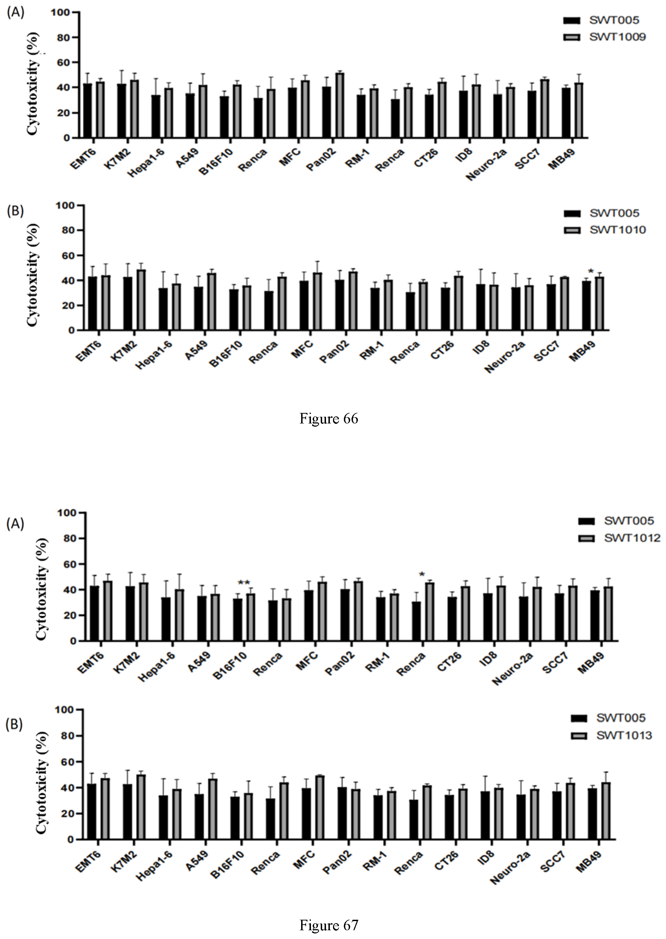

Figure 66 Killing effect diagram of strain SWT005 compared with strains SWT1009 and SWT1010 on EMT6, K7M2, Hepa1-6, A549, B16F10, Renca, MFC, Pan02, RM-1, Renca, CT26, ID8, Neuro-2a, SCC7, and MB49 cells.

(A)Comparison of the killing effects of strain SWT005 and strain SWT1009 on different cancer cell lines;
(B)Comparison of the killing effects of strain SWT005 and strain SWT1010 on different cancer cell lines.

Figure 67 Killing effect diagram of strain SWT005 compared with strains SWT1012 and SWT1013 on EMT6, K7M2, Hepa1-6, A549, B16F10, Renca, MFC, Pan02, RM-1, Renca, CT26, ID8, Neuro-2a, SCC7, and MB49 cells.

(A)Comparison of the killing effects of strain SWT005 and strain SWT1012 on different cancer cell lines;
(B)Comparison of the killing effects of strain SWT005 and strain SWT1013 on different cancer cell lines.

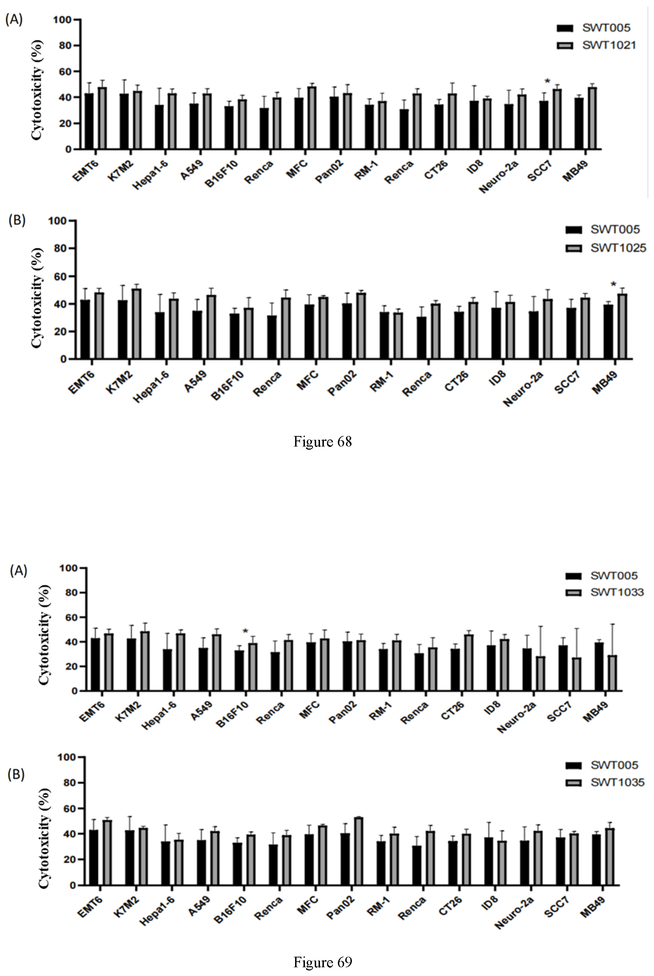

Figure 68 Killing effect diagram of strain SWT005 compared with strains SWT1021 and SWT1025 on EMT6, K7M2, Hepa1-6, A549, B16F10, Renca, MFC, Pan02, RM-1, Renca, CT26, ID8, Neuro-2a, SCC7, and MB49 cells.

(A)Comparison of the killing effects of strain SWT005 and strain SWT1021 on different cancer cell lines;
(B)Comparison of the killing effects of strain SWT005 and strain SWT1025 on different cancer cell lines.

Figure 69 Killing effect diagram of strain SWT005 compared with strains SWT1033 and SWT1035 on EMT6, K7M2, Hepa1-6, A549, B16F10, Renca, MFC, Pan02, RM-1, Renca, CT26, ID8, Neuro-2a, SCC7, and MB49 cells.

(A)Comparison of the killing effects of strain SWT005 and strain SWT1033 on different cancer cell lines;
(B)Comparison of the killing effects of strain SWT005 and strain SWT1035 on different cancer cell lines.

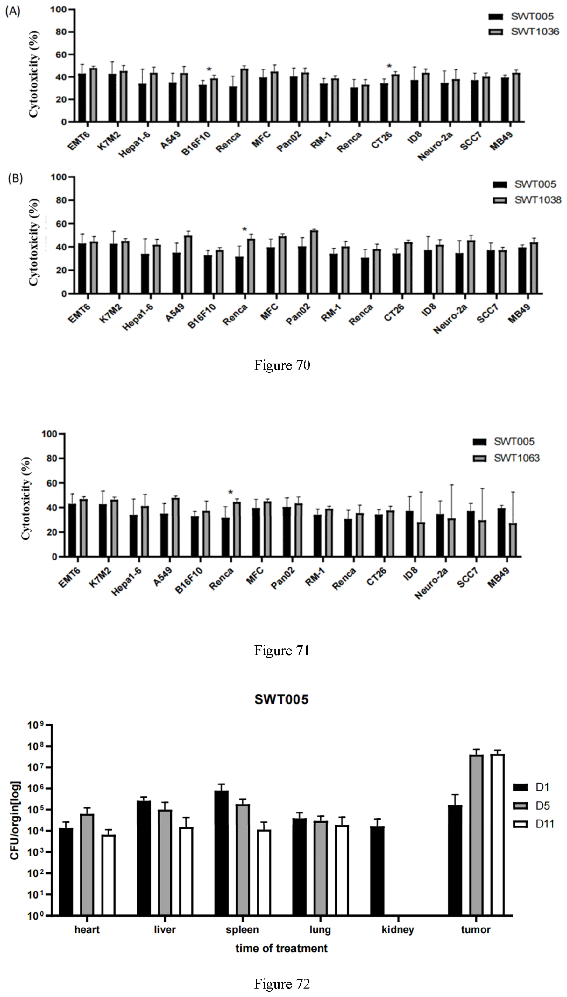

Figure 70 Killing effect diagram of strain SWT005 compared with strains SWT1036 and SWT1038 on EMT6, K7M2, Hepa1-6, A549, B16F10, Renca, MFC, Pan02, RM-1, Renca, CT26, ID8, Neuro-2a, SCC7, and MB49 cells.

(A)Comparison of the killing effects of strain SWT005 and strain SWT1036 on different cancer cell lines;
(B)Comparison of the killing effects of strain SWT005 and strain SWT1038 on different cancer cell lines.

Figure 71 Killing effect diagram of strain SWT005 compared with strain SWT1063 on EMT6, K7M2, Hepa1-6, A549, B16F10, Renca, MFC, Pan02, RM-1, Renca, CT26, ID8, Neuro-2a, SCC7, and MB49 cells.
Figure 72 Targeting evaluation diagram of strain SWT005 in mouse tumor model.
Bacterial counts in Heart, Liver, Spleen, Lung, Kidney, and Tumor at day 1 (D1), day 5 (D5), and day 11 (D11) after intravenous injection of SWT005 via the tail vein in mice.

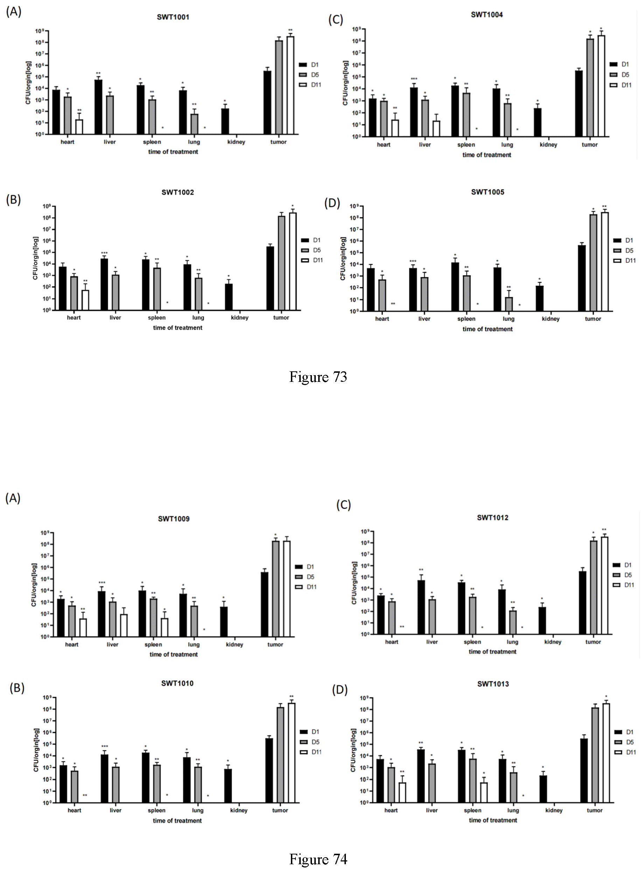

Figure 73 Targeting evaluation diagram of strain SWT1001, SWT1002, SWT1004 and SWT1005 in mouse tumor model.

Bacterial counts in Heart, Liver, Spleen, Lung, Kidney, and Tumor at day 1 (D1), day 5 (D5), and day 11 (D11) after intravenous injection of Salmonella strain via the tail vein in mice. Statistical analysis was performed in comparison to strain SWT005.

(A)Strain SWT1001;
(B)Strain SWT1002;
(C)Strain SWT1004;
(D)Strain SWT1005.

Figure 74 Targeting evaluation diagram of strain SWT1009, SWT1010, SWT1012 and SWT1013 in mouse tumor model.

Bacterial counts in Heart, Liver, Spleen, Lung, Kidney, and Tumor at day 1 (D1), day 5 (D5), and day 11 (D11) after intravenous injection of Salmonella strain via the tail vein in mice. Statistical analysis was performed in comparison to strain SWT005.

(A)Strain SWT1009;
(B)Strain SWT1010;
(C)Strain SWT1012;
(D)Strain SWT1013.

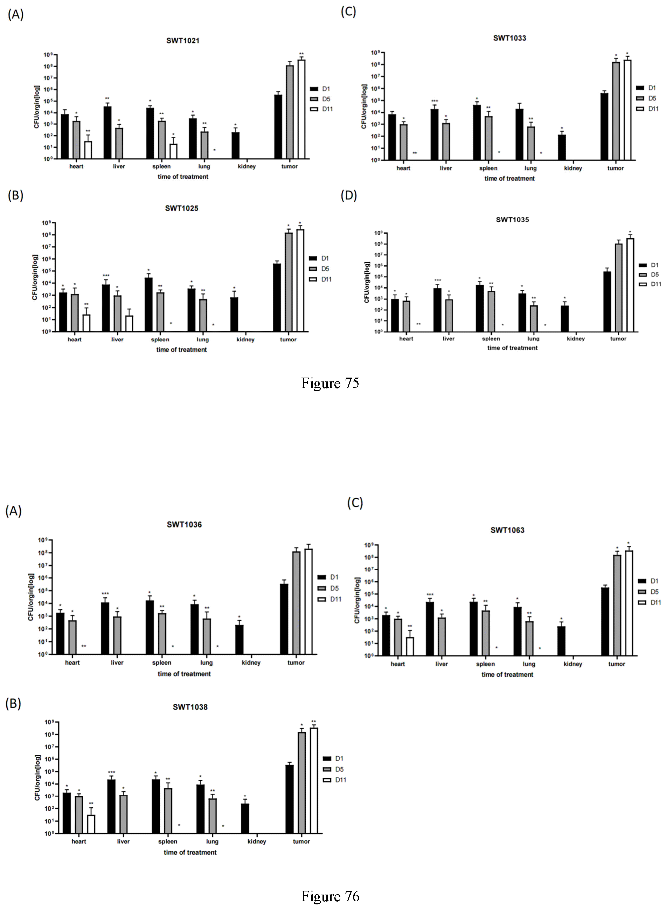

Figure 75 Targeting evaluation diagram of strain SWT1021, SWT1025, SWT1033 and SWT1035 in mouse tumor model.

Bacterial counts in Heart, Liver, Spleen, Lung, Kidney, and Tumor at day 1 (D1), day 5 (D5), and day 11 (D11) after intravenous injection of Salmonella strain via the tail vein in mice. Statistical analysis was performed in comparison to strain SWT005.

(A)Strain SWT1021;
(B)Strain SWT1025;
(C)Strain SWT1033;
(D)Strain SWT1035.

Figure 76 Targeting evaluation diagram of strain SWT1036, SWT1038 and SWT1063 in mouse tumor model.

Bacterial counts in Heart, Liver, Spleen, Lung, Kidney, and Tumor at day 1 (D1), day 5 (D5), and day 11 (D11) after intravenous injection of Salmonella strain via the tail vein in mice. Statistical analysis was performed in comparison to strain SWT005.

(A)Strain SWT1036;
(B)Strain SWT1038;
(C)Strain SWT1063.

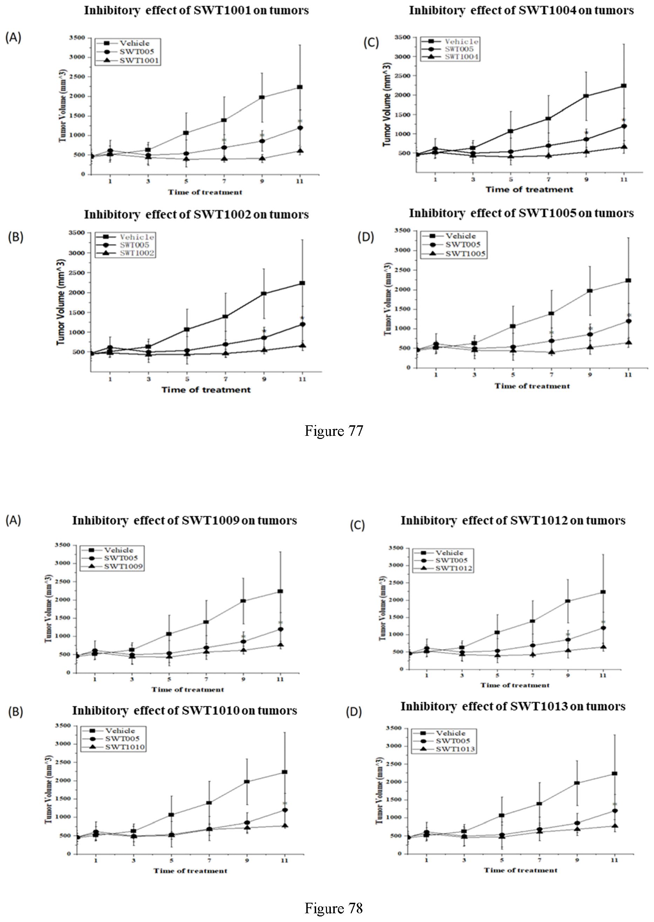

Figure 77 Comparison diagram of tumor suppressive effects between strain SWT005 and strains SWT1001, SWT1002, SWT1004, SWT1005 in mouse tumor model, with Vehicle as the control group.

(A) Tumor suppression comparison graph between strain SWT005 and strain SWT1001 in mouse tumor model;
(B) Tumor suppression comparison graph between strain SWT005 and strain SWT1002 in mouse tumor model;
(C) Tumor suppression comparison graph between strain SWT005 and strain SWT1004 in mouse tumor model;
(D) Tumor suppression comparison graph between strain SWT005 and strain SWT1005 in mouse tumor model.

Figure 78 Comparison diagram of tumor suppressive effects between strain SWT005 and strains SWT1009, SWT1010, SWT1012, SWT1013 in mouse tumor model, with Vehicle as the control group.

(A) Tumor suppression comparison graph between strain SWT005 and strain SWT1009 in mouse tumor model;
(B) Tumor suppression comparison graph between strain SWT005 and strain SWT1010 in mouse tumor model;
(C) Tumor suppression comparison graph between strain SWT005 and strain SWT1012 in mouse tumor model;
(D) Tumor suppression comparison graph between strain SWT005 and strain SWT1013 in mouse tumor model.

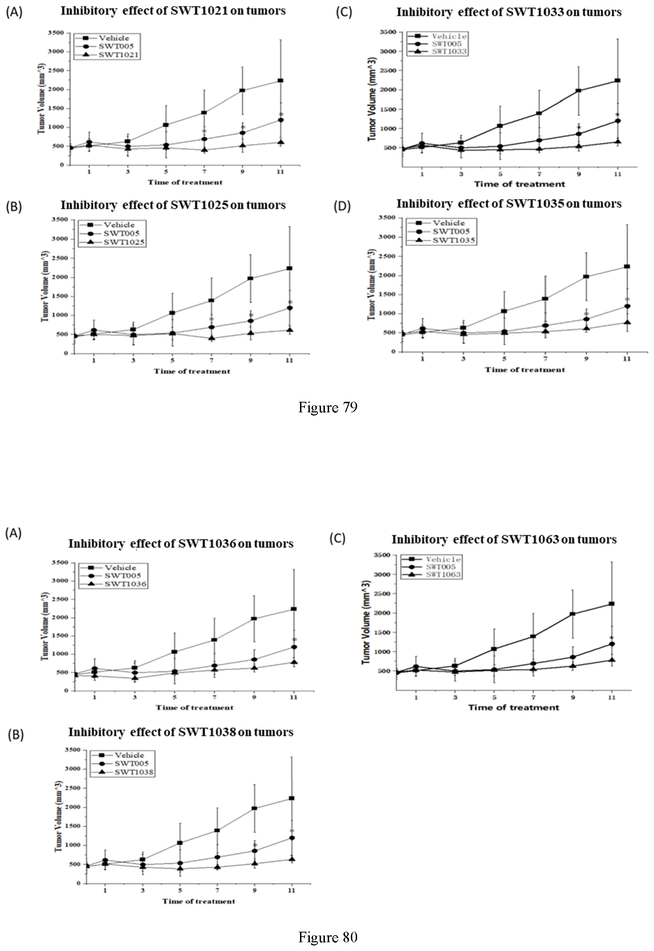

Figure 79 Comparison diagram of tumor suppressive effects between strain SWT005 and strains SWT1021, SWT1025, SWT1033, SWT1035 in mouse tumor model, with Vehicle as the control group.

(A) Tumor suppression comparison graph between strain SWT005 and strain SWT1021 in mouse tumor model;
(B) Tumor suppression comparison graph between strain SWT005 and strain SWT1025 in mouse tumor model;
(C) Tumor suppression comparison graph between strain SWT005 and strain SWT1033 in mouse tumor model;
(D) Tumor suppression comparison graph between strain SWT005 and strain SWT1035 in mouse tumor model.

Figure 80 Comparison diagram of tumor suppressive effects between strain SWT005 and strains SWT1036, SWT1038, SWT1063 in mouse tumor model, with Vehicle as the control group.

(A) Tumor suppression comparison graph between strain SWT005 and strain SWT1036 in mouse tumor model;
(B) Tumor suppression comparison graph between strain SWT005 and strain SWT1038 in mouse tumor model;
(C) Tumor suppression comparison graph between strain SWT005 and strain SWT1063 in mouse tumor model.

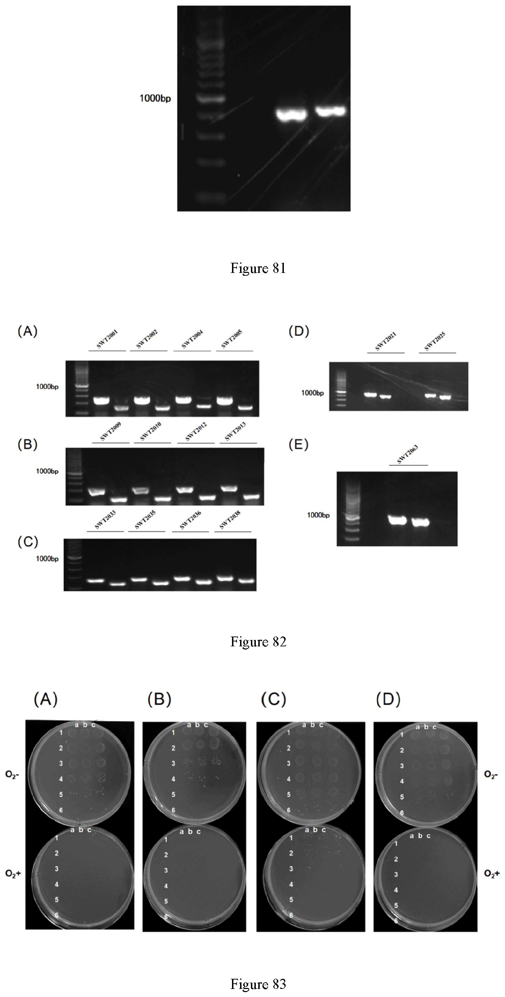

Figure 81 Verification diagram of PCR-amplified products of strain SWT007 identified by agarose gel electrophoresis.
Figure 82 Verification diagram of strain library with insertion of hypoxia-specific gene expression cassette (*dadX* as an essential gene) identified by agarose gel electrophoresis.

(A)SWT2001, SWT2002, SWT2004, SWT2005;
(B)SWT2009, SWT2010, SWT2012, SWT2013;
(C)SWT2033, SWT2035, SWT2036, SWT2038;
(D)SWT2021, SWT2025;
(E)SWT2063.

Figure 83 Results diagram of oxygen adaptability validation tests for strains SWT2001, SWT2002, SWT2004, and SWT2005.

(A) SWT2001 cultured on LB plates without D-alanine, the upper part shows culture under anaerobic conditions, the lower part shows culture under aerobic conditions;
(B) SWT2002 cultured on LB plates without D-alanine, the upper part shows culture under anaerobic conditions, the lower part shows culture under aerobic conditions;
(C) SWT2004 cultured on LB plates without D-alanine, the upper part shows culture under anaerobic conditions, the lower part shows culture under aerobic conditions;
(D) SWT2005 cultured on LB plates without D-alanine, the upper part shows culture under anaerobic conditions, the lower part shows culture under aerobic conditions.

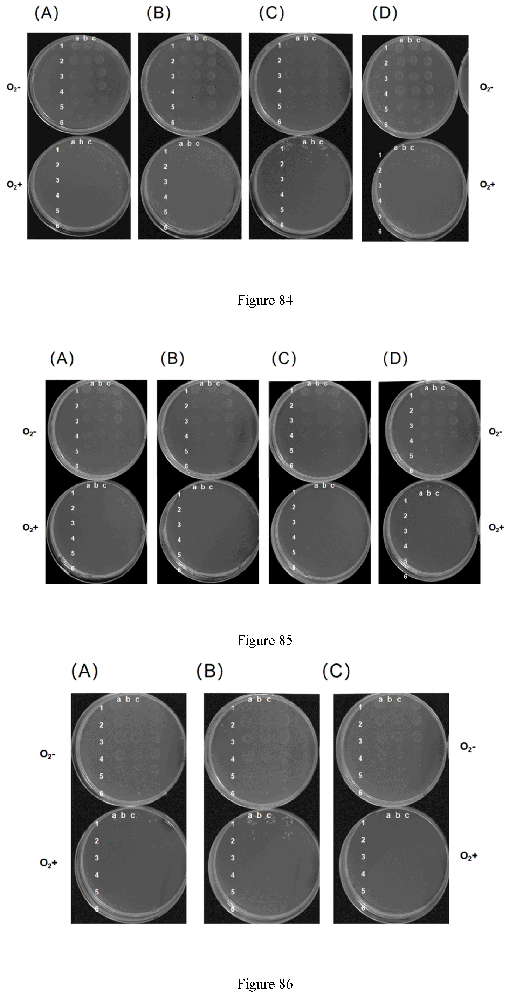

Figure 84 Results diagram of oxygen adaptability validation tests for strains SWT2009, SWT2010, SWT2012, and SWT2013.

(A) SWT2009 cultured on LB plates without D-alanine, the upper part shows culture under anaerobic conditions, the lower part shows culture under aerobic conditions;
(B) SWT2010 cultured on LB plates without D-alanine, the upper part shows culture under anaerobic conditions, the lower part shows culture under aerobic conditions;
(C) SWT2012 cultured on LB plates without D-alanine, the upper part shows culture under anaerobic conditions, the lower part shows culture under aerobic conditions;
(D) SWT2013 cultured on LB plates without D-alanine, the upper part shows culture under anaerobic conditions, the lower part shows culture under aerobic conditions.

Figure 85 Results diagram of oxygen adaptability validation tests for strains SWT2033, SWT2035, SWT2036, and SWT2038.

(A) SWT2033 cultured on LB plates without D-alanine, the upper part shows culture under anaerobic conditions, the lower part shows culture under aerobic conditions;
(B) SWT2035 cultured on LB plates without D-alanine, the upper part shows culture under anaerobic conditions, the lower part shows culture under aerobic conditions;
(C) SWT2036 cultured on LB plates without D-alanine, the upper part shows culture under anaerobic conditions, the lower part shows culture under aerobic conditions;
(D) SWT2038 cultured on LB plates without D-alanine, the upper part shows culture under anaerobic conditions, the lower part shows culture under aerobic conditions.

Figure 86 Results diagram of oxygen adaptability validation tests for strains SWT2021, SWT2025, and SWT2063.

(A) SWT2021 cultured on LB plates without D-alanine, the upper part shows culture under anaerobic conditions, the lower part shows culture under aerobic conditions;
(B) SWT2025 cultured on LB plates without D-alanine, the upper part shows culture under anaerobic conditions, the lower part shows culture under aerobic conditions;
(C) SWT2063 cultured on LB plates without D-alanine, the upper part shows culture under anaerobic conditions, the lower part shows culture under aerobic conditions.

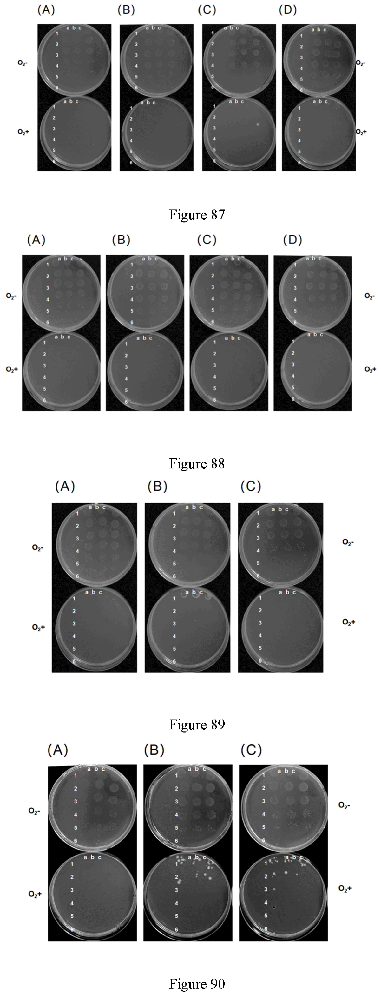

Figure 87 Results diagram of oxygen adaptability validation tests for strains SWT1065, SWT1066, SWT1068 and SWT1069.

(A) SWT1065 cultured on LB plates without D-alanine, the upper part shows culture under anaerobic conditions, the lower part shows culture under aerobic conditions;
(B) SWT1066 cultured on LB plates without D-alanine, the upper part shows culture under anaerobic conditions, the lower part shows culture under aerobic conditions;
(C) SWT1068 cultured on LB plates without D-alanine, the upper part shows culture under anaerobic conditions, the lower part shows culture under aerobic conditions;
(D) SWT1069 cultured on LB plates without D-alanine, the upper part shows culture under anaerobic conditions, the lower part shows culture under aerobic conditions.

Figure 88 Results diagram of oxygen adaptability validation tests for strains SWT1041, SWT1042, SWT1044 and SWT1045.

(A) SWT1041 cultured on LB plates without D-alanine, the upper part shows culture under anaerobic conditions, the lower part shows culture under aerobic conditions;
(B) SWT1042 cultured on LB plates without D-alanine, the upper part shows culture under anaerobic conditions, the lower part shows culture under aerobic conditions;
(C) SWT1044 cultured on LB plates without D-alanine, the upper part shows culture under anaerobic conditions, the lower part shows culture under aerobic conditions;
(D) SWT1045 cultured on LB plates without D-alanine, the upper part shows culture under anaerobic conditions, the lower part shows culture under aerobic conditions.

Figure 89 Results diagram of oxygen adaptability validation tests for strains SWT1008, SWT1016 and SWT1024.

(A) SWT1008 cultured on LB plates without D-alanine, the upper part shows culture under anaerobic conditions, the lower part shows culture under aerobic conditions;
(B) SWT1016 cultured on LB plates without D-alanine, the upper part shows culture under anaerobic conditions, the lower part shows culture under aerobic conditions;
(C) SWT1024 cultured on LB plates without D-alanine, the upper part shows culture under anaerobic conditions, the lower part shows culture under aerobic conditions.

Figure 90 Results diagram of oxygen adaptability validation tests for strains SWT1073, SWT1074 and SWT1075.

(A) SWT1073 cultured on LB plates without D-alanine, the upper part shows culture under anaerobic conditions, the lower part shows culture under aerobic conditions;
(B) SWT1074 cultured on LB plates without D-alanine, the upper part shows culture under anaerobic conditions, the lower part shows culture under aerobic conditions;
(C) SWT1075 cultured on LB plates without D-alanine, the upper part shows culture under anaerobic conditions, the lower part shows culture under aerobic conditions.

<u>Detailed Description of the Embodiments</u>

The present application will be further described in detail below with reference to the accompanying drawings and embodiments. Through these descriptions, the features and advantages of the present application will become clearer and more apparent.

**Example 1: Construction of a hypoxia-specific gene expression cassette using the *alr* gene as the survival -ssential gene**

In this example, the forward hypoxia promoters used were *Salmonella yhbU* (yhbU-S) (SEQ ID NO: 1), *Escherichia coli ynfK* (ynfK-E) (SEQ ID NO: 2), *Salmonella tdcA* (tdcA-S) (SEQ ID NO: 3), *Escherichia coli tdcA* (tdcA-E) (SEQ ID NO: 4), *Escherichia coli yecH* (yecH-E) (SEQ ID NO: 5), *Salmonella ynfK* (ynfK-S) (SEQ ID NO: 6), *Escherichia coli cydA* (cydA-E) (SEQ ID NO: 7), *Escherichia coli focA* (focA-E) (SEQ ID NO: 8), and *Escherichia coli yhbU* (yhbU-E) (SEQ ID NO: 48). The survival-essential gene was the *alr* gene from *Salmonella* (SEQ ID NO: 17). The reverse hyperoxia promoters used were *Salmonella cyoA* (cyoA-S) (SEQ ID NO: 9), *Salmonella phoH* (phoH-S) (SEQ ID NO: 10), *Salmonella lldP* (lldP-S) (SEQ ID NO: 11), *Salmonella argT* (argT-S) (SEQ ID NO: 12), *Salmonella ydcI* (ydcI-S) (SEQ ID NO: 13), *Escherichia coli phoH* (phoH-E) (SEQ ID NO: 14), *Escherichia coli lldP* (lldP-E) (SEQ ID NO: 15), *Escherichia coli ydcI* (ydcI-E) (SEQ ID NO: 16), and *Escherichia coli mqo* (SEQ ID NO: 62) to construct the hypoxia-specific gene expression cassette.

The combinations for the hypoxia-specific gene expression cassette are shown in Table 1.

Table 1. Combination Table of Forward Hypoxia Promoters and Reverse Hyperoxia Promoters

| (wherein the survival-essential gene is the *alr* gene from *Salmonella*) | | | | | | | | |
|---|---|---|---|---|---|---|---|---|
| | cyoA-S | phoH-S | lldP-S | argT-S | ydcI-S | phoH-E | lldP-E | ydcI-E | mqo |
| yhbU-S yhbU-S | pOL1001 | pOL1002 | pOL1003 | pOL1004 | pOL1005 | pOL1006 | pOL1007 | pOL1008 | pOL1073 |
| ynfK-E | pOL1009 | pOL1010 | pOL1011 | pOL1012 | pOL1013 | pOL1014 | pOL1015 | pOL1016 | pOL1074 |
| tdcA-S | pOL1017 | pOL1018 | pOL1019 | pOL1020 | pOL1021 | pOL1022 | pOL1023 | pOL1024 | / |
| tdcA-E | pOL1025 | pOL1026 | pOL1027 | pOL1028 | pOL1029 | pOL1030 | pOL1031 | pOL1032 | / |
| yecH-E | pOL1033 | pOL1034 | pOL1035 | pOL1036 | pOL1037 | pOL1038 | pOL1039 | pOL1040 | pOL1075 |
| ynfK-S | pOL1041 | pOL1042 | pOL1043 | pOL1044 | pOL1045 | pOL1046 | pOL1047 | pOL1048 | / |
| cydA-E | pOL1049 | pOL1050 | pOL1051 | pOL1052 | pOL1053 | pOL1054 | pOL1055 | pOL1056 | / |
| focA-E | pOL1057 | pOL1058 | pOL1059 | pOL1060 | pOL1061 | pOL1062 | pOL1063 | pOL1064 | / |
| yhbU-E | pOL1065 | pOL1066 | pOL1067 | pOL1068 | pOL1069 | pOL1070 | pOL1071 | pOL1072 | / |

**(I) Construction of the Forward Hypoxia Promoter Clone Library**

1. A single colony of wild-type *Salmonella Typhimurium* (strain SWT001, purchased from the China Center for Industrial Culture Collection, CICC) or *Escherichia coli* DH10B (purchased from Shanghai Weidi Biotechnology Co., Ltd.) was picked and inoculated into 5 ml of LB broth. The culture was then incubated in a constant temperature shaking incubator at 37°C and 220 rpm for 16 hours, until the $OD_{600}$ was between 2 and 3.
2. Two microliters (2 μl) of the corresponding bacterial culture were added to a reaction mixture containing the corresponding primers and a high-fidelity PCR enzyme (purchased from Takara), and the mixture was placed in a PCR amplification equipment.
The PCR program was as follows: 95°C for 2 minutes; followed by 30 cycles of 95°C for 30 seconds, 60°C for 30 seconds, and 72°C for 60 seconds; 72°C for 10 minutes; and holding at 4°C for 5 minutes.
3. The amplified PCR product was purified and recovered using a DNA gel purification kit.
4. The recovered product was mixed with a restriction digestion mixture containing restriction enzymes (purchased from NEB) and incubated at 37°C for 1 hour.
5. The digestion product was then purified and recovered using a DNA gel purification kit.

The primers and restriction enzymes used for the different forward hypoxia promoters were as follows:

1. yhbU-S (SEQ ID NO: 1): The promoter was amplified from strain SWT001 using primers SWTO19 and SWTO20. The product was then double-digested with NotI and HindIII and recovered.
2. ynfK-E (SEQ ID NO: 2): The promoter was amplified from *Escherichia coli* strain DH10B using primers SWTO21 and SWTO22. The product was then double-digested with NotI and HindIII and recovered.

3. tdcA-S (SEQ ID NO: 3): The promoter was amplified from strain SWT001 using primers SWTO23 and SWTO24. The product was then double-digested with NotI and HindIII and recovered.

4. tdcA-E (SEQ ID NO: 4): The promoter was amplified from *Escherichia coli* strain DH10B using primers SWTO25 and SWTO26. The product was then double-digested with NotI and HindIII and recovered.

5. yecH-E (SEQ ID NO: 5): The promoter was amplified from *Escherichia coli* strain DH10B using primers SWTO27 and SWTO28. The product was then double-digested with NotI and HindIII and recovered.

6. ynfK-S (SEQ ID NO: 6): The promoter was amplified from strain SWT001 using primers SWTO29 and SWTO30. The product was then double-digested with NotI and HindIII and recovered.

7. cydA-E (SEQ ID NO: 7): The promoter was amplified from *Escherichia coli* strain DH10B using primers SWTO31 and SWTO32. The product was then double-digested with NotI and HindIII and recovered.

8. focA-E (SEQ ID NO: 8): The promoter was amplified from *Escherichia coli* strain DH10B using primers SWTO33 and SWTO34. The product was then double-digested with NotI and HindIII and recovered.

9. yhbU-E (SEQ ID NO: 48): The promoter was amplified from *Escherichia coli* strain DH10B using primers SWTO82 and SWTO83. The product was then double-digested with NotI and HindIII and recovered.

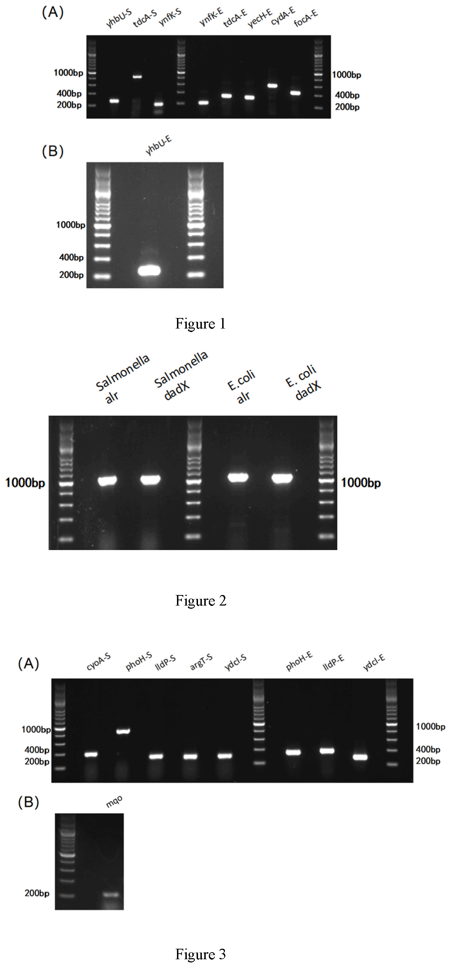

The results of the verification of the forward hypoxia promoter clone library by agarose gel electrophoresis are shown in Figure 1(A) and Figure 1(B).

## (II) Construction of the Survival-Essential Gene Library

1. A single colony of wild-type *Salmonella Typhimurium* (strain SWT001, purchased from the China Center for Industrial Culture Collection, CICC) or *Escherichia coli* DH10B (purchased from Shanghai Weidi Biotechnology Co., Ltd.) was picked and inoculated into 5 ml of LB broth. The culture was then incubated in a constant temperature shaking incubator at 37°C and 220 rpm for 16 hours.

2. Two microliters (2 μl) of the corresponding bacterial culture were added to a reaction mixture containing the corresponding primers and a high-fidelity PCR enzyme, and the mixture was placed in a PCR amplification equipment.

The PCR program was as follows: 95°C for 2 minutes; followed by 30 cycles of 95°C for 30 seconds, 60°C for 30 seconds, and 72°C for 60 seconds; 72°C for 10 minutes; and holding at 4°C for 5 minutes.

3. The amplified PCR product was purified and recovered using a DNA gel purification kit.

4. The recovered product was mixed with a restriction digestion mixture containing restriction enzymes and incubated at 37°C for 1 hour.

5. The digestion product was then purified and recovered using a DNA gel purification kit.

The different survival-essential genes were as follows:

1. *Salmonella alr* gene (SEQ ID NO: 17): The gene was amplified from strain SWT001 using primers SWTO35 and SWTO36 The product was then double-digested with HindIII and XhoI and recovered.

2. *Escherichia coli alr* gene (SEQ ID NO: 21): The gene was amplified from *Escherichia coli* strain DH10B using primers SWTO37 and SWTO38. The product was then double-digested with HindIII and XhoI and recovered.

3. *Salmonella dadX* gene (SEQ ID NO: 19): The gene was amplified from strain SWT001 using primers SWTO39 and SWTO40. The product was then double-digested with HindIII and XhoI and recovered.

4. *Escherichia coli dadX* gene (SEQ ID NO: 23): The gene was amplified from *Escherichia coli* strain DH10B using primers SWTO41 and SWTO42. The product was then double-digested with HindIII and XhoI and recovered.

The result of the verification of the survival-essential gene library by agarose gel electrophoresis is shown in Figure 2.

## (III) Construction of the Reverse Hyperoxia Promoter Library

1. A single colony of wild-type *Salmonella Typhimurium* (strain SWT001, purchased from the China Center for Industrial Culture Collection, CICC) or *Escherichia coli* DH10B (purchased from Shanghai Weidi Biotechnology Co., Ltd.) was picked and inoculated into 5 ml of LB broth. The culture was then incubated in a constant temperature shaking incubator at 37°C and 220 rpm for 16 hours.

2. Two microliters (2 µl) of the corresponding bacterial culture were added to a reaction mixture containing the corresponding primers and a high-fidelity PCR enzyme, and the mixture was placed in a PCR amplification equipment.

The PCR program was as follows: 95°C for 2 minutes; followed by 30 cycles of 95°C for 30 seconds, 60°C for 30 seconds, and 72°C for 60 seconds; 72°C for 10 minutes; and holding at 4°C for 5 minutes.

3. The amplified PCR product was purified and recovered using a DNA gel purification kit.

4. The recovered product was mixed with a restriction digestion mixture containing restriction enzymes and incubated at 37°C for 1 hour.

5. The digestion product was then purified and recovered using a DNA gel purification kit.

The primers and restriction enzymes used for the different reverse hyperoxia promoters were as follows:

1. cyoA-S (SEQ ID NO: 9): The promoter was amplified from strain SWT001 using primers SWTO43 and SWTO44. The product was then double-digested with XhoI and PstI and recovered.

2. phoH-S (SEQ ID NO: 10): The promoter was amplified from strain SWT001 using primers SWTO45 and SWTO46. The product was then double-digested with XhoI and PstI and recovered.

3. lldP-S (SEQ ID NO: 11): The promoter was amplified from strain SWT001 using primers SWTO47 and SWTO48. The product was then double-digested with XhoI and PstI and recovered.

4. argT-S (SEQ ID NO: 12): The promoter was amplified from strain SWT001 using primers SWTO49 and SWTO50. The product was then double-digested with XhoI and PstI and recovered.

5. ydcI-S (SEQ ID NO: 13): The promoter was amplified from strain SWT001 using primers SWTO51 and SWTO52. The product was then double-digested with XhoI and PstI and recovered.

6. phoH-E (SEQ ID NO: 14): The promoter was amplified from strain DH10B using primers SWTO53 and SWTO54. The product was then double-digested with XhoI and PstI and recovered.

7. lldP-E (SEQ ID NO: 15): The promoter was amplified from strain DH10B using primers SWTO55 and SWTO56. The product was then double-digested with XhoI and PstI and recovered.

8. ydcI-E (SEQ ID NO: 16): The promoter was amplified from strain DH10B using primers SWTO57 and SWTO58. The product was then double-digested with XhoI and PstI and recovered.

9. mqo (SEQ ID NO: 62): The promoter was amplified from strain DH10B using primers SWTO7 and SWTO8. The product was then double-digested with XhoI and PstI and recovered.

The results of the verification of the reverse hyperoxia promoter clone library by agarose gel electrophoresis are shown in Figure 3(A) and Figure 3(B).

**(IV) Construction of a Combinatorial Library of Forward Hypoxia Promoters, Survival-Essential Genes, and Reverse Hyperoxia Promoters**

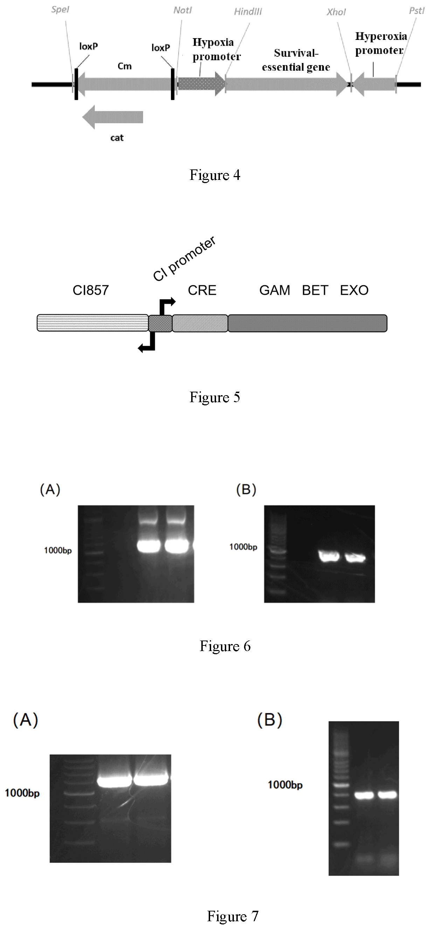

The composition of the hypoxia-specific gene expression cassette is shown in Figure 4. It is composed of a forward hypoxia promoter, a survival-essential gene, and a reverse hyperoxia promoter, arranged in sequence.

As shown in Table 1, the cloned library was formed by combining the following sequences: the forward hypoxia promoter library digested with NotI and HindIII; the survival-essential *Salmonella alr* gene (SEQ ID NO: 17) digested with HindIII and XhoI; and the reverse hyperoxia promoter library digested with XhoI and PstI. These were ligated into the plasmid pSWT003 vector (vector backbone: pBlueScript SK(+), purchased from Biofeng), which had been digested with SpeI and PstI, and the plasmid pSWT007 vector (containing bilateral co-directional *loxP* sequences and a DNA fragment of the chloramphenicol resistance gene (SEQ ID NO: 61)), which had been digested with SpeI and NotI.

The products mentioned above obtained by primer amplification from *Salmonella* SWT001 or *Escherichia coli* DH10B followed by enzymatic digestion and recovery, were subjected to combinatorial ligation via an enzymatic ligation reaction. The ligation products were spread onto LB plates containing 25 µg/mL chloramphenicol to obtain the corresponding plasmids shown in Table 1.

Construction of the strains of the present invention is as follows:

**1. Wild-type *Salmonella typhimurium* (SWT001)**

Wild-type *Salmonella typhimurium* (SWT001) was purchased from the China Center of Industrial Culture Collection (CICC).

## 2. Construction of *Salmonella* (SWT002) containing a temperature-inducible Lambda-RED recombinase and a *loxP*-CRE enzyme system

The Lambda-RED recombination system is widely used for homologous recombination in Gram-negative bacteria. In the present invention, this system consists of plasmid pSWT001. As shown in Figure 5, plasmid pSWT001 contains a Lambda-RED recombinase module (SEQ ID NO: 60) (functionally like the plasmid vector *p*sim6 from Biofeng) and a *loxP*-Cre recombinase module (SEQ ID NO: 60) (functionally like the plasmid vector 705-Cre from Gene Bridges). The Lambda-RED recombinase module is composed of three recombinases: EXO, BET, and GAM, and the recombinases are controlled by the CI857 temperature-sensitive regulator. Therefore, they cannot be expressed under culture conditions at 32°C and are expressed only at temperatures higher than 37°C. Under temperature-induced conditions, the EXO recombinase cleaves the 5' end of double-stranded linear DNA, creating single-stranded DNA with a protruding 3' end. The single-stranded DNA is bound by the BET protein and protected from degradation by other nucleases. The function of GAM is to inhibit bacterial endogenous nuclease activity. The homology arms for homologous recombination are approximately 35-50 bp in length and are added to both sides of the DNA fragment to be recombined via PCR. The advantage of this technology is that it precisely targets the region of interest in the bacterial chromosome without causing extra mutations. The outermost sides of the recombined double-stranded DNA fragment include the homology arm sequences for the recombination site, while the interior contains two *loxP* sequences in the same orientation, totaling 34 bp. Located between the two co-directional *loxP* sites is a chloramphenicol resistance gene used for screening recombinant bacteria. After successful recombination, the CRE enzyme system carried on the pSWT001 plasmid (also controlled by the CI857 temperature-sensitive regulator) specifically recognizes the *loxP* sequences and creates a cut within the sequence between the co-directional *loxP* sites, leaving one *loxP* sequence behind, thereby eliminating the chloramphenicol resistance gene. Repeating the above operations allows for continuous gene knockout and knock-in.

The specific operational steps are as follows:

(1) Strain SWT001 was streaked onto an LB plate and cultured overnight in a constant temperature incubator at 37°C;

(2) A single colony was picked and inoculated into 5 mL of LB liquid medium, then placed in a constant temperature shaker at 37°C and 220 rpm for 16 hours;

(3) The culture was inoculated into fresh LB liquid medium at a ratio of 1:100 and cultured for a further 2-3 hours until the bacterial density reached $OD_{600}$ = 0.3, then placed on ice for 1 hour;

(4) The bacterial cells were washed 3 times with sterilized purified water;

(5) The recovered bacterial cells were mixed with 10 ng of plasmid pSWT001 and electroporated at a voltage of 1.8 kV;

(6) The electroporated bacterial cells were spread onto LB plates containing 100 μg/mL ampicillin sodium and cultured overnight in a constant temperature incubator at 32°C until single colonies grew. The resulting strain was named SWT002.

## 3. Construction of *aroA* Gene-Knockout Attenuated *Salmonella* (SWT003)

(1) A single colony of SWT002 was picked and inoculated into 5 mL of LB liquid medium containing 100 μg/mL ampicillin sodium, and cultured in a constant temperature shaker at 32°C and 220 rpm for 16 hours.

(2) The culture was inoculated at a ratio of 1:100 into fresh LB liquid medium containing 100 μg/mL ampicillin sodium and cultured for a further 2-3 hours. When the bacterial density reached an $OD_{600}$ of 0.3, the culture flask was placed in a 42°C water bath and incubated with shaking for 15 minutes, then placed on ice for 1 hour.

(3) The bacterial cells were washed 3 times with sterilized purified water.

(4) Preparation of PCR products using primers SWTO1 and SWTO2:

Primers SWTO1 and SWTO2 were mixed with plasmid pSWT002 (containing bilateral co-directional *loxP* sequences with a chloramphenicol resistance gene in between) and a high-fidelity PCR amplification enzyme system, and placed into PCR amplification equipment.

The amplification program was: 95°C for 2 minutes; 30 cycles of 95°C for 30 seconds, 60°C for 30 seconds, and 72°C for 60 seconds; followed by 72°C for 10 minutes; and 4°C for 5 minutes.

The amplified PCR products were verified by agarose gel electrophoresis. The verification results of the PCR amplification products of the *aroA* gene knockout fragment using SWTO1 and SWTO2 are shown in Figure 6A.

The products were purified and recovered using a DNA gel extraction system. The concentration and purity of the recovered PCR products were determined using a nanodrop.

(5) The recovered bacterial cells were mixed with 100 ng of the PCR products generated by SWTO1 and SWTO2, and electroporated at a voltage of 1.8 kV.

(6) The electroporated bacterial cells were spread onto plates containing 25 μg/mL chloramphenicol and cultured overnight in a constant temperature incubator at 32°C until single colonies grew.

(7) Positive clones were identified by colony PCR. Primers SWTO4 and SWTO6, and SWTO3 and SWTO5 were used to verify the insertion of the chloramphenicol resistance gene.

The PCR amplification products of the target fragment of strain SWT003 were verified by agarose gel electrophoresis, and the verification results are shown in Figure 6B.

(8) The positive single colonies were inoculated into 5 mL of LB medium and cultured in a constant temperature shaker at 37°C and 220 rpm for 16 hours to allow the CRE enzyme to function and excise the chloramphenicol resistance gene.

**4. Construction of *alr* Gene-Knockout Attenuated *Salmonella* (SWT004)**

(1) A single colony of SWT003 was picked and inoculated into 5 mL of LB liquid medium, and cultured in a constant temperature shaker at 32°C and 220 rpm for 16 hours.

(2) The culture was inoculated at a ratio of 1:100 into fresh LB liquid medium and cultured for a further 2-3 hours. When the bacterial density reached an $OD_{600}$ of 0.3, the culture flask was placed in a 42°C water bath and incubated with shaking for 15 minutes, then placed on ice for 1 hour.

(3) The bacterial cells were washed 3 times with sterilized purified water.

(4) Preparation of PCR products using primers SWTO70 and SWTO71:

Primers SWTO70 and SWTO71 were mixed with plasmid pSWT002 and a high-fidelity PCR amplification enzyme system, and placed into PCR amplification equipment.

The amplification program was: 95°C for 2 minutes; 30 cycles of 95°C for 30 seconds, 60°C for 30 seconds, and 72°C for 60 seconds; followed by 72°C for 10 minutes; and 4°C for 5 minutes.

The amplified PCR products were purified and recovered using a DNA gel extraction system. The concentration and purity of the recovered PCR products were determined using a nanodrop.

(5) The recovered bacterial cells were mixed with 100 ng of the PCR products generated by SWTO70 and SWTO71, and electroporated at a voltage of 1.8 kV.

The PCR amplification products of the *alr* gene knockout fragment using SWTO70 and SWTO71 were verified by agarose gel electrophoresis, and the verification results are shown in Figure 7A.

(6) The electroporated bacterial cells were spread onto plates containing 25 μg/mL chloramphenicol and cultured overnight in a constant temperature incubator at 32°C until single colonies grew.

(7) Positive clones were identified by colony PCR. Primers SWTO72 and SWTO5, and SWTO6 and SWTO73 were used to verify the insertion of the chloramphenicol resistance gene.

The PCR amplification products of the target fragment of strain SWT004 were verified by agarose gel electrophoresis, and the verification results are shown in Figure 7B (primers used: SWTO72, SWTO5, SWTO6, SWTO73).

(8) The positive single colonies were inoculated into 5 mL of LB medium and cultured in a constant temperature shaker at 37°C and 220 rpm for 16 hours to allow the CRE enzyme to function and excise the chloramphenicol resistance gene.

**5. Construction of YB1-like *Salmonella* (SWT005)**

To investigate *a* more optimal combination of oxygen-regulated promoters, the present invention constructed the YB1 *Salmonella* strain described in Chinese Patent CN104471057B to serve as a prior art strain for comparison with the strains constructed in the present invention.

The YB1-like *Salmonella* strain includes an *aroA* gene defect and an oxygen regulation system composed of the forward hypoxia promoter *pepT*, the essential gene *asd*, and the reverse hyperoxia promoter *sodA*.

**(1) Construction of Plasmid pSWT004**

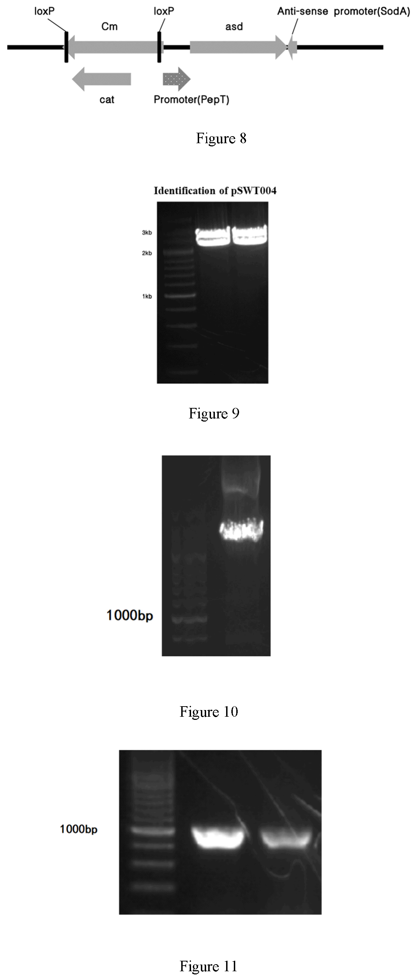

① Construction of plasmid pSWT004 is shown in Figure 8. It contains a chloramphenicol resistance gene (*cm*) flanked by *loxP* sequence*s* (SEQ ID NO: 43), the *pepT* promoter (SEQ ID NO: 44), the *asd* gene (SEQ ID NO: 45), and the reverse *sodA* promoter (SEQ ID NO: 47).

② Plasmid pSWT002 was digested with NotI, and a 1130 bp fragment was recovered. Strain SWT001 was amplified using primers SWTO11 and SWTO12; the product was double-digested with NotI and HindIII and recovered.

Strain SWT001 was amplified using primers SWTO13 and SWTO14; the product was double-digested with XhoI and HindIII and recovered.

Primers SWTO15 and SWTO16 were directly annealed to generate XhoI and PstI overhangs and recovered.

③ Plasmid pSWT003 (vector backbone) was double-digested with NotI and PstI. The five fragments were ligated using T4 DNA ligase. The product was spread onto LB plates containing both ampicillin and chloramphenicol. Single colonies were screened and named pSWT004 (YB1-like construction plasmid, including *cm-pepT-asd-sodA*).

④ Plasmid pSWT004 was extracted, and 100 ng was mixed with SacI and KpnI endonuclease systems and incubated at 37°C for 1 hour. Identification was performed via agarose gel electrophoresis, as shown in Figure 9.

**(2) Construction of Recombinant YB1-like *Salmonella* SWT005 (YB1-like)**

Plasmid pSWT004 was amplified using primers SWTO17 and SWTO18 and recovered. This fragment was recombined into SWT003 to construct the YB1-like *Salmonella* SWT005.

① A single colony of SWT003 was picked and inoculated into 5 mL of LB liquid medium and cultured in a constant temperature shaker at 32°C and 220 rpm for 16 hours.

② the culture was inoculated at a ratio of 1:100 into fresh LB liquid medium and cultured for a further 2-3 hours until the bacterial density reached $OD_{600} = 0.3$. The culture flask was placed in a 42°C water bath and incubated with shaking for 15 minutes, then placed on ice to rest for 1 hour.

③ the bacterial cells were washed 3 times with sterilized purified water and set aside.

④ pSWT004 was amplified using primers SWTO17 and SWTO18, purified, and recovered. The recovered PCR product of the *cm-pepT-asd-sodA* fragment generated by SWTO17 and SWTO18 was verified by agarose gel electrophoresis. The verification results are shown in Figure 10. The DNA concentration and purity were determined using a nanodrop.

⑤ the recovered bacterial cells were mixed with 100 ng of the PCR product and electroporated at a voltage of 1.8 kV.

⑥ the electroporated bacterial cells were spread onto plates containing 25 μg/mL chloramphenicol and 100 μg/mL DAP (diaminopimelic acid), and cultured overnight in a constant temperature incubator at 32°C until single colonies grew.

⑦ Positive clones were identified by colony PCR. Primers SWTO93 and SWTO5, and SWTO94 and SWTO95 were used to verify the insertion of the chloramphenicol resistance gene. The results of the PCR amplification product verification for *Salmonella* SWT005 (using SWTO93 and SWTO5, SWTO94 and SWTO95) by agarose gel electrophoresis are shown in Figure 11.

**(3) Oxygen Adaptability Verification of YB1-like *Salmonella* SWT005**

① A single colony of YB1-like *Salmonella* SWT005 was picked and inoculated into 5 mL of LB liquid medium containing 100 μg/mL DAP (diaminopimelic acid) and cultured in a constant temperature shaker at 37°C and 200 rpm for 16 hours.

② After culturing, the strain was diluted 10-fold, and the absorbance (OD value) at 600 nm was measured.

③ the volume of bacterial liquid (in microliters) corresponding to 1 OD of bacteria was calculated according to the following formula, and deionized water was added to reach 1 mL:

$$\text{Volume of bacterial culture for 1 OD} = 1/(\text{Absorbance} \times 10) \times 1000$$

④ 10 μL of the above bacterial suspension was spotted onto two LB plates without DAP, labeled as "1". This was repeated three times, labeled "a", "b", and "c".

⑤ After a 10-fold serial dilution, 10 μL of the diluted solution was spotted onto the medium, labeled as "2".

⑥ This 10-fold serial dilution was continued until label "8".

⑦ One plate was placed in an anaerobic jar at 37°C. The operation involved placing an anaerobic gas generating bag (Brand: Mitsubishi, Japan, Cat. No.: D-119) into a 7.0 L sealed culture jar (Brand: Mitsubishi, Japan, Cat. No.: D-112) and culturing for 24 hours in an anoxic environment (oxygen concentration less than 0.01%). The other plate was cultured for 24 hours in an atmospheric environment (21% oxygen concentration) at 37°C.

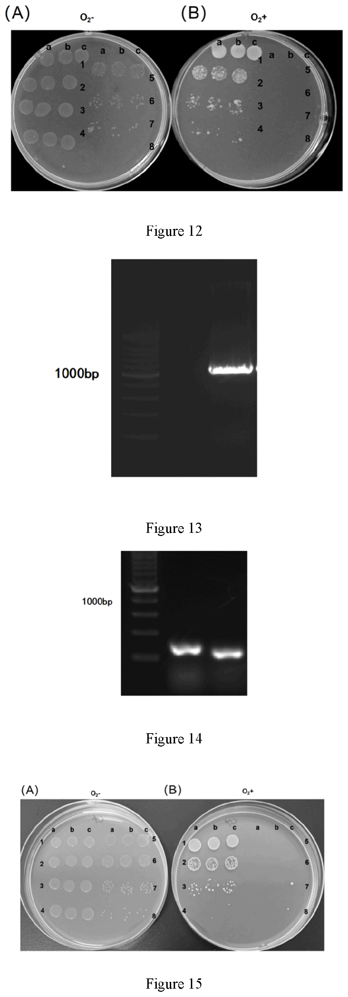

The results of the oxygen adaptability verification test for YB1-like *Salmonella* SWT005 are shown in Figure 12.

From the oxygen adaptability verification data of YB1-like *Salmonella* SWT005, it can be concluded that: YB1-like *Salmonella* SWT005 grew normally on LB plates without DAP under anoxic conditions (as shown in Figure 12(A)). Under aerobic (atmospheric) conditions, distinct strain growth was observed at concentration 1 ($10^{-2}$ OD), concentration 2 ($10^{-3}$ OD), concentration 3 ($10^{-4}$ OD), and concentration 4 ($10^{-5}$ OD) (as shown in Figure 12(B)).

Therefore, YB1-like *Salmonella* SWT005 did not achieve strict regulation of bacterial growth in an aerobic environment. This is consistent with previous reports regarding YB1, further indicating that the oxygen regulation system of YB1 *Salmonella* SWT005 has certain defects.

Consequently, the present invention proposes an improvement plan: on the one hand, replacing the essential gene fragment, and on the other hand, replacing the upstream and downstream promoter fragments, to achieve stricter regulation of bacteria in the presence of oxygen and a greater tendency to target tumor hypoxic regions.

### 6. Verification of Essential Gene Replace*ment* and Construction of Strain SWT006

Based on the experimental data of YB1-like *Salmonella* SWT005, the inventor of the present invention considers that the selection of the essential gene *asd* in YB1-like *Salmonella* SWT005 (*pepT-asd-sodA* strain) is not optimal.

Therefore, the present invention replaced the essential gene *asd* in the oxygen-regulated expression cassette of YB1-like *Salmonella* SWT005 with the *alr* gene, creating a new hypoxia-specific regulatory expression cassette (*pepT-alr-sodA*), and verified its oxygen regulation function.

Since a lethal phenotype is caused only by a double mutation of the *alr* and *dadX* genes, it is necessary to construct strain SWT004 (an *aroA* gene-knockout and *alr* gene-knockout strain) firstly, and then integrate the hypoxia-specific regulatory expression cassette (*pepT-alr-sodA*) into the *dadX* gene locus of strain SWT004 (disrupting its function) to construct strain SWT006.

### (1) Construction of Plasmid pSWT005

Using SWT004 as a basis, the hypoxia-specific gene expression cassette containing the *pepT* promoter, *alr* as the essential gene, and the reverse *sodA* promoter was integrated into the *dadX* gene locus of the strain SWT004. The construction process was as follows:

① Plasmid pSWT004 (YB1-like plasmid, including *cm-pepT-asd-sodA*) was digested with HindIII and XhoI to excise the *asd* fragment, and the remaining fragment was recovered.

② Strain SWT001 was amplified using primers SWTO35 and SWTO36 to obtain the amplification product of the *Salmonella alr* gene. The product was double-digested with HindIII and XhoI and recovered.

③ the above fragments were ligated using T4 DNA ligase.

④ the ligation product was transformed into DH10B competent cells, spread onto LB plates containing both ampicillin and chloramphenicol, and incubated inverted at 37°C for 16 hours.

⑤ Single colonies were picked and identified by PCR using primers SWTO11 and SWTO36. The PCR amplification products of SWTO11 and SWTO36 were verified by agarose gel electrophoresis, and the verification results are shown in Figure 13.

⑥ the bacteria were cultured, and the plasmid was extracted to obtain plasmid pSWT005 (containing the *cm-pepT-alr-sodA* element).

### (2) Construction of the Hypoxia-Specific Gene Expression Cassette *pepT-alr-sodA* Strain (SWT006, *aroA⁻* & *alr⁻* & *pepT-alr-sodA*)

① A single colony of the *alr* gene-knockout attenuated bacteria SWT004 was picked and inoculated into 5 mL of LB liquid medium, and cultured in a constant temperature shaker at 32°C and 220 rpm for 16 hours.

② the culture was inoculated at a ratio of 1:100 into fresh LB liquid medium and cultured for a further 2-3 hours until the bacterial density reached $OD_{600} = 0.3$. The culture flask was placed in a 42°C water bath and incubated with shaking for 15 minutes, then placed on ice to rest for 1 hour.

③ the bacterial cells were washed 3 times with sterilized purified water and set aside.

④ pSWT005 (*cm-pepT-alr-sodA*) was amplified using primers SWTO89 and SWTO90, purified, and recovered. The concentration and purity of the recovered PCR product were determined using a nanodrop.

⑤ the recovered bacterial cells were mixed with 100 ng of the PCR product and electroporated at a voltage of 1.8 kV.

⑥ the electroporated bacterial cells were spread onto plates containing 25 $\mu$g/mL chloramphenicol and 100 $\mu$g/mL D-

alanine, and cultured overnight in a constant temperature incubator at 32°C until single colonies grew.

⑦ Positive clones were identified by colony PCR. Primers SWTO87 and SWTO76, and SWTO59 and SWTO77 were used to identify the insertion of the chloramphenicol resistance gene. The PCR amplification products of *Salmonella* SWT006 were verified by agarose gel electrophoresis, and the verification results are shown in Figure 14.

**(3) Oxygen Adaptability Verification of Strain SWT006**

① A single colony of strain SWT006 was picked and inoculated into 5 mL of fresh LB liquid medium containing 100 μg/mL D-alanine, and cultured in a constant temperature shaker at 37°C and 200 rpm for 16 hours.

② After culturing, the strain was diluted 10-fold, and the absorbance (OD value) at 600 nm was measured.

③ the volume of bacterial liquid corresponding to 1 OD of bacteria was calculated according to the following formula, and deionized water was added to reach 1 mL.

$$\text{Volume of bacterial culture for 1 OD} = 1/(\text{Absorbance} \times 10) \times 1000$$

④ 10 μL of the above bacterial suspension was spotted onto two LB plates without D-alanine, labeled as "1". This was repeated three times, labeled "a", "b", and "c".

⑤ After a 10-fold serial dilution, 10 μL of the diluted solution was spotted onto the medium, labeled as "2".

⑥ This 10-fold serial dilution was continued until label "8".

⑦ One plate was placed in an anaerobic environment (oxygen concentration less than 0.01%) at 37°C, and the other plate was placed in an atmospheric environment (21% oxygen concentration) at 37°C.

The results of the oxygen adaptability verification test for strain SWT006 are shown in Figure 15(A). Strain SWT006 grew normally in the anaerobic environment.

From the oxygen adaptability verification results of strain SWT006, compared to the oxygen verification experimental results of SWT005 YB1-like, the selection of *alr* as the essential gene provided better regulation under aerobic conditions than the *asd* gene of SWT005 YB1-like. As shown in Figure 15(B), distinct strain growth was observed only at concentration 1 ($10^{-2}$ OD), concentration 2 ($10^{-3}$ OD), and concentration 3 ($10^{-4}$ OD).

To further optimize and achieve stricter oxygen regulation, the present invention further adjusted the forward hypoxia promoter and the reverse hyperoxia promoter of the hypoxia-specific gene expression cassette.

**Example 2: Integration of the Hypoxia-Specific Gene Expression Cassette into the *dadX* Gene Locus (Disrupting its Function) on the Chromosome of Strain SWT004**

**(I) Construction of *alr*-Deficient Attenuated Bacteria Containing the Hypoxia-Specific Gene Expression Cassette**

1. A single colony of the *alr* gene-knockout attenuated bacteria SWT004 was picked and inoculated into 5 mL of fresh LB liquid medium, and cultured in a constant temperature shaker at 32°C and 220 rpm for 16 hours.

2. The culture was inoculated at a ratio of 1:100 into fresh LB liquid medium and cultured for a further 2-3 hours until the bacterial density reached an $OD_{600}$ of 0.3. The culture flask was then placed in a 42°C water bath and incubated with shaking for 15 minutes, followed by resting on ice for 1 hour.

3. The bacterial cells were washed 3 times with sterilized purified water.

4. Preparation of PCR products for constructing strains containing the hypoxia-specific expression cassette: Primers SWTO78 and SWTO79 were mixed with the hypoxia-specific gene expression cassette plasmid and a high-fidelity PCR amplification enzyme system, and placed into PCR amplification equipment.

The amplification program was: 95°C for 2 minutes; 30 cycles of 95°C for 30 seconds, 60°C for 30 seconds, and 72°C for 120 seconds; followed by 72°C for 10 minutes; and 4°C for 5 minutes.

The amplified PCR products were purified and recovered using a DNA gel extraction system. The concentration and purity of the recovered PCR products were determined using a nanodrop.

5. The recovered bacterial cells were mixed with 100 ng of the PCR product and electroporated at a voltage of 1.8 kV.

6. The electroporated bacterial cells were spread onto plates containing 25 μg/mL chloramphenicol and 100 μg/mL D-alanine, and cultured overnight in a constant temperature incubator at 32°C until single colonies grew.

7. A single colony was inoculated into 5 mL of fresh LB liquid medium containing 100 μg/mL D-alanine and cultured in a constant temperature shaker at 37°C and 220 rpm for 16 hours to allow the CRE enzyme to function and excise the chloramphenicol resistance gene.

8. Clones were identified by colony PCR using the corresponding primers. The identification results are shown in Figure 16.

Information regarding the corresponding strains is shown in Table 2, and the correspondence between strains and identification primers is shown in Table 3.

Table 2 Strain construction table

| Strain Name | Characteristic Description | Source (Description) |
|---|---|---|
| SWT001 | Wild-type *Salmonella* | Purchase from CICC |
| SWT002 | Wild-type *Salmonella* SWT001 transformed with a temperature-inducible Lambda-RED recombinase and loxP-Cre enzyme system (plasmid pSWT001) | Construct |
| SWT003 | Derived from wild-type SWT001, *aroA* gene knockout | Construct |
| SWT004 | Derived from wild-type SWT001, *aroA* gene knockout, *alr* gene knockout | Construct |
| SWT005 | Derived from wild-type SWT001, *aroA* gene knockout, *asd* gene knockout, with the oxygen regulation system *pepT-asd-sodA* (derived from plasmid pSWT004) integrated into the *asd* gene locus (Δ*asd*::*pepT-asd-sodA*); serving as a YB1-like strain | Construct |
| SWT006 | Derived from wild-type SWT001, *aroA* gene knockout, *alr* gene knockout, *dadX* gene knockout, with the oxygen regulation system *pepT-alr-sodA* (derived from plasmid pSWT005) integrated into the *dadX* gene locus (Δ*dadX*::*pepT-alr-sodA*) | Construct |
| SWT007 | Derived from wild-type SWT001, *aroA* gene knockout, *dadX* gene knockout | Construct |
| SWT1001 | Derived from wild-type SWT001, *aroA* gene knockout, *alr* gene knockout, *dadX* gene knockout, with the oxygen regulation system *yhbU*-S-*alr-cyoA*-S (derived from plasmid pOL1001) integrated into the *dadX* gene locus (Δ*dadX*::*yhbU*-S-*alr-cyoA*-S) | Construct |
| SWT1002 | Derived from wild-type SWT001, *aroA* gene knockout, *alr* gene knockout, *dadX* gene knockout, with the oxygen regulation system *yhbU*-S-*alr-phoH*-S (derived from plasmid pOL1002) integrated into the *dadX* gene locus (Δ*dadX*::*yhbU*-S-*alr-phoH*-S) | Construct |
| SWT1003 | Derived from wild-type SWT001, *aroA* gene knockout, *alr* gene knockout, *dadX* gene knockout, with the oxygen regulation system *yhbU*-S-*alr-lldP*-S (derived from plasmid pOL1003) integrated into the *dadX* gene locus (Δ*dadX*::*yhbU*-S-*alr-lldP*-S) | Construct |
| SWT1004 | Derived from wild-type SWT001, *aroA* gene knockout, *alr* gene knockout, *dadX* gene knockout, with the oxygen regulation system *yhbU*-S-*alr-argT*-S (derived from plasmid pOL1004) integrated into the *dadX* gene locus (Δ*dadX*::*yhbU*-S-*alr-argT*-S) | Construct |
| SWT1005 | Derived from wild-type SWT001, *aroA* gene knockout, *alr* gene knockout, *dadX* gene knockout, with the oxygen regulation system *yhbU*-S-*alr-ydcI*-S (derived from plasmid pOL1005) integrated into the *dadX* gene locus (Δ*dadX*::*yhbU*-S-*alr-ydcI*-S) | Construct |
| SWT1006 | Derived from wild-type SWT001, *aroA* gene knockout, *alr* gene knockout, *dadX* gene knockout, with the oxygen regulation system *yhbU*-S-*alr-phoH*-E (derived from plasmid pOL1006) integrated into the *dadX* gene locus (Δ*dadX*::*yhbU*-S-*alr-phoH*-E) | Construct |
| SWT1007 | Derived from wild-type SWT001, *aroA* gene knockout, *alr* gene knockout, *dadX* gene knockout, with the oxygen regulation system *yhbU*-S-*alr-lldP*-E (derived from plasmid pOL1007) integrated into the *dadX* gene locus (Δ*dadX*::*yhbU*-S-*alr-lldP*-E) | Construct |
| SWT1008 | Derived from wild-type SWT001, *aroA* gene knockout, *alr* gene knockout, *dadX* gene knockout, with the oxygen regulation system *yhbU*-S-*alr-ydcI*-E (derived from plasmid pOL1008) integrated into the *dadX* gene locus (Δ*dadX*::*yhbU*-S-*alr-ydcI*-E) | Construct |
| SWT1009 | Derived from wild-type SWT001, *aroA* gene knockout, *alr* gene knockout, *dadX* gene knockout, with the oxygen regulation system *ynfK*-E-*alr-cyoA*-S (derived from plasmid pOL1009) integrated into the *dadX* gene locus (Δ*dadX*::*ynfK*-E-*alr-cyoA*-S) | Construct |

(continued)

| Strain Name | Characteristic Description | Source (Description) |
|---|---|---|
| SWT1010 | Derived from wild-type SWT001, *aroA* gene knockout, *alr* gene knockout, *dadX* gene knockout, with the oxygen regulation system *ynfK*-E-*alr*-*phoH*-S (derived from plasmid pOL1010) integrated into the *dadX* gene locus (Δ*dadX*::*ynfK*-E-*alr*-*phoH*-S) | Construct |
| SWT1011 | Derived from wild-type SWT001, *aroA* gene knockout, *alr* gene knockout, *dadX* gene knockout, with the oxygen regulation system *ynfK*-E-*alr*-*lldP*-S (derived from plasmid pOL1011) integrated into the *dadX* gene locus (Δ*dadX*::*ynfK*-E-*alr*-*lldP*-S) | Construct |
| SWT1012 | Derived from wild-type SWT001, *aroA* gene knockout, *alr* gene knockout, *dadX* gene knockout, with the oxygen regulation system *ynfK*-E-*alr*-*argT*-S (derived from plasmid pOL1012) integrated into the *dadX* gene locus (Δ*dadX*::*ynfK*-E-*alr*-*argT*-S) | Construct |
| SWT1013 | Derived from wild-type SWT001, *aroA* gene knockout, *alr* gene knockout, *dadX* gene knockout, with the oxygen regulation system *ynfK*-E-*alr*-*ydcI*-S (derived from plasmid pOL1013) integrated into the *dadX* gene locus (Δ*dadX*::*ynfK*-E-*alr*-*ydcI*-S) | Construct |
| SWT1014 | Derived from wild-type SWT001, *aroA* gene knockout, *alr* gene knockout, *dadX* gene knockout, with the oxygen regulation system *ynfK*-E-*alr*-*phoH*-E (derived from plasmid pOL1014) integrated into the *dadX* gene locus (Δ*dadX*::*ynfK*-E-*alr*-*phoH*-E) | Construct |
| SWT1015 | Derived from wild-type SWT001, *aroA* gene knockout, *alr* gene knockout, *dadX* gene knockout, with the oxygen regulation system *ynfK*-E-*alr*-*lldP*-E (derived from plasmid pOL1015) integrated into the *dadX* gene locus (Δ*dadX*::*ynfK*-E-*alr*-*lldP*-E) | Construct |
| SWT1016 | Derived from wild-type SWT001, *aroA* gene knockout, *alr* gene knockout, *dadX* gene knockout, with the oxygen regulation system *ynfK*-E-*alr*-*ydcI*-E (derived from plasmid pOL1016) integrated into the *dadX* gene locus (Δ*dadX*::*ynfK*-E-*alr*-*ydcI*-E) | Construct |
| SWT1017 | Derived from wild-type SWT001, *aroA* gene knockout, *alr* gene knockout, *dadX* gene knockout, with the oxygen regulation system *tdcA*-S-*alr*-*cyoA*-S (derived from plasmid pOL1017) integrated into the *dadX* gene locus (Δ*dadX*::*tdcA*-S-*alr*-*cyoA*-S) | Construct |
| SWT1018 | Derived from wild-type SWT001, *aroA* gene knockout, *alr* gene knockout, *dadX* gene knockout, with the oxygen regulation system *tdcA*-S-*alr*-*phoH*-S (derived from plasmid pOL1018) integrated into the *dadX* gene locus (Δ*dadX*::*tdcA*-S-*alr*-*phoH*-S) | Construct |
| SWT1019 | Derived from wild-type SWT001, *aroA* gene knockout, *alr* gene knockout, *dadX* gene knockout, with the oxygen regulation system *tdcA*-S-*alr*-*lldP*-S (derived from plasmid pOL1019) integrated into the *dadX* gene locus (Δ*dadX*::*tdcA*-S-*alr*-*lldP*-S) | Construct |
| SWT1020 | Derived from wild-type SWT001, *aroA* gene knockout, *alr* gene knockout, *dadX* gene knockout, with the oxygen regulation system *tdcA*-S-*alr*-*argT*-S (derived from plasmid pOL1020) integrated into the *dadX* gene locus (Δ*dadX*::*tdcA*-S-*alr*-*argT*-S) | Construct |
| SWT1021 | Derived from wild-type SWT001, *aroA* gene knockout, *alr* gene knockout, *dadX* gene knockout, with the oxygen regulation system *tdcA*-S-*alr*-*ydcI*-S (derived from plasmid pOL1021) integrated into the *dadX* gene locus (Δ*dadX*::*tdcA*-S-*alr*-*ydcI*-S) | Construct |
| SWT1022 | Derived from wild-type SWT001, *aroA* gene knockout, *alr* gene knockout, *dadX* gene knockout, with the oxygen regulation system *tdcA*-S-*alr*-*phoH*-E (derived from plasmid pOL1022) integrated into the *dadX* gene locus (Δ*dadX*::*tdcA*-S-*alr*-*phoH*-E) | Construct |
| SWT1023 | Derived from wild-type SWT001, *aroA* gene knockout, *alr* gene knockout, *dadX* gene knockout, with the oxygen regulation system *tdcA*-S-*alr*-*lldP*-E (derived from plasmid pOL1023) integrated into the *dadX* gene locus (Δ*dadX*::*tdcA*-S-*alr*-*lldP*-E) | Construct |
| SWT1024 | Derived from wild-type SWT001, *aroA* gene knockout, *alr* gene knockout, *dadX* gene knockout, with the oxygen regulation system *tdcA*-S-*alr*-*ydcI*-E (derived from plasmid pOL1024) integrated into the *dadX* gene locus (Δ*dadX*::*tdcA*-S-*alr*-*ydcI*-E) | Construct |
| SWT1025 | Derived from wild-type SWT001, *aroA* gene knockout, *alr* gene knockout, *dadX* gene knockout, with the oxygen regulation system *tdcA*-E-*alr*-*cyoA*-S (derived from plasmid pOL1025) integrated into the *dadX* gene locus (Δ*dadX*::*tdcA*-E-*alr*-*cyoA*-S) | Construct |

(continued)

| Strain Name | Characteristic Description | Source (Description) |
|---|---|---|
| SWT1026 | Derived from wild-type SWT001, *aroA* gene knockout, *alr* gene knockout, *dadX* gene knockout, with the oxygen regulation system *tdcA*-E-*alr*-*phoH*-S (derived from plasmid pOL1026) integrated into the *dadX* gene locus (Δ*dadX*::*tdcA*-E-*alr*-*phoH*-S) | Construct |
| SWT1027 | Derived from wild-type SWT001, *aroA* gene knockout, *alr* gene knockout, *dadX* gene knockout, with the oxygen regulation system *tdcA*-E-*alr*-*lldP*-S (derived from plasmid pOL1027) integrated into the *dadX* gene locus (Δ*dadX*::*tdcA*-E-*alr*-*lldP*-S) | Construct |
| SWT1028 | Derived from wild-type SWT001, *aroA* gene knockout, *alr* gene knockout, *dadX* gene knockout, with the oxygen regulation system *tdcA*-E-*alr*-*argT*-S (derived from plasmid pOL1028) integrated into the *dadX* gene locus (Δ*dadX*::*tdcA*-E-*alr*-*argT*-S) | Construct |
| SWT1029 | Derived from wild-type SWT001, *aroA* gene knockout, *alr* gene knockout, *dadX* gene knockout, with the oxygen regulation system *tdcA*-E-*alr*-*ydcl*-S (derived from plasmid pOL1029) integrated into the *dadX* gene locus (△*dadX*::*tdcA*-E-*alr*-*ydcl*-S) | Construct |
| SWT1030 | Derived from wild-type SWT001, *aroA* gene knockout, *alr* gene knockout, *dadX* gene knockout, with the oxygen regulation system *tdcA*-E-*alr*-*phoH*-E (derived from plasmid pOL1030) integrated into the *dadX* gene locus (Δ*dadX*::*tdcA*-E-*alr*-*phoH*-E) | Construct |
| SWT1031 | Derived from wild-type SWT001, *aroA* gene knockout, *alr* gene knockout, *dadX* gene knockout, with the oxygen regulation system *tdcA*-E-*alr*-*lldP*-E (derived from plasmid pOL1031) integrated into the *dadX* gene locus (Δ*dadX*::*tdcA*-E-*alr*-*lldP*-E) | Construct |
| SWT1032 | Derived from wild-type SWT001, *aroA* gene knockout, *alr* gene knockout, *dadX* gene knockout, with the oxygen regulation system *tdcA*-E-*alr*-*ydcl*-E (derived from plasmid pOL1032) integrated into the *dadX* gene locus (Δ*dadX*::*tdcA*-E-*alr*-*ydcl*-E) | Construct |
| SWT1033 | Derived from wild-type SWT001, *aroA* gene knockout, *alr* gene knockout, *dadX* gene knockout, with the oxygen regulation system *yecH*-E-*alr*-*cyoA*-S (derived from plasmid pOL1033) integrated into the *dadX* gene locus (Δ*dadX*::*yecH*-E-*alr*-*cyoA*-S) | Construct |
| SWT1034 | Derived from wild-type SWT001, *aroA* gene knockout, *alr* gene knockout, *dadX* gene knockout, with the oxygen regulation system *yecH*-E-*alr*-*phoH*-S (derived from plasmid pOL1034) integrated into the *dadX* gene locus (Δ*dadX*::*yecH*-E-*alr*-*phoH*-S) | Construct |
| SWT1035 | Derived from wild-type SWT001, *aroA* gene knockout, *alr* gene knockout, *dadX* gene knockout, with the oxygen regulation system *yecH*-E-*alr*-*lldP*-S (derived from plasmid pOL1035) integrated into the *dadX* gene locus (Δ*dadX*::*yecH*-E-*alr*-*lldP*-S) | Construct |
| SWT1036 | Derived from wild-type SWT001, *aroA* gene knockout, *alr* gene knockout, *dadX* gene knockout, with the oxygen regulation system *yecH*-E-*alr*-*argT*-S (derived from plasmid pOL1036) integrated into the *dadX* gene locus (Δ*dadX*::*yecH*-E-*alr*-*argT*-S) | Construct |
| SWT1037 | Derived from wild-type SWT001, *aroA* gene knockout, *alr* gene knockout, *dadX* gene knockout, with the oxygen regulation system *yecH*-E-*alr*-*ydcl*-S (derived from plasmid pOL1037) integrated into the *dadX* gene locus (Δ*dadX*::*yecH*-E-*alr*-*ydcl*-S) | Construct |
| SWT1038 | Derived from wild-type SWT001, *aroA* gene knockout, *alr* gene knockout, *dadX* gene knockout, with the oxygen regulation system *yecH*-E-*alr*-*phoH*-E (derived from plasmid pOL1038) integrated into the *dadX* gene locus (Δ*dadX*::*yecH*-E-*alr*-*phoH*-E) | Construct |
| SWT1039 | Derived from wild-type SWT001, *aroA* gene knockout, *alr* gene knockout, *dadX* gene knockout, with the oxygen regulation system *yecH*-E-*alr*-*lldP*-E (derived from plasmid pOL1039) integrated into the *dadX* gene locus (Δ*dadX*::*yecH*-E-*alr*-*lldP*-E) | Construct |
| SWT1040 | Derived from wild-type SWT001, *aroA* gene knockout, *alr* gene knockout, *dadX* gene knockout, with the oxygen regulation system *yecH*-E-*alr*-*ydcl*-E (derived from plasmid pOL1040) integrated into the *dadX* gene locus (Δ*dadX*::*yecH*-E-*alr*-*ydcl*-E) | Construct |
| SWT1041 | Derived from wild-type SWT001, *aroA* gene knockout, *alr* gene knockout, *dadX* gene knockout, with the oxygen regulation system *ynfK*-S-*alr*-*cyoA*-S (derived from plasmid pOL1041) integrated into the *dadX* gene locus (Δ*dadX*::*ynfK*-S-*alr*-*cyoA*-S) | Construct |

(continued)

| Strain Name | Characteristic Description | Source (Description) |
|---|---|---|
| SWT1042 | Derived from wild-type SWT001, *aroA* gene knockout, *alr* gene knockout, *dadX* gene knockout, with the oxygen regulation system *ynfK*-S-*alr*-*phoH*-S (derived from plasmid pOL1042) integrated into the *dadX* gene locus (Δ*dadX*::*ynfK*-S-*alr*-*phoH*-S) | Construct |
| SWT1043 | Derived from wild-type SWT001, *aroA* gene knockout, *alr* gene knockout, *dadX* gene knockout, with the oxygen regulation system *ynfK*-E-*alr*-*lldP*-S (derived from plasmid pOL1043) integrated into the *dadX* gene locus (Δ*dadX*::*ynfK*-E-*alr*-*lldP*-S) | Construct |
| SWT1044 | Derived from wild-type SWT001, *aroA* gene knockout, *alr* gene knockout, *dadX* gene knockout, with the oxygen regulation system *ynfK*-S-*alr*-*argT*-S (derived from plasmid pOL1044) integrated into the *dadX* gene locus (Δ*dadX*::*ynfK*-S-*alr*-*argT*-S) | Construct |
| SWT1045 | Derived from wild-type SWT001, *aroA* gene knockout, *alr* gene knockout, *dadX* gene knockout, with the oxygen regulation system *ynfK*-S-*alr*-*ydcI*-S (derived from plasmid pOL1045) integrated into the *dadX* gene locus (Δ*dadX*::*ynfK*-S-*alr*-*ydcI*-S) | Construct |
| SWT1046 | Derived from wild-type SWT001, *aroA* gene knockout, *alr* gene knockout, *dadX* gene knockout, with the oxygen regulation system *ynfK*-S-*alr*-*phoH*-E (derived from plasmid pOL1046) integrated into the *dadX* gene locus (Δ*dadX*::*ynfK*-S-*alr*-*phoH*-E) | Construct |
| SWT1047 | Derived from wild-type SWT001, *aroA* gene knockout, *alr* gene knockout, *dadX* gene knockout, with the oxygen regulation system *ynfK*-S-*alr*-*lldP*-E (derived from plasmid pOL1047) integrated into the *dadX* gene locus (Δ*dadX*::*ynfK*-S-*alr*-*lldP*-E) | Construct |
| SWT1048 | Derived from wild-type SWT001, *aroA* gene knockout, *alr* gene knockout, *dadX* gene knockout, with the oxygen regulation system *ynfK*-S-*alr*-*ydcI*-E (derived from plasmid pOL1048) integrated into the *dadX* gene locus (Δ*dadX*::*ynfK*-S-*alr*-*ydcI*-E) | Construct |
| SWT1049 | Derived from wild-type SWT001, *aroA* gene knockout, *alr* gene knockout, *dadX* gene knockout, with the oxygen regulation system *cydA*-E-*alr*-*cyoA*-S (derived from plasmid pOL1049) integrated into the *dadX* gene locus (Δ*dadX*::*cydA*-E-*alr*-*cyoA*-S) | Construct |
| SWT1050 | Derived from wild-type SWT001, *aroA* gene knockout, *alr* gene knockout, *dadX* gene knockout, with the oxygen regulation system *cydA*-E-*alr*-*phoH*-S (derived from plasmid pOL1050) integrated into the *dadX* gene locus (Δ*dadX*::*cydA*-E-*alr*-*phoH*-S) | Construct |
| SWT1051 | Derived from wild-type SWT001, *aroA* gene knockout, *alr* gene knockout, *dadX* gene knockout, with the oxygen regulation system *cydA*-E-*alr*-*lldP*-S (derived from plasmid pOL1051) integrated into the *dadX* gene locus (Δ*dadX*::*cydA*-E-*alr*-*lldP*-S) | Construct |
| SWT1052 | Derived from wild-type SWT001, *aroA* gene knockout, *alr* gene knockout, *dadX* gene knockout, with the oxygen regulation system *cydA*-E-*alr*-*argT*-S (derived from plasmid pOL1052) integrated into the *dadX* gene locus (Δ*dadX*::*cydA*-E-*alr*-*argT*-S) | Construct |
| SWT1053 | Derived from wild-type SWT001, *aroA* gene knockout, *alr* gene knockout, *dadX* gene knockout, with the oxygen regulation system *cydA*-E-*alr*-*ydcI*-S (derived from plasmid pOL1053) integrated into the *dadX* gene locus (Δ*dadX*::*cydA*-E-*alr*-*ydcI*-S) | Construct |
| SWT1054 | Derived from wild-type SWT001, *aroA* gene knockout, *alr* gene knockout, *dadX* gene knockout, with the oxygen regulation system *cydA*-E-*alr*-*phoH*-E (derived from plasmid pOL1054) integrated into the *dadX* gene locus (Δ*dadX*::*cydA*-E-*alr*-*phoH*-E) | Construct |
| SWT1055 | Derived from wild-type SWT001, *aroA* gene knockout, *alr* gene knockout, *dadX* gene knockout, with the oxygen regulation system *cydA*-E-*alr*-*lldP*-E (derived from plasmid pOL1055) integrated into the *dadX* gene locus (Δ*dadX*::*cydA*-E-*alr*-*lldP*-E) | Construct |
| SWT1056 | Derived from wild-type SWT001, *aroA* gene knockout, *alr* gene knockout, *dadX* gene knockout, with the oxygen regulation system *cydA*-E-*alr*-*ydcI*-E (derived from plasmid pOL1056) integrated into the *dadX* gene locus (Δ*dadX*::*cydA*-E-*alr*-*ydcI*-E) | Construct |
| SWT1057 | Derived from wild-type SWT001, *aroA* gene knockout, *alr* gene knockout, *dadX* gene knockout, with the oxygen regulation system *focA*-E-*alr*-*cyoA*-S (derived from plasmid pOL1057) integrated into the *dadX* gene locus (Δ*dadX*::*focA*-E-*alr*-*cyoA*-S) | Construct |

(continued)

| Strain Name | Characteristic Description | Source (Description) |
|---|---|---|
| SWT1058 | Derived from wild-type SWT001, *aroA* gene knockout, *alr* gene knockout, *dadX* gene knockout, with the oxygen regulation system *focA*-E-*alr*-*phoH*-S (derived from plasmid pOL1058) integrated into the *dadX* gene locus (ΔdadX::*focA*-E-*alr*-*phoH*-S) | Construct |
| SWT1059 | Derived from wild-type SWT001, *aroA* gene knockout, *alr* gene knockout, *dadX* gene knockout, with the oxygen regulation system *focA*-E-*alr*-*lldP*-S (derived from plasmid pOL1059) integrated into the *dadX* gene locus (ΔdadX::*focA*-E-*alr*-*lldP*-S) | Construct |
| SWT1060 | Derived from wild-type SWT001, *aroA* gene knockout, *alr* gene knockout, *dadX* gene knockout, with the oxygen regulation system *focA*-E-*alr*-*argT*-S (derived from plasmid pOL1060) integrated into the *dadX* gene locus (ΔdadX::*focA*-E-*alr*-*argT*-S) | Construct |
| SWT1061 | Derived from wild-type SWT001, *aroA* gene knockout, *alr* gene knockout, *dadX* gene knockout, with the oxygen regulation system *focA*-E-*alr*-*ydcI*-S (derived from plasmid pOL1061) integrated into the *dadX* gene locus (ΔdadX::*focA*-E-*alr*-*ydcI*-S) | Construct |
| SWT1062 | Derived from wild-type SWT001, *aroA* gene knockout, *alr* gene knockout, *dadX* gene knockout, with the oxygen regulation system *focA*-E-*alr*-*phoH*-E (derived from plasmid pOL1062) integrated into the *dadX* gene locus (ΔdadX::*focA*-E-*alr*-*phoH*-E) | Construct |
| SWT1063 | Derived from wild-type SWT001, *aroA* gene knockout, *alr* gene knockout, *dadX* gene knockout, with the oxygen regulation system *focA*-E-*alr*-*lldP*-E (derived from plasmid pOL1063) integrated into the *dadX* gene locus (ΔdadX::*focA*-E-*alr*-*lldP*-E) | Construct |
| SWT1064 | Derived from wild-type SWT001, *aroA* gene knockout, *alr* gene knockout, *dadX* gene knockout, with the oxygen regulation system *focA*-E-*alr*-*ydcI*-E (derived from plasmid pOL1064) integrated into the *dadX* gene locus (ΔdadX::*focA*-E-*alr*-*ydcI*-E) | Construct |
| SWT1065 | Derived from wild-type SWT001, *aroA* gene knockout, *alr* gene knockout, *dadX* gene knockout, with the oxygen regulation system *yhbU*-E-*alr*-*cyoA*-S (derived from plasmid pOL1065) integrated into the *dadX* gene locus (ΔdadX::*yhbU*-E-*alr*-*cyoA*-S) | Construct |
| SWT1066 | Derived from wild-type SWT001, *aroA* gene knockout, *alr* gene knockout, *dadX* gene knockout, with the oxygen regulation system *yhbU*-E-*alr*-*phoH*-S (derived from plasmid pOL1066) integrated into the *dadX* gene locus (ΔdadX::*yhbU*-E-*alr*-*phoH*-S) | Construct |
| SWT1067 | Derived from wild-type SWT001, *aroA* gene knockout, *alr* gene knockout, *dadX* gene knockout, with the oxygen regulation system *yhbU*-E-*alr*-*lldP*-S (derived from plasmid pOL1067) integrated into the *dadX* gene locus (ΔdadX::*yhbU*-E-*alr*-*lldP*-S) | Construct |
| SWT1068 | Derived from wild-type SWT001, *aroA* gene knockout, *alr* gene knockout, *dadX* gene knockout, with the oxygen regulation system *yhbU*-E-*alr*-*argT*-S (derived from plasmid pOL1068) integrated into the *dadX* gene locus (ΔdadX::*yhbU*-E-*alr*-*argT*-S) | Construct |
| SWT1069 | Derived from wild-type SWT001, *aroA* gene knockout, *alr* gene knockout, *dadX* gene knockout, with the oxygen regulation system *yhbU*-E-*alr*-*ydcI*-S (derived from plasmid pOL1069) integrated into the *dadX* gene locus (ΔdadX::*yhbU*-E-*alr*-*ydcI*-S) | Construct |
| SWT1070 | Derived from wild-type SWT001, *aroA* gene knockout, *alr* gene knockout, *dadX* gene knockout, with the oxygen regulation system *yhbU*-E-*alr*-*phoH*-E (derived from plasmid pOL1070) integrated into the *dadX* gene locus (ΔdadX::*yhbU*-E-*alr*-*phoH*-E) | Construct |
| SWT1071 | Derived from wild-type SWT001, *aroA* gene knockout, *alr* gene knockout, *dadX* gene knockout, with the oxygen regulation system *yhbU*-E-*alr*-*lldP*-E (derived from plasmid pOL1071) integrated into the *dadX* gene locus (ΔdadX::*yhbU*-E-*alr*-*lldP*-E) | Construct |
| SWT1072 | Derived from wild-type SWT001, *aroA* gene knockout, *alr* gene knockout, *dadX* gene knockout, with the oxygen regulation system *yhbU*-E-*alr*-*ydcI*-E (derived from plasmid pOL1072) integrated into the *dadX* gene locus (ΔdadX::*yhbU*-E-*alr*-*ydcI*-E) | Construct |
| SWT1073 | Derived from wild-type SWT001, *aroA* gene knockout, *alr* gene knockout, *dadX* gene knockout, with the oxygen regulation system *yhbU*-S-*alr*-*mqo* (derived from plasmid pOL1073) integrated into the *dadX* gene locus (ΔdadX::*yhbU*-S-*alr*-*mqo*) | Construct |

(continued)

| Strain Name | Characteristic Description | Source (Description) |
|---|---|---|
| SWT1074 | Derived from wild-type SWT001, *aroA* gene knockout, *alr* gene knockout, *dadX* gene knockout, with the oxygen regulation system *ynfK*-E-*alr-mqo* (derived from plasmid pOL1074) integrated into the *dadX* gene locus (Δ*dadX*::*ynfK*-E-*alr-mqo*) | Construct |
| SWT1075 | Derived from wild-type SWT001, *aroA* gene knockout, *alr* gene knockout, *dadX* gene knockout, with the oxygen regulation system *yecH*-E-*alr-mqo* (derived from plasmid pOL1075) integrated into the *dadX* gene locus (Δ*dadX*::*yecH*-E-*alr-mqo*) | Construct |
| SWT2001 | Derived from wild-type SWT001, *aroA* gene knockout, *alr* gene knockout, *dadX* gene knockout, with the oxygen regulation system *yhbU*-S-*dadX-cyoA*-S (derived from plasmid pOL2001) integrated into the *alr* gene locus (Δ*alr*::*yhbU*-S-*dadX-cyoA*-S) | Construct |
| SWT2002 | Derived from wild-type SWT001, *aroA* gene knockout, *alr* gene knockout, *dadX* gene knockout, with the oxygen regulation system *yhbU*-S-*dadX-phoH*-S (derived from plasmid pOL2002) integrated into the *alr* gene locus (Δ*alr*::*yhbU*-S-*dadX-phoH*-S) | Construct |
| SWT2004 | Derived from wild-type SWT001, *aroA* gene knockout, *alr* gene knockout, *dadX* gene knockout, with the oxygen regulation system *yhbU*-S-*dadX-argT*-S (derived from plasmid pOL2004) integrated into the *alr* gene locus (Δ*alr*::*yhbU*-S-*dadX-argT*-S) | Construct |
| SWT2005 | Derived from wild-type SWT001, *aroA* gene knockout, *alr* gene knockout, *dadX* gene knockout, with the oxygen regulation system *yhbU*-S-*dadX-ydcI*-S (derived from plasmid pOL2005) integrated into the *alr* gene locus (Δ*alr*::*yhbU*-S-*dadX-ydcI*-S) | Construct |
| SWT2009 | Derived from wild-type SWT001, *aroA* gene knockout, *alr* gene knockout, *dadX* gene knockout, with the oxygen regulation system *ynfK*-E-*dadX-cyoA*-S (derived from plasmid pOL2009) integrated into the *alr* gene locus (Δ*alr*::*yhbU*-S-*dadX-cyoA*-S) | Construct |
| SWT2010 | Derived from wild-type SWT001, *aroA* gene knockout, *alr* gene knockout, *dadX* gene knockout, with the oxygen regulation system *ynfK*-E-*dadX-phoH*-S (derived from plasmid pOL2010) integrated into the *alr* gene locus (Δ*alr*::*ynfK*-E-*dadX-phoH*-S) | Construct |
| SWT2012 | Derived from wild-type SWT001, *aroA* gene knockout, *alr* gene knockout, *dadX* gene knockout, with the oxygen regulation system *ynfK*-E-*dadX-argT*-S (derived from plasmid pOL2012) integrated into the *alr* gene locus (Δ*alr*::*ynfK*-E-*dadX-argT*-S) | Construct |
| SWT2013 | Derived from wild-type SWT001, *aroA* gene knockout, *alr* gene knockout, *dadX* gene knockout, with the oxygen regulation system *ynfK*-E-*dadX-ydcI*-S (derived from plasmid pOL2013) integrated into the *alr* gene locus (Δ*alr*::*ynfK*-E-*dadX-ydcI*-S) | Construct |
| SWT2021 | Derived from wild-type SWT001, *aroA* gene knockout, *alr* gene knockout, *dadX* gene knockout, with the oxygen regulation system *tdcA*-S-*dadX-ydcI*-S (derived from plasmid pOL2021) integrated into the *alr* gene locus (Δ*alr*::*tdcA*-S-*dadX-ydcI*-S) | Construct |
| SWT2025 | Derived from wild-type SWT001, *aroA* gene knockout, *alr* gene knockout, *dadX* gene knockout, with the oxygen regulation system *tdcA*-E-*dadX-ydcI*-S (derived from plasmid pOL2025) integrated into the *alr* gene locus (Δ*alr*::*tdcA*-E-*dadX-ydcI*-S) | Construct |

(continued)

| Strain Name | Characteristic Description | Source (Description) |
|---|---|---|
| SWT2033 | Derived from wild-type SWT001, *aroA* gene knockout, *alr* gene knockout, *dadX* gene knockout, with the oxygen regulation system *yecH*-E-*dadX-ydcI*-S (derived from plasmid pOL2033) integrated into the *alr* gene locus (Δ*alr*::*yecH*-E-*dadX-ydcI*-S) | Construct |
| SWT2035 | Derived from wild-type SWT001, *aroA* gene knockout, *alr* gene knockout, *dadX* gene knockout, with the oxygen regulation system *yecH*-E-*dadX-lldP*-S (derived from plasmid pOL2035) integrated into the *alr* gene locus (Δ*alr*::*yecH*-E-*dadX-lldP*-S) | Construct |
| SWT2036 | Derived from wild-type SWT001, *aroA* gene knockout, *alr* gene knockout, *dadX* gene knockout, with the oxygen regulation system *yecH*-E-*dadX-argT*-S (derived from plasmid pOL2036) integrated into the *alr* gene locus (Δ*alr*::*yecH*-E-*dadX-argT*-S) | Construct |
| SWT2038 | Derived from wild-type SWT001, *aroA* gene knockout, *alr* gene knockout, *dadX* gene knockout, with the oxygen regulation system *yecH*-E-*dadX-phoH*-E (derived from plasmid pOL2038) integrated into the *alr* gene locus (Δ*alr*::*yecH*-E-*dadX-phoH*-E) | Construct |
| SWT2063 | Derived from wild-type SWT001, *aroA* gene knockout, *alr* gene knockout, *dadX* gene knockout, with the oxygen regulation system *focA*-E-*dadX-lldP*-E (derived from plasmid pOL2063) integrated into the *alr* gene locus (Δ*alr*::*focA*-E-*dadX-lldP*-E) | Construct |

Table 3 Correspondence between strains and identification primers

| Strain Name | Identification Primers | Identification Result |
|---|---|---|
| Strain SWT1001 (yhbU-S + alr + cyoA-S combination) | SWTO61, 20; SWTO75, 20 | Figure 16A |
| Strain SWT1002 (yhbU-S + alr + phoH-S combination) | SWTO61, 20; SWTO75, 20 | Figure 16A |
| Strain SWT1003 (yhbU-S + alr + lldP-S combination) | SWTO61, 20; SWTO75, 20 | Figure 16A |
| Strain SWT1004 (yhbU-S + alr + argT-S combination) | SWTO61, 20; SWTO75, 20 | Figure 16A |
| Strain SWT1005 (yhbU-S + alr + ydcI-S combination) | SWTO61, 20; SWTO75, 20 | Figure 16A |
| Strain SWT1006 (yhbU-S + alr + phoH-E combination) | SWTO61, 20; SWTO75, 20 | Figure 16A |
| Strain SWT1007 (yhbU-S + alr + lldP-E combination) | SWTO61, 20; SWTO75, 20 | Figure 16A |
| Strain SWT1008 (yhbU-S + alr + ydcI-E combination) | SWTO61, 20; SWTO75, 20 | Figure 16A |
| Strain SWT1009 (ynfK-E + alr + cyoA-S combination) | SWTO61, 22; SWTO75, 22 | Figure 16B |
| Strain SWT1010 (ynfK-E + alr + phoH-S combination) | SWTO61, 22; SWTO75, 22 | Figure 16B |
| Strain SWT1011 (ynfK-E + alr + lldP-S combination) | SWTO61, 22; SWTO75, 22 | Figure 16B |
| Strain SWT1012 (ynfK-E + alr + argT-S combination) | SWTO61, 22; SWTO75, 22 | Figure 16B |
| Strain SWT1013 (ynfK-E + alr + ydcI-S combination) | SWTO61, 22; SWTO75, 22 | Figure 16B |
| Strain SWT1014 (ynfK-E + alr + phoH-E combination) | SWTO61, 22; SWTO75, 22 | Figure 16B |
| Strain SWT1015 (ynfK-E + alr + lldP-E combination) | SWTO61, 22; SWTO75, 22 | Figure 16B |
| Strain SWT1016 (ynfK-E + alr + ydcI-E combination) | SWTO61, 22; SWTO75, 22 | Figure 16B |
| Strain SWT1017 (tdcA-S + alr + cyoA-S combination) | SWTO61, 84; SWTO75, 84 | Figure 16C |
| Strain SWT1018 (tdcA-S + alr + phoH-S combination) | SWTO61, 84; SWTO75, 84 | Figure 16C |
| Strain SWT1019 (tdcA-S + alr + lldP-S combination) | SWTO61, 84; SWTO75, 84 | Figure 16C |
| Strain SWT1020 (tdcA-S + alr + argT-S combination) | SWTO61, 84; SWTO75, 84 | Figure 16C |
| Strain SWT1021 (tdcA-S + alr + ydcI-S combination) | SWTO61, 84; SWTO75, 84 | Figure 16C |

(continued)

| Strain Name | Identification Primers | Identification Result |
|---|---|---|
| Strain SWT1022 (tdcA-S + alr + phoH-E combination) | SWTO61, 84; SWTO75, 84 | Figure 16C |
| Strain SWT1023 (tdcA-S + alr + lldP-E combination) | SWTO61, 84; SWTO75, 84 | Figure 16C |
| Strain SWT1024 (tdcA-S + alr + ydcl-E combination) | SWTO61, 84; SWTO75, 84 | Figure 16C |
| Strain SWT1025 (tdcA-E + alr + cyoA-S combination) | SWTO61, 26;SWTO75, 26 | Figure 16D |
| Strain SWT1026 (tdcA-E + alr + phoH-S combination) | SWTO61, 26; SWTO75, 26 | Figure 16D |
| Strain SWT1027 (tdcA-E + alr + lldP-S combination) | SWTO61, 26; SWTO75, 26 | Figure 16D |
| Strain SWT1028 (tdcA-E + alr + argT-S combination) | SWTO61, 26; SWTO75, 26 | Figure 16D |
| Strain SWT1029 (tdcA-E + alr + ydcl-S combination) | SWTO61, 26; SWTO75, 26 | Figure 16D |
| Strain SWT1030 (tdcA-E + alr + phoH-E combination) | SWTO61, 26; SWTO75, 26 | Figure 16D |
| Strain SWT1031 (tdcA-E + alr + lldP-E combination) | SWTO61, 26; SWTO75, 26 | Figure 16D |
| Strain SWT1032 (tdcA-E + alr + ydcl-E combination) | SWTO61, 26; SWTO75, 26 | Figure 16D |
| Strain SWT1033 (yecH-E + alr + cyoA-S combination) | SWTO61, 28; SWTO75, 28 | Figure 16E |
| Strain SWT1034 (yecH-E + alr + phoH-S combination) | SWTO61, 28; SWTO75, 28 | Figure 16E |
| Strain SWT1035 (yecH-E + alr + lldP-S combination) | SWTO61, 28; SWTO75, 28 | Figure 16E |
| Strain SWT1036 (yecH-E + alr + argT-S combination) | SWTO61, 28; SWTO75, 28 | Figure 16E |
| Strain SWT1037 (yecH-E + alr + ydcl-S combination) | SWTO61, 28; SWTO75, 28 | Figure 16E |
| Strain SWT1038 (yecH-E + alr + phoH-E combination) | SWTO61, 28; SWTO75, 28 | Figure 16E |
| Strain SWT1039 (yecH-E + alr + lldP-E combination) | SWTO61, 28; SWTO75, 28 | Figure 16E |
| Strain SWT1040 (yecH-E + alr + ydcl-E combination) | SWTO61, 28; SWTO75, 28 | Figure 16E |
| Strain SWT1041 (ynfK-S + alr + cyoA-S combination) | SWTO75, 30; SWTO87, 30 | Figure 16F |
| Strain SWT1042 (ynfK-S + alr + phoH-S combination) | SWTO75, 30; SWTO87, 30 | Figure 16F |
| Strain SWT1043 (ynfK-S + alr + lldP-S combination) | SWTO75, 30; SWTO87, 30 | Figure 16F |
| Strain SWT1044 (ynfK-S + alr + argT-S combination) | SWTO75, 30; SWTO87, 30 | Figure 16F |
| Strain SWT1045 (ynfK-S + alr + ydcl-S combination) | SWTO75, 30; SWTO87, 30 | Figure 16F |
| Strain SWT1046 (ynfK-S + alr + phoH-E combination) | SWTO75, 30; SWTO87, 30 | Figure 16F |
| Strain SWT1047 (ynfK-S + alr + lldP-E combination) | SWTO75, 30; SWTO87, 30 | Figure 16F |
| Strain SWT1048 (ynfK-S + alr + ydcl-E combination) | SWTO75, 30; SWTO87, 30 | Figure 16F |
| Strain SWT1049 (cydA-E + alr + cyoA-S combination) | SWTO75, 88; SWTO87, 88 | Figure 16G |
| Strain SWT1050 (cydA-E + alr + phoH-S combination) | SWTO75, 88; SWTO87, 88 | Figure 16G |
| Strain SWT1051 (cydA-E + alr + lldP-S combination) | SWTO75, 88; SWTO87, 88 | Figure 16G |
| Strain SWT1052 (cydA-E + alr + argT-S combination) | SWTO75, 88; SWTO87, 88 | Figure 16G |
| Strain SWT1053 (cydA-E + alr + ydcl-S combination) | SWTO75, 88; SWTO87, 88 | Figure 16G |
| Strain SWT1054 (cydA-E + alr + phoH-E combination) | SWTO75, 88; SWTO87, 88 | Figure 16G |
| Strain SWT1055 (cydA-E + alr + lldP-E combination) | SWTO75, 88; SWTO87, 88 | Figure 16G |
| Strain SWT1056 (cydA-E + alr + ydcl-E combination) | SWTO75, 88; SWTO87, 88 | Figure 16G |
| Strain SWT1057 (focA-E + alr + cyoA-S combination) | SWTO75, 96; SWTO87, 96 | Figure 16H |
| Strain SWT1058 (focA-E + alr + phoH-S combination) | SWTO75, 96; SWTO87, 96 | Figure 16H |
| Strain SWT1059 (focA-E + alr + lldP-S combination) | SWTO75, 96; SWTO87, 96 | Figure 16H |
| Strain SWT1060 (focA-E + alr + argT-S combination) | SWTO75, 96; SWTO87, 96 | Figure 16H |

(continued)

| Strain Name | Identification Primers | Identification Result |
|---|---|---|
| Strain SWT1061 (focA-E + alr + ydcl-S combination) | SWTO75, 96; SWTO87, 96 | Figure 16H |
| Strain SWT1062 (focA-E + alr + phoH-E combination) | SWTO75, 96; SWTO87, 96 | Figure 16H |
| Strain SWT1063 (focA-E + alr + lldP-E combination) | SWTO75, 96; SWTO87, 96 | Figure 16H |
| Strain SWT1064 (focA-E + alr + ydcl-E combination) | SWTO75, 96; SWTO87, 96 | Figure 16H |
| Strain SWT1065 (yhbU-E + alr + cyoA-S combination) | SWTO75, 97; SWTO87, 97 | Figure 16I |
| Strain SWT1066 (yhbU-E + alr + phoH-S combination) | SWTO75, 97; SWTO87, 97 | Figure 16I |
| Strain SWT1067 (yhbU-E + alr + lldP-S combination) | SWTO75, 97; SWTO87, 97 | Figure 16I |
| Strain SWT1068 (yhbU-E + alr + argT-S combination) | SWTO75, 97; SWTO87, 97 | Figure 16I |
| Strain SWT1069 (yhbU-E + alr + ydcl-S combination) | SWTO75, 97; SWTO87, 97 | Figure 16I |
| Strain SWT1070 (yhbU-E + alr + phoH-E combination) | SWTO75, 97; SWTO87, 97 | Figure 16I |
| Strain SWT1071 (yhbU-E + alr + lldP-E combination) | SWTO75, 97; SWTO87, 97 | Figure 16I |
| Strain SWT1072 (yhbU-E + alr + ydcl-E combination) | SWTO75, 97; SWTO87, 97 | Figure 16I |
| Strain SWT1073 (yhbU-S + alr + mqo combination) | SWTO35, 59; SWTO35, 60 | Figure 16J |
| Strain SWT1074 (ynfK-E + alr + mqo combination) | SWTO35, 59; SWTO35, 60 | Figure 16J |
| Strain SWT1075 (yecH-E + alr + mqo combination) | SWTO35, 59; SWTO35, 60 | Figure 16J |

**(II) Verification of Oxygen Adaptability of the *Salmonella* Strain Library Containing Hypoxia-Specific Gene Expression Cassette**

1. Single colonies of strains containing the hypoxia-specific expression cassette were picked and inoculated into 5 mL of fresh LB liquid medium containing 100 μg/mL D-alanine, and cultured in a constant temperature shaker at 37°C and 200 rpm for 16 hours.
2. After culturing was completed, the strains were diluted 10-fold, and the absorbance (OD value) at 600 nm was measured.
3. The volume of bacterial culture corresponding to 1 OD of bacteria was calculated according to the following formula, and deionized water was added to reach 1 mL.

$$\text{Volume of bacterial culture for 1 OD} = 1/\,(\text{Absorbance} \times 10) \times 1000$$

4. 10 μL of the above bacterial suspension was taken and spotted onto two LB plates without D-alanine for culture, labeled as "1". This was repeated three times, labeled as "a", "b", and "c".
5. After performing a 10-fold serial dilution, 10 μL of the diluted solution was taken and spotted onto the above medium, labeled as "2".
6. This 10-fold serial dilution was continued in the same manner until labeled "8".
7. One of the plates was placed in an anaerobic environment at 37°C for culture, and the other plate was placed in an atmospheric environment (21% oxygen concentration) at 37°C for culture.

The verification test results of the oxygen adaptability of the strains containing the hypoxia-specific expression cassette are shown in Figures 17-31.

Based on the data of the aforementioned YB1-like (SWT005) and the essential gene replacement strain (SWT006), the present invention designed novel forward hypoxia promoters, survival-essential genes, and reverse hyperoxia promoters, and composed new hypoxia-specific gene expression cassette. The results demonstrate that *Salmonella* strains SWT1001, SWT1002, SWT1004, SWT1005, SWT1009, SWT1010, SWT1012, SWT1013, SWT1021, SWT1025, SWT1033, SWT1035, SWT1036, SWT1038, and SWT1063, which contain the described hypoxia-specific gene expression cassette, all exhibited superior effects in the mutability assessment compared to YB1-like (SWT005) and the essential gene replacement strain (SWT006).

**(III)Oxygen Concentration Simulation for the Strain Library Containing Hypoxia-Specific Gene Expression Cassette**

Oxygen in the sealed culture jar is consumed by using an anaerobic gas-generating bag, and the oxygen concentration in the sealed culture jar is determined using an oxygen meter. After using the anaerobic gas-generating bag for a certain period, the oxygen concentration in the sealed culture jar can be fixed within a specific concentration range. The specific method is as follows:

An anaerobic gas-generating bag (Brand: Mitsubishi, Japan; Cat. No.: D-119) was placed into a 7.0 L sealed culture jar (Brand: Mitsubishi, Japan; Cat. No.: D-112), and an oxygen meter (Brand: Meicheng Electronics; Cat. No.: OX-100A) was placed inside simultaneously.

The above-mentioned anaerobic gas-generating bag combination was used to verify the growth status of the genetically modified strains under an oxygen concentration below 0.8%.

**1. Adaptability Verification of the Strain Library Containing Hypoxia-Specific Gene Expression Cassettes Under an Oxygen Concentration Below 0.8%**

Since the range of the pathological hypoxia zone in tumors corresponds to an oxygen concentration of less than 1%, the present invention intends to demonstrate that *Salmonella* modified with the hypoxia-specific gene expression cassette can still grow normally when the oxygen concentration is less than 1% (or approaches 1%).

The regulation of oxygen concentration was achieved using an anaerobic gas-generating bag. After consumption by the anaerobic gas-generating bag for 1 hour, the reading on the oxygen meter indicated that the oxygen concentration was between 0.8% and 1%. Therefore, this experiment simulated the intratumoral hypoxic environment to verify the growth of *Salmonella* on culture plates after the anaerobic gas-generating bag had consumed oxygen in the sealed culture jar for 1 hour, thereby simulating the growth conditions of *Salmonella* strains containing the hypoxia-specific gene expression cassette within the intratumoral hypoxic environment.

The operational procedure for the spotting assay is as follows:

(1) Single colonies of strains containing the hypoxia-specific expression cassette were picked and inoculated into 5 mL of fresh LB liquid medium containing 100 $\mu$g/mL D-alanine, and cultured in a constant temperature shaker at 37°C and 200 rpm for 16 hours.
(2) After culturing was completed, the strains were diluted 10-fold, and the absorbance (OD value) at 600 nm was measured.
(3) The volume of bacterial culture corresponding to 1 OD of bacteria was calculated according to the following formula, and deionized water was added to reach 1 mL.

$$\text{Volume of bacterial culture for 1 OD} = 1/(\text{Absorbance} \times 10) \times 1000$$

(4) 10 $\mu$L of the above bacterial suspension was taken and spotted onto an LB plate containing D-alanine for culture, labeled as "1". This was repeated three times, labeled as "a", "b", and "c".
(5) Simultaneously, 10 $\mu$L of the above bacterial suspension was taken and spotted onto another LB plate without D-alanine for culture, labeled as "1" with three replicates labeled as "a", "b", and "c".
(6) After performing a 10-fold serial dilution, 10 $\mu$L of the diluted solution was taken and spotted onto the above media, labeled as "2".
(7) This 10-fold serial dilution was continued in the same manner until labeled "8".
(8) The two plates were placed in an environment with 0.8% oxygen at 37°C for culture.

As shown in Figure 32, the present invention compared the growth of different strains (Figure 32(A) SWT1001, Figure 32(B) SWT1005, Figure 32(C) SWT1009, Figure 32(D) SWT1013) on LB plates without supplemented D-alanine to judge the growth status of each strain under an oxygen concentration below 0.8%. Since the medium was not supplemented with D-alanine, the corresponding *Salmonella* strains relied on their own oxygen regulation systems to grow normally. The results confirmed that strains containing the hypoxia-specific expression cassette, which can grow normally in an anaerobic environment, can all grow normally under an oxygen concentration below 0.8%.

**2. Immunohistochemical Staining of Strain SWT1005 Containing a Hypoxia-Specific Gene Expression Cassette and Hypoxic Tumor Regions**

To demonstrate the accumulation of the *Salmonella* strain containing a hypoxia-specific gene expression

**EP 4 782 543 A1**

cassette within the hypoxic regions of a tumor, tumor models from mice administered with the *Salmonella* via tail vein injection were sectioned and stained with antibodies of anti-*Salmonella* and hypoxiainducible factor anti-HIF-1α to observe the distribution of the *Salmonella* in the tumor model.

**(1) Establishment of the Tumor Model**

BALB/c mice (purchased from Zhejiang Vital River Laboratory Animal Technology Co., Ltd., approximate weight 18 g, housed in an SPF environment) were subcutaneously inoculated with $1\times10^6$ CT26 murine colon carcinoma cells to establish a subcutaneous colon carcinoma model. Experiments were initiated 10-12 days post-inoculation when the tumor volume reached approximately 100 mm$^3$. The mice were divided into a PBS group and an SWT1005 group, 5 mice per group. Tumor-bearing mice were administered $1\times10^7$ CFU of bacteria in a volume of 125 μl via tail vein injection.

**(2) Bacterial Preparation**

Using strain SWT1005 as an example, the strain was streaked onto LB agar plates supplemented with D-alanine and incubated still overnight in a 37°C incubator. A single colony of SWT1005 was picked and inoculated into 5 ml of fresh LB liquid medium containing 100 μg/ml D-alanine, and cultured for 16 hours in a constant temperature shaker at 37°C and 220 rpm. The optical density (OD) of the bacterial culture was measured at 600 nm. A volume of the culture equivalent to an $OD_{600}$ of 1.0 was calculated and taken, washed three times with PBS, and resuspended in PBS to prepare the bacterial suspension.

**(3) Preparation of Tumor Sections**

On day 3 post-administration, mice were sacrificed, and tumors were dissected and immersed in formalin for fixation. The tissues were subjected to graded dehydration and embedded in paraffin. After complete cooling and solidification, the paraffin blocks were sectioned and the sections were deparaffinized. The sections were immersed in a sodium citrate antigen retrieval solution and boiled for 10 minutes for antigen retrieval, followed by three washes with PBS. The sections after antigen retrieval were then immersed in a 3% hydrogen peroxide solution for 10 minutes, followed by three washes with PBS. The sections after removing catalase were subsequently blocked with a 2% BSA-PBS solution for 1 hour at room temperature, followed by three washes with PBS. The blocked tissue sections were then separately incubated with primary antibodies of anti-Salmonella (Abcam, Cat. No. ab35156) or anti-HIF1α (Abcam, Cat. No. ab51608), overnight at 4°C, followed by three washes with PBS. A secondary antibody solution was added to the tissue sections and incubated for 25 minutes, followed by three washes with PBS. The sections were dropped with a DAB (3,3'-Diaminobenzidine) solution and counterstained with hematoxylin for 3 minutes. The sections were then subjected to graded dehydration and mounted with a mounting medium. The sections were observed and photographed under a microscope, as shown in Figure 33.

The results indicate that, by comparing the antibody-stained sections, the brown (darkly stained) regions represent positive antibody staining. It was found that the *Salmonella* strain containing the hypoxia-specific gene expression cassette accumulated in the hypoxic regions of the tumor.

**(IV) Evaluation of the Cell Invasion Capacity of Strains Containing a Hypoxia-Specific Gene Expression Cassette**

Transform pSWT006 (a plasmid with strong EGFP expression under the control of a prokaryotic promoter) into *Salmonella* strains modified with a hypoxia-specific gene expression cassette, and determine the invasion of tumor cells by *Salmonella* by observing the location of fluorescence and comparing cellular distribution.

The cell invasion capacity of the strains containing the hypoxia-specific gene expression cassette was evaluated against a panel of cancer cell lines, including breast cancer (EMT6 cell line), osteosarcoma (K7M2 cell line), liver cancer (Hepa1-6 cell line), lung cancer (A549 cell line), melanoma (B16F10 cell line), renal cancer (Renca cell line), gastric cancer (MFC cell line), pancreatic cancer (Pan02 cell line), prostate cancer (RM-1 cell line), colon cancer (CT26 cell line), ovarian cancer (ID8 cell line), neuroblastoma (Neuro-2a cell line), squamous cell carcinoma (SCC7 cell line), and bladder cancer (MB49 cell line).

**1. Evaluation of the effects of strains SWT1001, SWT1002, SWT1004, SWT1005, SWT1009, SWT1010, SWT1012, SWT1013, SWT1021, SWT1025, SWT1033, SWT1035, SWT1036, SWT1038, and SWT1063** modified **by hypoxia-specific gene expression cassettes on cancer cell invasion**

(1) The plasmid pSWT006 was introduced into the strains modified with the hypoxia-specific gene expression cassette via electroporation to enable the expression of the EGFP protein, thereby facilitating the observation of the cell invasion phenomenon.

(2) The modified strains carrying pSWT006 were streaked onto LB agar plates supplemented with D-alanine and incubated still overnight in a 37°C constant temperature incubator.

(3) A single colony of the modified strain carrying pSWT006 was picked and inoculated into 5 ml of fresh LB liquid medium containing 100 $\mu$g/ml D-alanine, and cultured for 16 hours in a constant temperature shaker at 37°C and 220 rpm.

(4) A volume of the culture equivalent to an $OD_{600}$ of 1.0 was taken and washed three times with PBS.

(5) In a 6-well plate used for cell culture, add $2.5 \times 10^6$ corresponding cancer cells per well, and incubate overnight in complete medium (DMEM base medium + 10% fetal bovine serum + penicillin/streptomycin) in a 37°C, 5% $CO_2$ incubator.

After culturing, cells from two wells were counted. Based on the cell count, the strains SWT1001, SWT1002, SWT1004, SWT1005, SWT1009, SWT1010, SWT1012, SWT1013, SWT1021, SWT1025, SWT1033, SWT1035, SWT1036, SWT1038, and SWT1063, each carrying pSWT006 modified by hypoxia-specific gene expression cassettes, were added to the remaining wells at a Multiplicity of Infection (MOI) of 200:1. The plates were placed in an anaerobic chamber with an oxygen concentration below 0.8% and co-cultured for 2 hours to allow for bacterial invasion of the cells. Subsequently, the cells were carefully washed with an equal volume of PBS, and the supernatant was removed. DMEM medium containing 100 $\mu$g/mL gentamicin was added, and the plates were further incubated for 24 hours in the anaerobic chamber ($O_2 < 0.8$%). Following incubation, images were captured using a fluorescence microscope. The invasion effects of the strains modified with the hypoxia-specific gene expression cassette on various cancer cell lines are shown in Figures 34-63.

The results demonstrate that the *Salmonella* strains containing the hypoxia-specific gene expression cassette possess a strong capacity to invade a wide variety of cancer cell lines. The intracellular growth and distribution of the various *Salmonella* strains carrying the hypoxia-specific gene expression cassette can be clearly observed, indicating that *Salmonella* invasion into cancer cells is universal.

**2. Cytotoxic Effect of Strains Containing a Hypoxia-Specific Gene Expression Cassette on Cancer Cells Under Hypoxic Conditions**

The strains were co-cultured with cancer cells under *in vitro* hypoxic conditions to verify the cytotoxic effect of strain SWT005 (YB1-like) and the *Salmonella* strains of the present invention containing a hypoxia-specific gene expression cassette (SWT1001, SWT1002, SWT1004, SWT1005, SWT1009, SWT1010, SWT1012, SWT1013, SWT1021, SWT1025, SWT1033, SWT1035, SWT1036, SWT1038, and SWT1063) on various cancer cells.

(1) Strain SWT005 was streaked onto LB agar plates supplemented with diaminopimelic acid (DAP). The modified strains SWT1001, SWT1002, SWT1004, SWT1005, SWT1009, SWT1010, SWT1012, SWT1013, SWT1021, SWT1025, SWT1033, SWT1035, SWT1036, SWT1038, and SWT1063 were separately streaked onto LB agar plates supplemented with D-alanine. All plates were incubated still overnight in a 37°C constant temperature incubator.

(2) A single colony of strain SWT005 was picked and inoculated into 5 ml of fresh LB liquid medium containing 100 $\mu$g/ml DAP. Single colonies of the modified strains SWT1001, SWT1002, SWT1004, SWT1005, SWT1009, SWT1010, SWT1012, SWT1013, SWT1021, SWT1025, SWT1033, SWT1035, SWT1036, SWT1038, and SWT1063 were separately picked and inoculated into 5 ml of fresh LB liquid medium containing 100 $\mu$g/ml D-alanine. All cultures were incubated for 16 hours in a constant temperature shaker at 37°C and 220 rpm.

(3) The optical density (OD) of the bacterial cultures was measured at 600 nm. A volume of culture equivalent to an $OD_{600}$ of 1.0 was taken, washed twice with PBS, and then washed once with the corresponding cell culture medium. The bacterial pellet was then resuspended in cell culture medium to achieve a Multiplicity of Infection (MOI) of 200:1.

(4) Each of the prepared bacterial suspensions was separately co-cultured with EMT6, K7M2, Hepa1-6, A549, B16F10, Renca, MFC, Pan02, RM-1, CT26, ID8, Neuro-2a, SCC7, and MB49 cells for 2 hours in an anaerobic chamber with an oxygen concentration below 0.8%. And then the cells were washed three times with PBS containing gentamicin. The medium was then replaced, and the cells were further incubated for 24 hours in the anaerobic chamber ($O_2 < 0.8$%).

(5) The CCK-8 reagent was mixed with the cell suspension from the above step and incubated for 1 hour.

(6) The absorbance was measured at a detection wavelength of 450 nm using a microplate reader. The percentage of cytotoxicity was calculated based on the absorbance readings.

Compared with bacterial strain SWT005, the cytotoxic effects of strains SWT1001, SWT1002, SWT1004, SWT1005, SWT1009, SWT1010, SWT1012, SWT1013, SWT1021, SWT1025, SWT1033, SWT1035, SWT1036, SWT1038, and SWT1063, each containing a hypoxia-specific gene expression cassette, on EMT6, K7M2, Hepa1-6, A549, B16F10, Renca, MFC, Pan02, RM-1, CT26, ID8, Neuro-2a, SCC7, and MB49 cells are shown in Figures 64-71.

The experimental results were analyzed using the T-test, with $p<0.05$ (*), $p<0.01$ (**), and $p\leq0.001$ (***) indicating significant differences.

The above results demonstrate that the *Salmonella* strains modified with the hypoxia-specific gene expression cassette exhibit a significant cytotoxic effect on a wide variety of cancer cells. Furthermore, their cytotoxic effect is stronger than that of strain SWT005 (YB1-like), thereby indicating the broad-spectrum applicability of the killing effect of the *Salmonella* strains modified with the hypoxia-specific gene expression cassette.

## 3. Evaluation of the Tumor-Targeting Specificity of Strains Containing the Hypoxia-Specific Gene Expression Cassette in Murine Tumor Model

To investigate the tumor-targeting specificity of the strains containing the hypoxia-specific gene expression cassette in murine tumor model, an EMT6 tumor mouse model was established.

BALB/c mice (purchased from Zhejiang Vital River Laboratory Animal Technology Co., Ltd., approximate weight 18 g, housed in a Specific Pathogen-Free (SPF) environment) were subcutaneously inoculated with $1\times10^6$ EMT6 cancer cells to establish a subcutaneous murine breast cancer model. Experiments were initiated 14-18 days post-inoculation when the tumor volume reached approximately 500 mm³.

The tumor-bearing mice were divided into a total of 17 groups: PBS (Vehicle) group, SWT005 group, SWT1001 group, SWT1002 group, SWT1004 group, SWT1005 group, SWT1009 group, SWT1010 group, SWT1012 group, SWT1013 group, SWT1021 group, SWT1025 group, SWT1033 group, SWT1035 group, SWT1036 group, SWT1038 group, and SWT1063 group, with 6 mice per group. The latter 16 groups of tumor-bearing mice were administered a dose of $1\times10^7$ CFU of the respective bacteria in a volume of 125 μl via tail vein injection. On day 1 (D1), day 5 (D5), and day 11 (D11) post-administration, mice were sacrificed and dissected. The bacterial distribution of the aforementioned strains was quantified in normal organs (heart, liver, spleen, lung, kidney) and in the tumor tissue. The results of the targeting specificity evaluation for each *Salmonella* strain containing the hypoxia-specific gene expression cassette, in comparison to the YB1-like strain, are shown in figures 72-76.

The above results demonstrate that the bacterial distribution of the *Salmonella* strains containing the hypoxia-specific gene expression cassette in the heart, liver, spleen, lung, and kidney of the mouse model was lower than that of the YB1-like strain at all time points (wherein the absence of a bar indicates complete clearance of the *Salmonella*). However, these strains were able to rapidly accumulate within the tumor at levels significantly higher than the YB1-like strain.

The experimental results were analyzed using T-test, where $p<0.05$ (*), $p<0.01$ (**), and $p\leq0.001$ (***) indicate a significant difference.

## 4. Evaluation of the Tumor Suppression Efficacy of Strains Containing the Hypoxia-Specific Gene Expression Cassette in Murine Tumor Model

To verify the inhibitory effect of the strains containing the hypoxia-specific gene expression cassette on tumor growth in mice, the present invention designed the tumor suppression study in the murine EMT6 tumor model, comparing the YB1-like strain SWT005 with the *Salmonella* strains containing the hypoxia-specific gene expression cassette: SWT1001, SWT1002, SWT1004, SWT1005, SWT1009, SWT1010, SWT1012, SWT1013, SWT1021, SWT1025, SWT1033, SWT1035, SWT1036, SWT1038, and SWT1063.

BALB/c mice (purchased from Zhejiang Vital River Laboratory Animal Technology Co., Ltd., approximate weight 18 g, housed in a Specific Pathogen-Free (SPF) environment) were subcutaneously inoculated with $1\times10^6$ EMT6 cancer cells to establish a subcutaneous murine breast cancer model. Experiments were initiated 14-18 days post-inoculation when the tumor volume reached approximately 500 mm³.

(1) Strain SWT005 was streaked onto LB agar plates supplemented with DAP, and the constructed *Salmonella* strains were streaked onto LB agar plates supplemented with D-alanine, followed by static incubation overnight in a 37°C constant temperature incubator.

(2) A single colony of strain SWT005 was picked and inoculated into 5 ml of LB liquid medium supplemented with DAP. Single colonies of the constructed *Salmonella* strains SWT1001, SWT1002, SWT1004, SWT1005, SWT1009, SWT1010, SWT1012, SWT1013, SWT1021, SWT1025, SWT1033, SWT1035, SWT1036, SWT1038, and SWT1063 were picked and inoculated into 5 ml of LB liquid medium supplemented with D-alanine. All strains incubated for 16 hours in a constant temperature shaker at 37°C and 220 rpm.

(3) The optical density (OD) of the bacterial cultures was measured at 600 nm. A volume of culture equivalent to an

$OD_{600}$ of 1.0 was taken and washed three times with PBS.

(4) The EMT6 model mice were administered with a dose of $1 \times 10^7$ CFU of the bacteria in a volume of 125 $\mu$l via tail vein injection.

(5) The day of administration was designated as Day 0. The length and width of the tumors were measured on days 1, 3, 5, 7, 9, and 11 post-administration, and the tumor volume was calculated according to the following formula:

$$Mouse\ Tumor\ Volume = (Length \times Width^2) \times 0.52$$

The tumor suppression effects of each *Salmonella* strain containing the hypoxia-specific gene expression cassette in the murine tumor model, as compared to the YB1-like strain SWT005, are shown in figures 77-80.

The experimental results were analyzed using T-test, where *p<0.05* (*), *p<0.01* (**), and *p≤0.001* (***) indicate a statistically significant difference.

Conclusion: The *Salmonella* strains containing the hypoxia-specific gene expression cassette exhibit a significant tumor growth suppression effect, which is superior to that of the YB1-like strain SWT005.

**Example 3: Construction of the Hypoxia-Specific Gene Expression Cassette with *dadX* as the Survival-essential Gene**

As alanine racemase, the genomes of bacteria such as *Salmonella* and *E. coli* contain two isoenzyme genes, namely the *alr* gene and the *dadX* gene. To verify the effect, accordingly, the survival-essential gene in the aforementioned validated hypoxia-specific gene expression cassette was replaced from the *alr* gene to its corresponding homologous gene, the *dadX* gene, to identify the oxygen-regulated effect of this hypoxia-specific **gene expression cassette**.

**(I) Integration of the Hypoxia-Specific Gene Expression Cassette into the *alr* Gene Locus on the Chromosome of Strain SWT007**

**1. Construction of the *dadX* Gene Knockout Attenuated Bacterium (SWT007)**

(1) A single colony of SWT003 was picked and inoculated into 5 ml of LB liquid medium, and placed in a constant temperature shaker at 32°C and 220 rpm for 16 hours of cultivation.

(2) The culture was inoculated into fresh LB liquid medium at a ratio of 1:100 and cultivation were continued for 2-3 hours until the bacterial density grew to an $OD_{600}=0.3$, at which point the culture flask was placed in a 42°C water bath and incubated with shaking for 15 minutes, then placed on ice to stand for 1 hour.

(3) The bacterial cells were washed 3 times with sterilized pure water.

(4) Preparation of the PCR product of SWTO78 and SWTO79.

Primers SWTO78 and SWTO79 were mixed with plasmid pSWT002 (as described above) and a high-fidelity PCR amplification enzyme system, and placed into a PCR amplification instrument.

The amplification program was: 95°C for 2 minutes; 30 cycles of 95°C for 30 seconds, 60°C for 30 seconds, 72°C for 60 seconds; 72°C for 10 minutes; 4°C for 5 minutes.

The amplified PCR product was purified and recovered using a DNA gel recovery system, and the concentration and purity of the recovered PCR product DNA were measured using a nanodrop.

(5) The recovered bacterial cells were mixed with 100 ng of the PCR product of SWTO78 and SWTO79, and electroporated at a voltage of 1.8 kV.

(6) The electroporated bacterial cells were spread onto plates containing 25 $\mu$g/mL chloramphenicol and cultured overnight in a constant temperature incubator at 32°C until single colonies grew.

(7) Positive clones were identified through colony PCR using primers SWTO59, SWTO5, SWTO6, and SWTO87 to verify the insertion of the chloramphenicol resistance gene, as shown in figure 81.

(8) The positive single clones were inoculated into 5 ml of LB medium and cultured for 16 hours in a constant temperature shaker at 37°C and 220 rpm, allowing the CRE recombinase to act and excise the chloramphenicol resistance gene.

**2. Construction of Plasmids Containing the Hypoxia-Specific Gene Expression Cassette with the *Salmonella dadX* Gene as the Survival-Essential Gene**

(1) A single colony of *Salmonella* strain SWT001 was picked with a pipette tip and mixed into a high-fidelity PCR amplification enzyme system with primers SWTO39 and SWTO40, and the mixture was placed into a PCR amplification instrument.

The amplification program was: 95°C for 2 minutes; 30 cycles of 95°C for 30 seconds, 60°C for 30 seconds, and 72°C for 60 seconds; followed by 72°C for 10 minutes; and 4°C for 5 minutes.

(2) The amplified PCR product (the *Salmonella dadX* gene (SEQ ID NO: 19)) was purified and recovered using a DNA gel recovery system, as shown in figure 2.

(3) The recovered product was mixed with a HindIII and XhoI restriction enzyme digestion system and incubated at 37°C for 1 hour, and the digested product was purified and recovered using a DNA gel recovery system.

(4) 5µg each of plasmids pOL1001, pOL1002, pOL1004, pOL1005, pOL1009, pOL1010, pOL1012, pOL1013, pOL1021, pOL1025, pOL1033, pOL1035, pOL1036, pOL1038, and pOL1063 were taken and separately mixed with a HindIII and XhoI restriction enzyme digestion system and incubated at 37°C for 1 hour; the vector fragments were recovered and mixed with the fragment from step (3), and ligation was performed using T4 DNA ligase.

(5) The ligation product was transformed into DH10B competent cells and plated on plates containing chloramphenicol and ampicillin, and single colonies were selected.

(6) The successfully constructed vectors were designated as pOL2001, pOL2002, pOL2004, pOL2005, pOL2009, pOL2010, pOL2012, pOL2013, pOL2021, pOL2025, pOL2033, pOL2035, pOL2036, pOL2038, and pOL2063, respectively, as shown in Table 4.

Table 4: Combination Table of Forward Hypoxia Promoters and Reverse Hyperoxia Promoters

|  | cyoA-S | phoH-S | lldP-S | argT-S | ydcl-S | phoH-E | lldP-E |
|---|---|---|---|---|---|---|---|
| yhbU-S | pOL2001 | pOL2002 | / | pOL2004 | pOL2005 | / | / |
| ynfK-E | pOL2009 | pOL2010 |  | pOL2012 | pOL2013 | / | / |
| tdcA-S | / | / | / | / | pOL2021 | / | / |
| tdcA-E | pOL2025 | / | / | / | / | / | / |
| yecH-E | pOL2033 | / | pOL2035 | pOL2036 | / | pOL2038 | / |
| focA-E | / | / | / | / | / | / | pOL2063 |

The survival-essential gene in Table 4 is the *dadX* gene from *Salmonella.*

**3. Construction of a Deficient and Attenuated Bacterial Strain Containing the Hypoxia-Specific Gene Expression Cassette and with the *Salmonella dadX* Gene as the Survival-Essential Gene**

(1) A single colony of the *dadX* gene knockout attenuated bacterium, SWT007, was picked and inoculated into 5 ml of LB liquid medium, and cultured for 16 hours in a constant temperature shaker at 32°C and 220 rpm.

(2) The culture was inoculated into fresh LB liquid medium at a ratio of 1:100, and cultivation was continued for 2-3 hours until the bacterial density reached an $OD_{600}$ of 0.3, at which point the culture flask was placed in a 42°C water bath and incubated with shaking for 15 minutes, then placed on ice to stand for 1 hour.

(3) The bacterial cells were washed 3 times with sterilized pure water.

(4) To prepare the PCR product for constructing the strain containing the hypoxia-specific gene expression cassette, primers SWTO85 and SWTO86 were mixed with the hypoxia-specific gene expression cassette plasmid (as shown in Table 4) and a high-fidelity PCR amplification enzyme system, and the mixture was placed into a PCR amplification instrument.

The amplification program was: 95°C for 2 minutes; 30 cycles of 95°C for 30 seconds, 60°C for 30 seconds, and 72°C for 120 seconds; followed by 72°C for 10 minutes; and 4°C for 5 minutes.

The amplified PCR product was purified and recovered using a DNA gel recovery system, and the concentration and purity of the recovered PCR product DNA were measured using a Nanodrop spectrophotometer.

(5) The recovered bacterial cells were mixed with 100 ng of the PCR product, and electroporation was performed at a voltage of 1.8 kV.

(6) The electroporated cells were plated on plates containing 25 μg/ml chloramphenicol and 100 μg/ml D-alanine, and incubated overnight in a constant temperature incubator at 32°C until single colonies grew.

(7) The positive single colony was inoculated into 5 ml of LB medium and cultured for 16 hours in a constant temperature shaker at 37°C and 220 rpm, allowing the CRE recombinase to act and excise the chloramphenicol resistance gene.

(8) Colony PCR was performed using corresponding primers to identify the clones; the identification results are shown in figure 82. The correspondence between the strains and the identification primers is shown in Table 5.

Table 5: Correspondence Table between Strains and Identification Primers

| Strain Name | Identification Primers | Identification Result |
|---|---|---|
| Strain SWT2001 (yhbU-S+dadX+cyoA-S) | SWTO98, 20; SWTO99, 20 | Figure 82A |
| Strain SWT2002 (yhbU-S+dadX+phoH-S) | SWTO98, 20; SWTO99, 20 | Figure 82A |
| Strain SWT2004 (yhbU-S+dadX+argT-S) | SWTO98, 20; SWTO99, 20 | Figure 82A |
| Strain SWT2005 (yhbU-S+dadX+ydcI-S) | SWTO98, 20; SWTO99, 20 | Figure 82A |
| Strain SWT2009 (ynfK-E+dadX+cyoA-S) | SWTO98, 22; SWTO99, 22 | Figure 82B |
| Strain SWT2010 (ynfK-E+dadX+phoH-S) | SWTO98, 22; SWTO99, 22 | Figure 82B |
| Strain SWT2012 (ynfK-E+dadX+argT-S) | SWTO98, 22; SWTO99, 22 | Figure 82B |
| Strain SWT2013 (ynfK-E+dadX+ydcI-S) | SWTO98, 22; SWTO99, 22 | Figure 82B |
| Strain SWT2033 (yecH-E+dadX+cyoA-S) | SWTO98, 28; SWTO99, 28 | Figure 82C |
| Strain SWT2035 (yecH-E+dadX+lldP-S) | SWTO98, 28; SWTO99, 28 | Figure 82C |
| Strain SWT2036 (yecH-E+dadX+argT-S) | SWTO98, 28; SWTO99, 28 | Figure 82C |
| Strain SWT2038 (yecH-E+dadX+phoH-E) | SWTO98, 28; SWTO99, 28 | Figure 82C |
| Strain SWT2021 (tdcA-S+dadX+ydcI-S) | SWTO98, 84; SWTO99, 84 | Figure 82D |
| Strain SWT2025 (tdcA-E+dadX+cyoA-S) | SWTO98, 26; SWTO99, 26 | Figure 82D |
| Strain SWT2063 (focA-E+dadX+lldP-E) | SWTO98, 96; SWTO99, 96 | Figure 82E |

**(II) Oxygen Adaptability V*alidation* Assay for a Library of *Salmonella* Strains (*dadX* gene knockout, cassette integrate*d at* the *Salmonella alr* gene locus) Containing the Hypoxia-Specific Gene Expression Cassette wi*t*h the *Salmonella dadX* Gene as the Survival-Essential Gene**

(1) A single colony of the strain was picked and inoculated into 5 ml of LB liquid medium supplemented with D-alanine, and cultured for 16 hours in a constant temperature shaker at 37°C and 200 rpm.

(2) After culturing was completed, the strain was diluted 10-fold, and the optical density (OD) was measured at 600 nm.

(3) The volume of bacterial culture corresponding to 1 OD of bacteria was calculated according to the following formula, and deionized water was added to reach 1 mL.

$$\text{Volume of bacterial culture for 1 OD} = 1/(\text{Absorbance} \times 10) \times 1000$$

(4) 10 μl of the above bacterial suspension was taken and spotted onto two LB agar plates not containing D-alanine, labeled as "1," and this was repeated three times, labeled as "a," "b," and "c."

(5) After performing a 10-fold serial dilution, 10 μl of the diluted suspension was then spotted onto the above medium, labeled as "2."

(6) This 10-fold serial dilution was continued in the same manner until labeled "6".

(7) One of the plates was placed in a 37°C anaerobic environment for cultivation, while the other plate was placed in a 37°C atmospheric environment (21% oxygen concentration) for cultivation.

The results of the oxygen adaptability validation assay for the strains containing the hypoxia-specific gene expression cassette are shown in figure 83-86.

The above results indicate that the essential gene within the hypoxia-specific gene expression cassette can be either *alr* or *dadX*, both of which can achieve a similar oxygen-regulated effect.

**Example 4: Replacement Study of Source Strains for Constructing Forward Hypoxia Promoter**

To supplement and expand the range of identical forward hypoxia promoters from different source strains, **the present invention designed th**is example.

**(I) Forward Hypoxia Promoter *yhbU***

In the present invention, the forward hypoxia promoter used in strains SWT1001, SWT1002, SWT1004, and SWT1005 is the *yhbU* from *Salmonella*. The forward hypoxia promoter used in strains SWT1065, SWT1066, SWT1068, and SWT1069 is the *yhbU* from *E. coli*.

For Gram-negative facultative anaerobes, their *yhbU* promoters all contain a conserved FNR binding site, wherein the FNR binding site conforms to the characteristic sequence "CTGCCNNNNATCAA," where N represents any base from A, T, C, or G. The *yhbU* promoter conforms to the characteristic of a forward hypoxia promoter "TTGATNNNNATCAA," wherein any base in the TTGAT and ATCAA sequences of the conserved binding site can be substituted, but the total number of substitutions does not exceed 3, and they are not 3 consecutively adjacent substituted bases.

The present invention has validated the *yhbU* promoters from *Salmonella* and *E. coli*, and the results demonstrate that *yhbU* promoters conforming to this conserved site all possess a corresponding oxygen-regulated function and can be used as the forward hypoxia promoter in the hypoxia-specific gene expression cassette described in the present invention.

Therefore, the source strains for the *yhbU* promoter used in the hypoxia-specific gene expression cassette designed in the present invention can be *Salmonella* (SEQ ID NO: 1), *Escherichia coli* (SEQ ID NO: 48), *Shigella* (SEQ ID NO: 49), *Yersinia* (SEQ ID NO: 50), *Enterobacter cloacae* (SEQ ID NO: 51), *Cronobacter* (SEQ ID NO: 52), *Klebsiella* (SEQ ID NO: 53), *Pantoea* (SEQ ID NO: 54), *Serratia* (SEQ ID NO: 55), or *Shimwellia* (SEQ ID NO: 56).

Through analysis of the *yhbU* promoter sequences from the above different source strains, it was found that although the promoter sequences differ considerably, their FNR binding sites are completely identical, all being CTGCCTTAAATCAA.

**Oxygen Adaptability Validation Study of Strains Containing the Hypoxia-Specific Gene Expression Cassette with *E. coli yhbU* promoter**

(1) Single colonies of strains SWT1065, SWT1066, SWT1068, and SWT1069 were separately picked and inoculated into 5 ml of fresh LB liquid medium containing 100 μg/ml D-alanine, and cultured for 16 hours in a constant temperature shaker at 37°C and 200 rpm.

(2) After culturing was completed, the strain was diluted 10-fold, and the optical density (OD) was measured at 600 nm.

(3) The volume of bacterial culture corresponding to 1 OD of bacteria was calculated according to the following formula, and deionized water was added to reach 1 mL.

$$\text{Volume of bacterial culture for 1 OD} = 1/(\text{Absorbance} \times 10) \times 1000$$

(4) 10 μl of the above bacterial suspension was taken and spotted onto two LB agar plates not containing D-alanine, labeled as "1," and this was repeated three times, labeled as "a," "b," and "c."

(5) After performing a 10-fold serial dilution, 10 μl of the diluted suspension was then spotted onto the above medium, labeled as "2."

(6) This 10-fold serial dilution was continued in the same manner until labeled "6".

(7) One of the plates was placed in a 37°C anaerobic environment for cultivation, while the other plate was placed in a 37°C atmospheric environment (21% oxygen concentration) for cultivation.

The results of the oxygen adaptability validation assay for *Salmonella* strains SWT1065, SWT1066, SWT1068, and SWT1069 are shown in figure 87.

The results show that replacing the forward hypoxia promoter from a *Salmonella* source to an *E. coli* source can achieve **a** similar oxygen-regulated effect.

**(II) Forward Hypoxia Promoter *ynfK***

The forward hypoxia promoter used in strains SWT1009, SWT1010, SWT1012, and SWT1013 is the *ynfK* from *Escherichia coli*.

The forward hypoxia promoter used in strains SWT1041, SWT1042, SWT1044, and SWT1045 is the *ynfK* from *Salmonella*.

For Gram-negative facultative anaerobes, their *ynfK* promoters all contain a conserved FNR binding site, wherein the FNR binding site conforms to the characteristic sequence "TTGCGNNNNCTCAA," where N represents any base from A, T, C, or G. The *ynfK* promoter conforms to the characteristic of a forward hypoxia promoter "TTGATNNNNATCAA," wherein any base in the TTGAT and ATCAA sequences of the conserved binding site can be substituted, but the total number of substitutions does not exceed 3, and they are not 3 consecutively adjacent substituted bases.

The present invention has validated the *ynfK* promoters from *Salmonella* and *E. coli*, and the results demonstrate that *ynfK* promoters conforming to this conserved site all possess a corresponding oxygen-regulated function and can be used as the forward hypoxic promoter in the hypoxia-specific gene expression cassette described in the present invention.

Therefore, the source strains for the *ynfK* promoter used in the hypoxia-specific gene expression cassette designed in the present invention can be *Salmonella* (SEQ ID NO: 6), *Escherichia coli* (SEQ ID NO: 2), *Serratia* (SEQ ID NO: 57), *Shigella* (SEQ ID NO: 58), or *Enterobacter ludwigii* (SEQ ID NO: 59).

**Oxygen Adaptability Validation Study of Strains Containing the Hypoxia-Specific Gene Expression Cassette with the *Salmonella ynfK* Promoter**

(1) Single colonies of strains SWT1041, SWT1042, SWT1044, and SWT1045 were separately picked and inoculated into 5 ml of fresh LB liquid medium containing 100 $\mu$g/ml D-alanine, and cultured for 16 hours in a constant temperature shaker at 37°C and 200 rpm.

(2) After culturing was completed, the strain was diluted 10-fold, and the optical density (OD) was measured at 600 nm.

(3) The volume of bacterial culture corresponding to 1 OD of bacteria was calculated according to the following formula, and deionized water was added to reach 1 mL.

$$\text{Volume of bacterial culture for 1 OD} = 1/ (\text{Absorbance} \times 10) \times 1000$$

(4) 10 $\mu$l of the above bacterial suspension was taken and spotted onto two LB agar plates not containing D-alanine, labeled as "1," and this was repeated three times, labeled as "a," "b," and "c."

(5) After performing a 10-fold serial dilution, 10 $\mu$l of the diluted suspension was then spotted onto the above medium, labeled as "2."

(6) This 10-fold serial dilution was continued in the same manner until labeled "6".

(7) One of the plates was placed in a 37°C anaerobic environment for cultivation, while the other plate was placed in a 37°C atmospheric environment (21% oxygen concentration) for cultivation.

The results of the oxygen adaptability validation assay for *Salmonella* strains SWT1041, SWT1042, SWT1044, and SWT1045 are shown in figure 88. The results show that replacing the forward hypoxic promoter from an *E. coli* source to a *Salmonella* source can achieve a similar oxygen-regulated effect.

The above results indicate that for promoters such as *yhbU* and *ynfK*, there are highly conserved FNR binding sites in different facultative anaerobes. By separately comparing the two promoters from *E. coli* and *Salmonella* when placed in the hypoxia-specific gene expression cassette, it was found that both possess a consistent oxygen-regulated function. From this, it can be determined that for the same gene promoter in different facultative anaerobes, promoters with a conserved FNR binding site fragment can be used for the construction of the hypoxia-specific gene expression cassette described in the present invention, and can achieve the same effect.

**Example 5: Study on the Replacement of the Source Organism for the Reverse hyperoxia**

**Promoter**

To supplement and expand the range of identical reverse hyperoxia promoters from different source strains, the present invention designed this example.

**(I) Reverse Hyperoxia Promoter *ydcI***

In the present invention, the reverse hyperoxia promoter used in strains SWT1005, SWT1013, and SWT1021 is the *ydcI* promoter from *Salmonella*.

The reverse hyperoxia promoter used in strains SWT1008, SWT1016, and SWT1024 is the *ydcI* promoter from *E. coli*.

For Gram-negative facultative anaerobes, their *ydcI* promoters all contain a conserved FNR binding site, wherein the FNR binding site conforms to the characteristic sequence "CTGCCNNNNATCAA," where N represents any base from A, T, C, or G. The *ydcI* promoter conforms to the characteristic of a reverse hyperoxia promoter "TTGATNNNNATCAA," wherein any base in the TTGAT and ATCAA sequences of the conserved binding site can be substituted, but the total number of substitutions does not exceed 3, and they are not 3 consecutively adjacent substituted bases; and it also conforms to the characteristic of a reverse hyperoxia promoter's ArcA binding site conforming to the pattern GTTAATTA, wherein any base can be substituted, but the total number of substitutions does not exceed 2.

The present invention has validated the *ydcI* promoters from *Salmonella* and *E. coli*, and the results demonstrate that *ydcI* promoters conforming to this conserved site all possess a corresponding oxygen-regulated function and can be used as the reverse hyperoxia promoter in the hypoxia-specific gene expression cassette described in the present invention.

Therefore, the source strain for the *ydcI* promoter used in the hypoxia-specific gene expression cassette designed in the present invention can be *Salmonella* (SEQ ID NO: 13) and *Escherichia coli* (SEQ ID NO: 16).

**Oxygen Adaptability Validation Study of Strains Containing the Hypoxia-Specific Gene Expression Cassette with the *E. coli ydcI* Promoter**

(1) Single colonies of strains SWT1008, SWT1016, and SWT1024 were separately picked and inoculated into 5 ml of fresh LB liquid medium containing 100 μg/ml D-alanine, and cultured for 16 hours in a constant temperature shaker at 37°C and 200 rpm.

(2) After culturing was completed, the strain was diluted 10-fold, and the optical density (OD) was measured at 600 nm.

(3) The volume of bacterial culture corresponding to 1 OD of bacteria was calculated according to the following formula, and deionized water was added to reach 1 mL.

$$\text{Volume of bacterial culture for 1 OD} = 1/\left(\text{Absorbance} \times 10\right) \times 1000$$

(4) 10 μl of the above bacterial suspension was taken and spotted onto two LB agar plates not containing D-alanine, labeled as "1," and this was repeated three times, labeled as "a," "b," and "c."

(5) After performing a 10-fold serial dilution, 10 μl of the diluted suspension was then spotted onto the above medium, labeled as "2."

(6) This 10-fold serial dilution was continued in the same manner until labeled "6".

(7) One of the plates was placed in a 37°C anaerobic environment for cultivation, while the other plate was placed in a 37°C atmospheric environment (21% oxygen concentration) for cultivation.

The results of the oxygen adaptability validation assay for *Salmonella* strains SWT1008, SWT1016, and SWT1024 are shown in figure 89.

The results show that replacing the reverse hyperoxia promoter from a *Salmonella* source to an *E. coli* source can achieve a similar oxygen-regulated effect.

**(II) Reverse Hyperoxia Promoter *mqo* Containing Two ArcA Binding Sites**

In the present invention, the reverse hyperoxia promoter used in strains SWT1004, SWT1012, and SWT1036 is the *argT* promoter from *Salmonella* (SEQ ID NO: 12).

The reverse hyperoxia promoter used in strains SWT1073, SWT1074, and SWT1075 is the *mqo* promoter from *E. coli* (SEQ ID NO: 62).

Both the *argT* promoter (SEQ ID NO: 12) and the *mqo* promoter (SEQ ID NO: 62) contain conserved dual ArcA binding sites, and conform to the characteristic of a reverse hyperoxia promoter's ArcA binding site conforming to the pattern GTTAATTA, wherein any base can be substituted, but the total number of substitutions does not exceed 2. The present invention has validated the *argT* promoter (SEQ ID NO: 12) and the *mqo* promoter (SEQ ID NO: 62), and the results demonstrate that promoters conforming to this characteristic of containing conserved dual ArcA binding sites all possess a corresponding oxygen-regulated function and can be used as the reverse hyperoxia promoter in the hypoxia-specific gene expression cassette described in the present invention.

Therefore, the reverse hyperoixa promoter used in the hypoxia-specific gene expression cassette designed in the present invention can be the *argT* promoter (SEQ ID NO: 12) or the *mqo* promoter (SEQ ID NO: 62), which contain dual ArcA binding sites.

**Oxygen Adaptability Validation Study of Strains Containing the Hypoxia-Specific Gene Expression Cassette with the *mqo* Promoter**

(1) Single colonies of strains SWT1073, SWT1074, and SWT1075 were separately picked and inoculated into 5 ml of fresh LB liquid medium containing 100 μg/ml D-alanine, and cultured for 16 hours in a constant temperature shaker at 37°C and 200 rpm.

(2) After culturing was completed, the strain was diluted 10-fold, and the optical density (OD) was measured at 600 nm.

(3) The volume of bacterial culture corresponding to 1 OD of bacteria was calculated according to the following formula, and deionized water was added to reach 1 mL.

$$\text{Volume of bacterial culture for 1 OD} = 1/\left(\text{Absorbance} \times 10\right) \times 1000$$

(4) 10 μl of the above bacterial suspension was taken and spotted onto two LB agar plates not containing D-alanine, labeled as "1," and this was repeated three times, labeled as "a," "b," and "c."

(5) After performing a 10-fold serial dilution, 10 μl of the diluted suspension was then spotted onto the above medium, labeled as "2."

(6) This 10-fold serial dilution was continued in the same manner until labeled "6".

(7) One of the plates was placed in a 37°C anaerobic environment for cultivation, while the other plate was placed in a 37°C atmospheric environment (21% oxygen concentration) for cultivation.

The results of the oxygen adaptability validation assay for *Salmonella* strains SWT1073, SWT1074, and SWT1075 are shown in figure 90.

The results show that replacing the reverse hyperoxia promoter from a *Salmonella* source to an *E. coli* source can achieve a similar oxygen-regulated effect.

The specific sequences of the forward hypoxia promoters, survival-essential genes, and reverse hyperoxia promoters of the present invention are shown in Table 6.

Table 6 The specific sequence of forward hypoxia promoters, survival-essential genes and reverse hyperoxia promoters of the present invention

| Sequence number | Description | Specific sequence |
|---|---|---|
| SEQ ID No.1 | *Salmonella yhbU* promoter | 5'-GCGTGAACGCAGTTTATCTAACACGAGCTTTACTCCCTGTTTCAATAGTCCTGTTATTTTGCCATATCCAATAAATCACATGGCGGCGTAAATCAACAATTGGGTCTGATTTTCTACCCCATTATTGGTGTTACCAATACGCCTTTAACTGCCTTAAATCAAAAATTGTCGCAGCAAGGTTAACTAAAATCCCTGTTCGTTAACAATTTTGCGTCTCAGACGTAACCCTCAGGATAA-3' |
| SEQ ID No.2 | *Escherichia coli ynfK* promoter | 5'-CGCTGCACTGGTAAAAGACGCGATGTATAACGGTTCTTTGTTGATTCGTCTGTTGCAGGGTTAACATTTTTTAACTGTTCTACCAAAATTTGCGCTATCTCAATTTGGGCCAGGAAAGCATAACTTAGACTTTCAAGGTTAATTATTTTCCTGGTTTATATTTGTGAAGCATAACGGTGGAGTTA-3' |

(continued)

| Sequence number | Description | Specific sequence |
|---|---|---|
| SEQ ID No.3 | *Salmonella tdcA* promoter | 5'-<br>CTCTTCATAAAACCCGTCAGTGTAAGCTGGCGGGTTTTTGCTTTTACCGGGCGAC AGGATGAATGACTGTCCACGACGCTATACCCAAAAGAAAGCGGCTTATCGGTCA GTATCATCTCTTCATAAAACCCGTCAGTGTAAGCTGGCGGGTTTTTGCTTTTACAG GGCGGCAGGATGAATGACTGTCCACGACGCTATACCCGAAAGAAAGCGGCTTAT CGGTCAGTTTCACCTCTTCATAAAACCCGTCAGTGTAAGCTGGCGGGTTTTTGCTT TTACAGGGCGGCAGGATGAATGACTGTCCACGACGCTATACCCGAAAGAAAGCG GCTTATCGGTCAGTTTCATCTCTTCATAAACCCCGTCAGTGTAAGCTGGCGGGTTT<br><br>TTGCTTTTACAGGGCGACAGGATGAATGACTGTCCACGACGCTATACCCGAAAGA AAGCGGCTTATCGGTCAGTTTCACCTCTTCATAAAATCCGCTTCGGCGGGTTTTTG CTTATCAGGCCGGTGAAGGGTATAGAGGTTGCAGAAGACGCCAGCGTCACCATC CGGCAATTAATTGACAGCCGTGGTGACATAATATTATTCGTTTTTGTGATGAGAA ACACAAAACGCTATGTATTGCGCTATTTTTAGTTGAAGTGAAAAGCAATCGATTT CTTTCACAAAATTACATCCTGAAAAACAGCAAGAAAGCGGTGTAAATTATTTGTT TTAAATGAAAATTAAATGTATGAAATATTTTGGTTAAAATTTTATTAAATGTTTCT TTGCAAACAAATAAGGTAAAAAAATGTGACGCGTAACGGTTTTTAAGCGCATTTA CCTTCGGTTTATACTTAAATTTGAGAAATTACGTTTTAAATTTAACGCTGGAATTG TCGATAAAATGTCCCGTAATTGATTGAAATATCGTCTTCATGGGATATCAAAAAT AGGAGATAAAATAGGCTGGTTATCGCCATAATATATCGTTATCCCGCCCCTGTTT TTTGATTGAAATCAGGCTAAGTTACTGCTTTATTTTTGTAACCTATTGAAATATCA GGTTTTCTTTATTTAATAAAAACCATGTAAAAATTGTGGTAAGTTAATTTGTGAGT GGTCGCACATAACCTTTAAGATTTAATTTGCTACACTTCCTGCCGTTCACAATGTT ATTTCAG-3' |
| SEQ ID No.4 | *Escherichia coli tdcA* promoter | 5'-<br>GCTCATTAAATTTAACTCAAATTTTGCCTGGTAATTATCCGGTAATTGCTTGAAAT ATGGTCTCAGCCCCTTTTTTGTATTAACCACATAACGAATGATGTTATCGCCATAA AATATGGTTATCCCCGTCATTTTTTTTGACAAAAATCAGGGTTTATGCTGATTTTT ATACTTTAACTTGTTGATATTTAAAGGTATTTAATTGTAATAACGATACTCTGGAA AGTATTGAAAGTTAATTTGTGAGTGGTCGCACATATCCTGTTCATTTCATTTTGAT ACACTTCATGCCGTCAATGAGGTAATTAACGTAGGTC-3' |
| SEQ ID No.5 | *Escherichia coli ynfK* promoter | 5'-<br>CGTAACGCTAAAACCAACACCGGCCGCAGCCAGCGGCATTGCCAGCATGCCTGC GCCAATTGTGGTTCCCGCCACGATAAAAACACTTCCCAGGGTTCTGTTTTTCACG CTTTCTTCTGTCCTGACGATCTTTATGAGTGATATCTGCGGCGCAGGTTAAGGCAT AACGGCTGCTTCGTCAAACCGACGTTACATATGGTGTAAATATAAATGTACAATA AAAGCATTCAGTCCTGCGCTGGCGCAAAGTCTTCATAAGCGGCAATCGCTACGC TAGGCTTTTTTTGAGGAGGG-3' |
| SEQ ID No.6 | *Salmonella ynfK* promoter | 5'-<br>AGACGCGATGTATAACGGCTCTTTGTTGATTCGTCTATTACAGGGTTAACATTTTT TAACTGTTGTACAAAAATTTGCGCTAACTCAAGCTGATTGCCCTTGCCATATCTTA GACTTTCTCCACTGTATTATTTTCCTGGCTTATATTTTCGAAGCATAACGGTGGAG TTAGTG-3' |
| SEQ ID No.7 | *Escherichia coli cydA* promoter | 5'-<br>GTTAATATTGGCGGGGTGGATTTATGCCTTTATTAGTAATCCTGAAACTCTGCGTC GTATTAGCCAGTGACCAAAAAAAGAATTAAGGTCAACCGTGCTGTTTTTGCTTCG TCTCTTTTTATCTTTAATTGCCAACCGAAACTAATTTCAGCCTTATAACTCACACA TTTTAAACATAAATGTCACTAAAGTTACCTTATTGAAACATGATTAACATAATTT GTAGGAATTGATATTTATCAATGTATAAGTCTTGGAAATGGGCATCAAAAAGAG ATAAATTGTTCTCGATCAAATTGGCTGAAAGGCGGTAATTTAGCTATAAATTGAT CACCGTCGAAAAATGCAAATTTGCTTCAACAAAAACCTGTTTATTGTAAGGATTT TGCGGCGTAATATATACGTGGGATCAATTTGAGTTTTTATTAACATGTTTGCAACC TTTCTTTACGCCGTTTTTGTGTGCATTCACATGGTATGATGAAAGTGTTCAAACAA ATTTCTATTGGGGCATGCGTGTGACCCTTTCTAACGGGGTTCACTCTCGGAGTCTT CATGCGATGAGCAAGGAGTC-3' |

(continued)

| Sequence number | Description | Specific sequence |
|---|---|---|
| SEQ ID No.8 | *Escherichia coli focA* promoter | 5'-AAAGCAGCATTCTGGGCAAAATAAAATCAAATAGCCTACGCAATGTAGGCTTAATGATTAGTCTGAGTTATATTACGGGGCGTTTTTTTAATGCCCCGCTTTACATATATTTGCATTAATAAAATAATTGTAATTATAAGGTTAAATATCGGTAATTTGTATTTAATAAATACGATCGATATTGTTACTTTATTCGCCTGATGCTCCCTTTTAATTAACTGTTTTAGCGGAGGATGCGGAAAAAATTCAACTCATTTGTTAATTTTTAAAATTTATTTTTATTTGGATAATCAAATATTTACTCCGTATTTGCATAAAAACCATGCGAGTTACGGGCCTATAAGCCAGGCGAGATATGATCTATATCAATTTCTCATCTATAATGCTTTGTTAGTATCTCGTCGCCGACTTAATAAAGAGAGAGTT-3' |
| SEQ ID No.9 | *Salmonella cyoA* promoter | 5'-AACATTATTTGCCGTTGTAATAACGACCCGGAATTATAACGGCTAAATAACCATCAATTGATAAAAAATATATTTGTCTTTTTGTGCGCAACAGAATTTTGGAAATTATTGGTAATTTCTACGCTACCTATTTTCTTTACACGATTCAGCCGATTAAATTTAAATTTCTGCCGTTGATTGTCCTTTTATTGATAATAAATTGTTAAGTAATTGTTTTATTTTTACATTGGTTATACCAATTATCCCTCAACATCCCACTTACAGACCTCATCCGTTATAACGCAAAGTTTCAACAGTGTCTTAAAGTTATTTCGACATACTTGGCAACATATGTGACATGCGCGGCAGAACTTGGTAACACAGAACCAAT-3' |
| SEQ ID No.10 | *Salmonella phoH* promoter | 5'-CCGACATGTCCGATAGCATTATTGCCATCGGACATATTTTACCAGGCGGCGTTATACGCCCGCCTGGTCCTCTGATAAGTCCCGGACCGATTGACTGAAGATGTTCAGGTAAATGAGGACGCACTCTCAAACTATTTTTAGCCCTTGAGCGCCGTTAACGCGGGCGTAATACATCTCGAGTAATCACTAACTAGCCATATATGAAATCGCCTGTTAATGGTACCAATAGCCTTGACGCAATAGAGTAATGACAAAAATTAAAACAAGTCAGCGTTACTGGCGTAAGTATGCCGCATAAAATTTTGCATAAATAATGCCGTTTTAGCGATGGGAGAGAGGACACGTTAATTATTCCATTTTAACCTTATATATGTTCAATATTCAATGGGTTATGGATGTTTTCAGCATTAATACCCGCAGTAGCTAATGATTATCTTTTTTAGTCTCCTGCCGATGAAATAATCGTGTAATCTTTCTGTAAGAGACTGACAATGACGCAATAATGTTTGGTTAATGCTTGGTGAATATATTGTTGCATTATTGATGTTTTGTGTTGTACTTAGTAGTAATAGCGGTAGTTCCCCGGCAGTGATGGTCACTCACTATGGAGATCGCGAATGGTAATGTCCGCACCAGGACACATTGTTTACAGTAGTTACAACACCCTGTACGGACATTCTCTCTCCGGTGGTGGTCTTGTCATCTTAAAAGCTCTCATCATTTCCCTTACTGACCATACCCATGACGTCATATGTGGTGCGCGTAGCCGTGTGTGGCGTCGTTTCAAAAAGCAAGCTAAGGCTTACAAGGAAGCCAACCCTCAGAT-3' |
| SEQ ID No.11 | *Salmonella lldP* promoter | 5'-TGAAGTAACAACACTCCCGACATCAAAGGCAATGGCATAATGCCATTGCCTTTTCACTTCCCTTTGCTGTCCTCTCCCCTCAATTGGCCCTACCAATTTTAGCCAGTTACCACAGCCTACTCACATTTCATTAAATTAACTCTACAATACCGTTGCCAATAAATAACATCTGGTTAACTATTTGTTGTCATTATCCATACACAACAATATTGGCAGGACCACTTTTACACATAATGTGACCCCAACGAGATGAGCAAAGATTCATCCCATTATGCGTGTGGTTCTCAGGAGACCTGCA-3' |
| SEQ ID No.12 | *Salmonella argT* promoter | 5'-TAGCGTTGTCTGTTTAGCGTGCATTATTGTCCCTGTTGCCCTGTTTCAGGGCAATTTTGTAACCGCGATCATATCCTCAACATTTAATTCGTTAAAAGCCGCCGAAACGCTGCGGTTCAACCGTCATACCTGCTATCTTCAACATCAGGACAATATTGCAACGTTTTATTAACAAATTTAACGTCGAATCGTTTTGCTGACGTGAAAATGGCATAAGACCTGCATGAAAAGTCTGCAAACACAACGCCACGTAAAACATAAGAAAATGACGCCACTTGAGGGGTATGT-3' |
| SEQ ID No.13 | *Salmonella ydcI* promoter | 5'-AATGTCATGCCTCCAGTGAATGTTACCTGGAGTGTAGAAAACCTCATCCATTCCGGGGAGAAGAATTTATAAAGTGTGATCCGACCTGAATTCATTATGCAGAACGGATTGACATGAGCTGGCATATCCCCGACCAGATCGCGGCTTAACACGCTTAACGGCAGTTGAATCATCAGTAAAATAAAACAGTTGTAACTTGAAATGGTAAAATGTTAATAATATTTTGCTATCAGGTTATCAAAAACAAACAACTTTACTTGTCACCCGCACGGCTCTGTTTTTAACATCGCTT-3' |

(continued)

| Sequence number | Description | Specific sequence |
|---|---|---|
| SEQ ID No.14 | *Escherichia coli phoH* promoter | 5'-TGTTTTTAGGCGGATAAGGCATTTGTGCGCAGATGCCTGATGCGACGCTTGCGCGTCTTATCATGCCTACAATCAGTGCGGGTTTGGTAGGCTGGATAAGGCGTTCACGCCGCATCCGGCGATCGTGCACTGATGCCTGATGCAAATCCTGCTGAAAGCACACAGCTTTTTTCATCACTGTCATCACTCTGTCATCTTTCCAGTAGAAACTAATGTCACTGAAATGGTGTTTTATAGTTAAATATAAGTAAATATATTGTTGCAATAAATGCGAGATCTGTTGTACTTATTAAGTAGCAGCGGAAGTTCCCGGCAGTGATAGTCAGTCACTATGGAGATCGCGG-3' |
| SEQ ID No.15 | *Escherichia coli lldP* promoter | 5'-GAACACAGTATCTACAGGGTGATTCTGCACATTCCTATAGGCCGAGTAAGGTGTTCACGCCGCATCCGGCAAGATAAGGCGCTCTGGATCAACAACCTAAGGGCAATTCTCTGATGAGGATTGCCCTTTTCTTTACCAGACATCTCCCCCCACAAGAATTGGCCCTACCAATTCTTCGCTTATCTGACCTCTGGTTCACAATTTCCCAATTAAAACTCACATCAATGTTGCCAATACATAACATTTAGTTAACCATTCATTGTCATTATCCCTACACAACACAATTGGCAGTGCCACTTTTACACAACGTGTGACAAGGAGATGAGCAACAGACTCATTACACGATGTGCGTGGACTCCAGGAGACCTGCA-3' |
| SEQ ID No.16 | *Escherichia coli ydcI* promoter | 5'-CTGACATTGCCTGCGAAAACTGTTCCCGAATCTCATCCGCCGTGATGCTGTTCGCCATAGTGTCTGCCTCCAGTGGTCAGTAATCTGGAGTGTAGGTAACCGCATTCACTCTTGCGGGAAGAATTTACAAACTGTGATCTCGCCGCGAAAACATCAATATTATCCATTTTGCTGTAACATAATTGCTTTAATTGTTAATAATATTTTGCAATCAAGTTATCATAATCAAACAACTTCACTTGTCAGCGACACCGCTTCGTTTTTAACATCGCTT-3' |
| SEQ ID No.17 | *Salmonella alr* gene | 5'-ATGCAAGCGGCAACAGTCGTCATTAACCGCCGCGCTCTGCGACACAACCTGCAACGTCTGCGTGAACTGGCGCCTGCCAGTAAGCTGGTTGCGGTGGTGAAAGCGAACGCTTATGGACACGGTCTTCTGGAGACCGCGCGAACGCTCCCTGATGCTGACGCTTTTGGCGTGGCGCGTCTTGAAGAGGCTCTACGTCTGCGAGCGGGCGGGATCACGCAGCCAATCCTGCTGCTGGAGGGTTTTTTCGACGCCGCCGATTTGCCGACCATTTCCGCGCAATGTCTGCATACCGCCGTACATAATCAAGAGCAGCTTGCCGCCCTGGAGGCGGTGGAGCTGGCGGAGCCGGTAACGGTCTGGATGAAGCTGGATACCGGTATGCATCGTCTCGGCGTGCGTCCCGAAGAGGCGGAGGCGTTCTACCAGCGTCTGACGCACTGTAAAAATGTACGCCAGCCGGTGAATATCGTCAGCCATTTTGCCCGTGCGGATGAGCCGGAATGCGGCGCTACCGAACATCAGCTCGACATTTTTAATGCCTTCTGTCAGGGTAAACCCGGTCAGCGCTCTATTGCCGCGTCTGGCGGTATCCTGCTGTGGCCGCAGTCTCACTTTGACTGGGCGCGTCCGGGCATCATTTTGTATGGCGTATCGCCGCTGGAGCACAAACCCTGGGGGCCGGATTTTGGTTTTCAGCCGGTGATGTCCTTAACCTCCAGTTTGATCGCGGTGCGTGACCACAAAGCGGGCGAACCGGTGGGCTACGGCGGGACATGGGTGAGTGAGCGCGACACGCGCCTGGGCGTGGTGGCGATGGGTTATGGCGATGGCTACCCACGAGCGGCGCCTTCCGGTACGCCAGTACTGGTCAATGGTCGTGAAGTTCCGATTGTCGGGCGGGTGGCGATGGATATGATTTGCGTAGATTTGGGGCCAAACGCGCAGGATAACGCGGGCGATCCGGTGGTCTTATGGGGTGAAGGTCTGCCGGTTGAACGTATCGCTGAAATGACAAAAGTAAGTGCTTACGAACTTATCACGCGCCTGACCTCAAGGGTGGCGATGAAGTATATTGATTAA-3' |
| SEQ ID No.18 | *Salmonella alr* protein | MQAATVVINRRALRHNLQRLRELAPASKLVAVVKANAYGHGLLETARTLPDADAFGVARLEEALRLRAGGITQPILLLEGFFDAADLPTISAQCLHTAVHNQEQLAALEAVELAEPVTVWMKLDTGMHRLGVRPEEAEAFYQRLTHCKNVRQPVNIVSHFARADEPECGATEHQLDIFNAFCQGKPGQRSIAASGGILLWPQSHFDWARPGIILYGVSPLEHKPWGPDFGFQPVMSLTSSLIAVRDHKAGEPVGYGGTWVSERDTRLGVVAMGYGDGYPRAAPSGTPVLVNGREVPIVGRVAMDMICVDLGPNAQDNAGDPVVLWGEGLPVERIAEMTKVSAYELITRLTSRVAMKYID |

(continued)

| Sequence number | Description | Specific sequence |
|---|---|---|
| SEQ ID No.19 | *Salmonella dadX* gene | 5'-ATGACCCGCCCTATACAGGCCAGCCTTGATTTACAGGTAATGAAACAAAATTTGGCTATTGTGCGCCGGGCGGCCCCAGAGGCGCGCGTCGGTCGGTAGTGAAAGCCAACGCCTACGGCCACGGTATTGAACGCGTCTGGAGCGCGCTGGGCGCCACAGATGGTTTCGCTATGCTCAACCTTGAAGAGGCGATTACCCTGCGTGAGCGCGGGTGGAAAGGGCCGATATTGATGCTGGAGGGGTTTTTCCATGCGCAAGACCTGGAGGCGTATGACACTTATCGGCTGACCACCTGCATCCACAGTAACTGGCAGTTGAAAGCGCTGCAAAATGCGCGGCTTAACGCGCCGCTGGATATCTATGTCAAAGTCAACAGCGGCATGAACCGACTCGGTTTTCAGCCTGAGCGGGCGCAGACCGTCTGGCAGCAATTGCGGGCAATGCGCAATGTCGGTGAAATGACCCTGATGTCACATTTTGCTCAGGCCGATCATCCGGAAGGTATCGGGGAGGCGATGAGGCGTATTGCGCTGGCGACGGAAGGTCTTCAGTGTGCATACTCGTTATCAAATTCGGCAGCGACGCTGTGGCATCCCCAGGCGCATTATGACTGGGTCAGGCCGGGCATTATTTTGTACGGCGCGTCGCCGTCAGGACAGTGGCGGGATATTGCCGATACCGGACTAAAACCTGTGATGACGCTGAGTAGCGAAATTATTGGCGTGCAGACGCTGAGCGCGGGTGAAAGGGTAGGCTATGGCGGAGGGTATTCTGTGACTCAGGAACAACGCATTGGCATCGTCGCGGCGGGTTATGCCGATGGCTATCCACGCCATGCGCCGACCGGGACGCCTGTGCTGGTGGACGGTATCCGTACCAGAACAGTAGGCACCGTTTCAATGGATATGCTGGCGGTGGATTTGACGCCGTGTCCGCAGGCGGGAATCGGCACGCCGGTTGAATTATGGGGCAAAGAAATTAAGGTCGATGATGTCGCTTCTGCGGCAGGCACGCTGGGCTATGAGCTACTGTGCGCCGTAGCGCCGCGTGTGCCGTTTGTGACAACGTAA-3' |
| SEQ ID No.20 | *Salmonella* dadX protein | MTRPIQASLDLQVMKQNLAIVRRAAPEARVWSVVKANAYGHGIERVWSALGATDGFAMLNLEEAITLRERGWKGPILMLEGFFHAQDLEAYDTYRLTTCIHSNWQLKALQNARLNAPLDIYVKVNSGMNRLGFQPERAQTVWQQLRAMRNVGEMTLMSHFAQADHPEGIGEAMRRIALATEGLQCAYSLSNSAATLWHPQAHYDWVRPGIILYGASPSGQWRDIADTGLKPVMTLSSEIIGVQTLSAGERVGYGGGYSVTQEQRIGIVAAGYADGYPRHAPTGTPVLVDGIRTRTVGTVSMDMLAVDLTPCPQAGIGTPVELWGKEIKVDDVASAAGTLGYELLCAVAPRVPFVTT |
| SEQ ID No.21 | *Escherichia coli alr* gene | 5'-ATGCAAGCGGCAACTGTTGTGATTAACCGCCGCGCTCTGCGACACAACCTGCAACGTCTTCGTGAACTGGCCCCTGCCAGTAAAATGGTTGCGGTGGTGAAAGCGAACGCTTATGGTCACGGTCTTCTTGAGACCGCGCGAACGCTCCCCGATGCTGACGCCTTTGGCGTAGCCCGTCTCGAAGAAGCTCTGCGACTGCGTGCGGGGGGGAATCACCAAACCTGTACTGTTACTCGAAGGCTTTTTTGATGCCAGAGATCTGCCGACGATTTCTGCGCAACATTTTCATACCGCCGTGCATAACGAAGAACAGCTGGCTGCGCTGGAAGAGGCTAGCCTGGACGAGCCGGTTACCGTCTGGATGAAACTCGATACCGGTATGCACCGTCTGGGCGTAAGGCCGGAACAGGCTGAGGCGTTTTATCATCGCCTGACCCAGTGCAAAAACGTTCGTCAGCCGGTGAATATCGTCAGCCATTTTGCGCGCGCGGATGAACCAAAATGTGGCGCAACCGAGAAACAACTCGCTATCTTTAATACCTTTTGCGAAGGCAAACCTGGTCAACGTTCCATTGCCGCGTCGGGTGGCATTCTGCTGTGGCCACAGTCGCATTTTGACTGGGTGCGCCCGGGCATCATTCTTTATGGCGTCTCGCCGCTGGAAGATCGCTCCACCGGTGCCGATTTTGGCTGTCAGCCAGTGATGTCACTAACCTCCAGCCTGATTGCCGTGCGTGAGCATAAAGCCGGAGAGCCTGTTGGTTATGGTGGAACCTGGGTAAGCGAACGTGATACCCGTCTTGGCGTAGTCGCGATGGGCTATGGCGATGGTTATCCGCGCGCCGCGCCGTCCGGTACGCCAGTGCTGGTGAACGGTCGCGAAGTACCGATTGTCGGGCGCGTGGCGATGGATATGATCTGCGTAGACTTAGGTCCACAGGCGCAGGACAAAGCCGGGGATCCGGTCATTTTATGGGGCGAAGGTTTGCCCGTAGAACGTATCGCTGAAATGACGAAAGTAAGCGCTTACGAACTTATTACGCGCCTGACTTCAAGGGTCGCGATGAAATACGTGGATTAA-3' |
| SEQ ID No.22 | *Escherichia coli* alr protein | MQAATVVINRRALRHNLQRLRELAPASKMVAVVKANAYGHGLLETARTLPDADAFGVARLEEALRLRAGGITKPVLLLEGFFDARDLPTISAQHFHTAVHNEEQLAALEEASLDEPVTVWMKLDTGMHRLGVRPEQAEAFYHRLTQCKNVRQPVNIVSHFARADEPKCGATEKQLAIFNTFCEGKPGQRSIAASGGILLWPQSHFDWVRPGIILYGVSPLEDRSTGADFGCQPVMSLTSSLIAVREHKAGEPVGYGGTWVSERDTRLGVVAMGYGDGYPRAAPSGTPVLVNGREVPIVGRVAMDMICVDLGPQAQDKAGDPVILWGEGLPVERIAEMTKVSAYELITRLTSRVAMKYVD |

(continued)

| Sequence number | Description | Specific sequence |
|---|---|---|
| SEQ ID No.23 | *Escherichia coli dadX* gene | 5'-ATGACCCGTCCGATACAGGCCAGCCTCGATCTGCAGGCATTAAAACAGAATCTGT CCATTGTCCGCCAGGCCGCGACGCACGCGCGCGTCTGGTCGGTGGTAAAAGCGA ACGCTTACGGGCATGGTATTGAGCGTATCTGGAGCGCGATCGGGGCCACCGATG GCTTTGCATTGCTTAACCTGGAAGAGGCAATAACGTTACGTGAGCGCGGCTGGAA AGGACCGATCCTGATGCTGGAAGGATTTTTCCATGCTCAGGATCTGGAGATTTAT GACCAGCACCGCCTGACCACCTGCGTACACAGCAACTGGCAGCTCAAAGCACTG CAAAATGCGCGGCTAAAAGCACCGTTGGATATTTATCTTAAAGTGAACAGTGGG ATGAATCGGTTGGGCTTCCAGCCCGATCGCGTGCTTACCGTCTGGCAGCAGTTGC GGGCAATGGCGAATGTTGGCGAAATGACCCTGATGTCGCATTTTGCCGAAGCGG AACATCCTGATGGAATTTCCGGCGCGATGGCGCGTATTGAGCAGGCGGCGGAGG GGCTGGAGTGTCGGCGTTCGTTGTCCAATTCGGCGGCGACTCTGTGGCACCCGGA AGCGCATTTTGACTGGGTTCGGCCTGGCATTATTTTGTATGGCGCTTCGCCGTCCG GTCAGTGGCGTGATATCGCCAATACCGGATTACGTCCGGTGATGACGCTAAGCAG TGAGATTATTGGTGTCCAGACGCTAAAAGCGGGCGAGCGTGTGGGCTACGGCGG TCGCTATACTGCGCGCGATGAACAGCGAATCGGCATTGTCGCCGCAGGGTACGCC GACGGTTATCCGCGCCACGCGCCTACCGGTACCCCTGTTTTAGTGGACGGCGTGC GCACCATGACGGTGGGGACCGTCTCGATGGATATGCTAGCGGTCGATTTAACGCC TTGCCCGCAGGCGGGTATTGGTACGCCGGTTGAGCTGTGGGGCAAGGAGATCAA AATTGATGATGTCGCCGCCGCTGCCGGAACGGTGGGCTATGAGTTGATGTGCGCG CTGGCGCTACGCGTCCCGGTTGTGACGGTGTAA-3' |
| SEQ ID No.24 | *Escherichia coli dadX* protein | MTRPIQASLDLQALKQNLSIVRQAATHARVWSVVKANAYGHGIERIWSAIGATDGF ALLNLEEAITLRERGWKGPILMLEGFFHAQDLEIYDQHRLTTCVHSNWQLKALQNAR LKAPLDIYLKVNSGMNRLGFQPDRVLTVWQQLRAMANVGEMTLMSHFAEAEHPDG ISGAMARIEQAAEGLECRRSLSNSAATLWHPEAHFDWVRPGIILYGASPSGQWRDIA NTGLRPVMTLSSEIIGVQTLKAGERVGYGGRYTARDEQRIGIVAAGYADGYPRHAPT GTPVLVDGVRTMTVGTVSMDMLAVDLTPCPQAGIGTPVELWGKEIKIDDVAAAAGT VGYELMCALALRVPVVTV |
| SEQ ID No.25 | *Shigella alr* gene | 5'-ATGCAAGCGGCAACTGTTGTGATTAACCGCCGCGCTCTGCGACACAACCTGCAAC GTCTTCGTGAACTGGCACCTGCCAGTAAAATGGTTGCGGTGGTGAAAGCGAACG CTTATGGTCACGGTCTTCTTGAGACCGCGCGAACGCTCCCCGATGCTGACGCCTT TGGCGTAGCCCGTCTCGAAGAAGCTCTGCGACTGCGTGCGGGGGGGAATCACCAA ACCTGTACTGTTACTCGAAGGCTTTTTTGATGCCAGAGATCTGCCGACGATTTCTG CGCAACATTTTCATACCGCCGTGCATAACGAAGAACAGCTGGCTGCGCTGGAAG AGGCTAGCTTGGACGAGCCGGTTACCGTCTGGATGAAACTCGATACCGGTATGCA CCGTCTGGGCGTAAGGCCGGAACAGGCTGAGGCGTTTTATCATCGCCTGACCCAG TGCAAAAACGTTCGTCAGCCGGTGAATATCGTCAGCCATTTTGCGCGCGCGGATG AACCAAAATGTGGCGCAACCGAGAAACAACTCGCTATCTTTAATACCTTTTGCGA AGGCAAACCTGGTCAACGTTCCATTGCCGCGTCGGGTGGCATTCTGCTGTGGCCA CAGTCGCATTTTGACTGGGTGCGCCCGGGCATCATTCTTTATGGCGTCTCGCCGCT GGAAGATCGCTCCACCGGTGCCGATTTTGGCTGTCAGCCAGTGATGTCACTAACC TCCAGCCTGATTGCCGTGCGTGAGCATAAAGCCGGAGAGCCTGTTGGTTATGGTG GAACCTGGGTAAGCGAACGTGATACCCGTCTTGGCGTAGTCGCGATGGGCTATG GCGATGGTTATCCGCGCGCCGCGCCGTCCGGTACGCCAGTGCTGGTGAACGGTCG CGAAGTACCGATTGTCGGGCGCGTGGCGATGGATATGATCTGCGTAGACTTAGGT CCACAGGCGCAGGACAAAGCCGGGGATCCGGTCATTTTATGGGGCGAAGGTTTG CCCGTAGAACGTATCGCTGAAATGACGAAAGTAAGCGCTTACGAACTTATTACGC GCCTGACTTCAAGGGTCGCGATGAAATACGTGGATTAA-3' |
| SEQ ID No.26 | *Shigella alr* protein | MQAATVVINRRALRHNLQRLRELAPASKMVAVVKANAYGHGLLETARTLPDADAF GVARLEEALRLRAGGITKPVLLLEGFFDARDLPTISAQHFHTAVHNEEQLAALEEASL DEPVTVWMKLDTGMHRLGVRPEQAEAFYHRLTQCKNVRQPVNIVSHFARADEPKC GATEKQLAIFNTFCEGKPGQRSIAASGGILLWPQSHFDWVRPGIILYGVSPLEDRSTGA DFGCQPVMSLTSSLIAVREHKAGEPVGYGGTWVSERDTRLGVVAMGYGDGYPRAA PSGTPVLVNGREVPIVGRVAMDMICVDLGPQAQDKAGDPVILWGEGLPVERIAEMT KVSAYELITRLTSRVAMKYVD |

(continued)

| Sequence number | Description | Specific sequence |
|---|---|---|
| SEQ ID No.27 | *Shigella dadX* gene | 5'-ATGACCCGTCCGATACAGGCCAGCCTCGATCTGCAGGCATTAAAACAGAATCTGTCCATTGTCCGCCAGGCCGCGCCGCACGCGCGCGTCTGGTCGGTGGTAAAAGCGAACGCTTACGGGCATGGTATTGAGCGTATCTGGAGCGCGCTCGGGGCCACCGATGGCTTTGCATTACTTAACCTGGAAGAGGCAATAACGTTACGTGAGCGCGGCTGGAAGGGGCCGATCCTGATGCTGGAAGGATTTTTCCATGCTCAGGATCTGGAGATTTATGACCAGCACCGCCTGACCACCTGCGTACACAGCAACTGGCAGCTCAAAGCACTGCAAAATGCGCGGCTAAAAGCACCGTTGGATATTTATCTTAAAGTGAACAGTGGGATGAATCGGTTGGGCTTCCAGTCCGATCGCGTGCTTACCGTCTGGCAGCAGTTGCGGGCAATGGCGAATGTTGGCGAAATGACCCTGATGTCGCATTTTGCTGAAGCGGAACATCCTGATGGAATTTCCGGCGCGATGGCGCGTATTGAGCAGGCGGCGGAGGGGCTGGAGTGTCGGCGTTCGTTGTCCAATTCGGCGGCGACTCTGTGGCACCCGGAAGCGCATTTTGACTGGGTTCGGCCTGGCATTATTTTGTATGGCGCTTCGCCGTCCGGTCAGTGGCGTGATATCGCCAATACCGGATTACGTCCGGTGATGACGCTAAGCAGTGAGATTATTGGTGTCCAGACGCTAAAAGCGGGCGAGCGTGTGGGCTACGGCGGTCGCTATACTGCGCGCGATGAACAGCGAATCGGCATTGTCGCCGCAGGATACGCCGACGGTTATCCGCGCCATGCGCCTACCGGTGCTCCTGTTTTAGTGGACGGCGTGCGCACCATGACGGTGGGGACCGTCTCGATGGATATGCTGGCGGTTGATTTAACGCCTTGCCCGCAGGCGGGGATTGGTACGCCGGTTGAGCTGTGGGGCAAGGAGATCAAAATTGATGATGTCGCCGCCGCTGCCGGAACGGTGGGCTATGAGTTGATGTGCGCACTGGCGTTACGCGTCCCGGTTGTGACAGTGTAA-3' |
| SEQ ID No.28 | *Shigella* dadX protein | MTRPIQASLDLQALKQNLSIVRQAAPHARVWSVVKANAYGHGIERIWSALGATDGFALLNLEEAITLRERGWKGPILMLEGFFHAQDLEIYDQHRLTTCVHSNWQLKALQNARLKAPLDIYLKVNSGMNRLGFQSDRVLTVWQQLRAMANVGEMTLMSHFAEAEHPDGISGAMARIEQAAEGLECRRSLSNSAATLWHPEAHFDWVRPGIILYGASPSGQWRDIANTGLRPVMTLSSEIIGVQTLKAGERVGYGGRYTARDEQRIGIVAAGYADGYPRHAPTGAPVLVDGVRTMTVGTVSMDMLAVDLTPCPQAGIGTPVELWGKEIKIDDVAAAAGTVGYELMCALALRVPVVTV |
| SEQ ID No.29 | *Klebsiella alr* gene | 5'-ATGCAAGCGGCAACTGTAGTCATTAACCGCCGCGCTCTGCGACACAACCTGCAACGTCTGCGTGAACTGGCGCCCGCCAGCAAACTAGTTGCGGTGGTGAAAGCGAACGCCTACGGACACGGTCTTCTTGAGACCGCGCGAACGCTCCCTGACGCCGATGCTTTTGGCGTCGCCCGTCTTGAAGAAGCTCTGCGCCTGCGCGCGGGCGGTATCGGCCAGCCGATTCTGCTGCTGGAGGGGTTCTTTGAGGCTGCCGACCTGGCGGTGATTTCCGCCCAGCGCCTGCACACGGCGGTACACAGCCCGGAACAGCTGGCGGCGCTGGAGGAAGCGGATTTACCCGAGCCGGTCACCGTATGGATGAAGCTGGATACCGGAATGCATCGATTAGGCGTTCTGCCTGAGCAGGCCGAAGCGTTTTATCAGCGTCTTAGCCAGTGTAAAAACGTCCGCCAGCCGGTCAACGTGGTCAGCCATTTTGCCCGTGCGGATGAGCCTGAGTGCGGCGCGACCGAACGTCAGCTGGATATCTTTACCACCTTCACGGAAGGGAAACCGGGACTGCGCTCTATCGCCGCATCCGGCGGTATATTGCTGTGGCCCCAGTCGCATTTCGACTGGGCGCGTCCGGGGATCATTCTCTACGGCGTGTCGCCGCTCGACGGTAGCTCTACCGGCGCGGACTTTGGCTGCCAGCCGGTGATGTCTCTGACCTCGAGCCTGATTGCGGTCCGCGAGCATAAAGCCGGGGAGCCGGTGGGCTACGGCGGAACCTGGATCAGCGAGCGTGATACGCGTTTAGGGGTGGTGGCGATGGGCTACGGTGACGGCTATCCGCGCGCTGCGCCGTCCGGGACCCCGGTGCTGGTCAACGGCCGCGAAGTGCCGATTGTGGGTCGCGTGGCGATGGATATGATTTGCGTCGATCTCGGGCCCGAAGCGCAGGATAAATCGGGCGACCCGGTGGTGCTGTGGGGCGAAGGCCTGCCCGTCGAGCGTATTGCCGAAATCACAAAAGTAAGTGCTTACGAACTTATTACCCGCCTGACTTCACGGGTATCAATGAAGTACGTGGATTGA-3' |
| SEQ ID No.30 | *Klebsiella* alr protein | MQAATVVINRRALRHNLQRLRELAPASKLVAVVKANAYGHGLLETARTLPDADAFGVARLEEALRLRAGGIGQPILLLEGFFEAADLAVISAQRLHTAVHSPEQLAALEEADLPEPVTVWMKLDTGMHRLGVLPEQAEAFYQRLSQCKNVRQPVNVVSHFARADEPECGATERQLDIFTTFTEGKPGLRSIAASGGILLWPQSHFDWARPGIILYGVSPLDGSSTGADFGCQPVMSLTSSLIAVREHKAGEPVGYGGTWISERDTRLGVVAMGYGDGYPRAAPSGTPVLVNGREVPIVGRVAMDMICVDLGPEAQDKSGDPVVLWGEGLPVERIAEITKVSAYELITRLTSRVSMKYVD |

(continued)

| Sequence number | Description | Specific sequence |
|---|---|---|
| SEQ ID No.31 | *Klebsiella dadX* gene | 5'-ATGTCTCGTCCTGTAAAGGCCAGCATTGATATGTCAGCCTTACGCCAGAATCAGCACATTGTTCGTCGCGCGGCACCCGGTTCGCGCCTGTGGGCGGTGGTCAAAGCCAATGCTTATGGCCACGGTCTGGCGAGGGTCTGGAACGCGCTTAGCGCCGCGGACGGTTTCGCGATGCTGAATCTTGAAGAGGCGATCCTGCTGCGCGAGCTGGGCTGGAAAGGGCCAATACTGATGCTGGAAGGCTTTTTCCACGCCGATGAGCTGGCCCTGTTTGATAAGTACCGTTTGACCACCAGCGTGCACAGTAACTGGCAAATCAAGGCGCTGCAGCAGGCGAAGCTGCACGCGCCGCTCGATATCTACGTCAAGGTGAATAGCGGGATGAATCGGCTGGGGGTTTATGCCGGATCGTCTGCACGTCGTCTGGCAGCAGCTGCGAGCGCTGCATAACGTCAGTGAAATGACGTTAATGTCACATTTTGCCGAAGCGGAAAACCCGGACGGCATCGTGGAGCCGATGCGGCGTATTGAGCAGGCGGCGGAAGGACTGGATTGCCCGCGCTCGCTGGCGAATTCGGCGGCCACCCTGTGGCATCCGGAGTCGCATTTTAACTGGGTGAGGCCGGGGGATCGTGCTGTACGGCGCTTCGCCTTCCGGCCTGTGGCAGGATGTGGCCAACACCGGGCTGAAGCCGGTGATGACGCTAAGCAG CGAAATTATTGCCGTACAGAACCTGAAAGCCGGTGAAGCGGTCGGCTACGGCGCAACCTGGCGTACGGCGGAGGAGCGGCGCATCGGCATCGTTGCCTGCGGCTACGCCGATGGCTACCCGCGTCTGGCGCCAAGCGGTACGCCGGTGCTGGTTGACGGCGTTCGCACCGCGACGGTAGGGCGCATCTCGATGGATATGCTGGCAGTGGATTTGACTCCCTGCCCGCAGGCAGGGATCGGTGCCCCGGTCGAGCTGTGGGGCAAAGAGATTAAAATCGACGATGTCGCATCCGCCTGCGGCACCGTCGGGTATGAGTTAATGTGCGCGCTGGCGCCGCGCGTACCGGTTGTGACGGTGTAA-3' |
| SEQ ID No.32 | *Klebsiella* dadX *protein* | MSRPVKASIDMSALRQNQHIVRRAAPGSRLWAVVKANAYGHGLARVWNALSAADGFAMLNLEEAILLRELGWKGPILMLEGFFHADELALFDKYRLTTSVHSNWQIKALQQAKLHAPLDIYVKVNSGMNRLGFMPDRLHVVWQQLRALHNVSEMTLMSHFAEAENPDGIVEPMRRIEQAAEGLDCPRSLANSAATLWHPESHFNWVRPGIVLYGASPSGLWQDVANTGLKPVMTLSSEIIAVQNLKAGEAVGYGATWRTAEERRIGIVACGYADGYPRLAPSGTPVLVDGVRTATVGRISMDMLAVDLTPCPQAGIGAPVELWGKEIKIDDVASACGTVGYELMCALAPRVPVVTV |
| SEQ ID No.33 | *Yersinia alr* gene | 5'-ATGAAAGCGGCAACAGCAGTAATCGACCGCCGCGCTCTGCGACATAATTTGCAACAGGTACGGCGCATGGCGCCGCAAAGTCGCCTGATTGCTGTTGTGAAAGCAAACGCTTATGGCCATGGGTTATTAGAGATAGCACATACTTTGCAGGATGCAGATTGCTACGGTGTAGCGCGGATCGGTGAAGCACTGATGCTCCGATCTGGTGGGATCGTGAAACCGATTTTGTTGTTGGAAGGGTTCTTTGCAGCAGAAGATCTGCCGGTTCTGGTGGCCAACCGCATTGAAACAGCAGTACATAGTATTGAGCAGTTGGAAGCATTGGAAGCTGCCGAGCTTTCTGCGCCAATCAATGTCTGGATGAAACTCGATACCGGTATGCACCGTCTGGGCGTTCGCCCGGAACAAGCTGAGGCTTTTTACCAGCGCTTAAGTGCGTGTAACAATGTTATTCAGCCAGTCAATATCATGAGCCATTTTAGCCGTGCTGATGAGCCACAAGTGGATACCACCCGCCAGCAACTGGACTGTTTTGATACTTTCGCCGCCGATAAGCCTGGCAAGCAATCTATTGCGGCATCTGGTGGCATATTACTGTGGCCAGAAGCGCACCGTGATTGGGTTCGTCCGGGAATCATCTTGTACGGTGTTTCGCCGATGGATGAGCCTTATGCCAGTCATTTTGGTCTATTACCTGCAATGGCATTGAAATCCAGTCTGATTGCTGTGCGTGAGCACAAAGCCGGTGAGCCTGTGGGTTATGGTGGCACTTGGGTGAGTGAGCGTGATACCCGTCTGGGTGTGGTGGCGATCGGTTATGGAGACGGTTATCCGCGTAGCGCTCCTTCAGGCACCCCGATGTGGTTGAATGGCCGCGAAGTTGGCATTGTGGGCCGGGTTTCAATGGATATGATTTCTGTTGATCTCGGGCCAAATGCAAAAGATAAAGTGGGTGACGAAGTATTGCTGTGGGGGGCTGAACTGCCGGTTGAAAAAATTGCAGCGTGCACAGGTATCAGCGCCTACGAATTGATTACAAAGCTGACCTCCCGCGTCGCGATGGAATATTTAGGTGAATAA-3' |
| SEQ ID No.34 | *Yersinia* alr protein | MKAATAVIDRRALRHNLQQVRRMAPQSRLIAVVKANAYGHGLLEIAHTLQDADCYGVARIGEALMLRSGGIVKPILLLEGFFAAEDLPVLVANRIETAVHSIEQLEALEAAELSAPINVWMKLDTGMHRLGVRPEQAEAFYQRLSACNNVIQPVNIMSHFSRADEPQVDTTRQQLDCFDTFAADKPGKQSIAASGGILLWPEAHRDWVRPGIILYGVSPMDEPYASHFGLLPAMALKSSLIAVREHKAGEPVGYGGTWVSERDTRLGVVAIGYGDGYPRSAPSGTPMWLNGREVGIVGRVSMDMISVDLGPNAKDKVGDEVLLWGAELPVEKIAACTGISAYELITKLTSRVAMEYLGE |

(continued)

| Sequence number | Description | Specific sequence |
|---|---|---|
| SEQ ID No.35 | *Yersinia dadX* gene | 5'-<br>ATGCCTCGCCCGATATCTGCCACGCTGAATTTATCTGCGCTGCACCATAATCTGA<br>ATGTTGCGCGCAGTCATGTGGGAACAGCAAAAATTTGGTCAGTGGTGAAAGCCA<br>ATGCCTACGGTCATGGTTTAGCGCGTGTCTGGCAAGGGCTGGCGGCGACGGATG<br>GATTTGCTTTACTGGATTTAAACGAAGCTATCCTGCTGCGAGAACAGGGCTGGCA<br>AGGGCCAATTTTATTGCTGGAGGGTTTCTTTCAAGCGCAGGATTTGGTGCTGCTG<br>GATCATTATCGCTTGACGACGGCGGTGCACAGTGACTGGCAACTTGAGGCTATCA<br>AAGCGGCCCGCCTGACGGCTCCACTCAATATTTACTTGAAGTTTAATAGCGGTAT<br>GAATCGTTTAGGGTTTCCAATTGGTCAGGCGGAAGCTGTGTGGCAATGGGCCAAC<br>AGCCTCAGCAATGTCGGCGAAATCACATTAATGAGCCACTTTGCTAATGCAGATA<br>ATCCTATTGGTGCTGATGAGCAATTTGCTCAGATCCAGCAGGCCAGTGGGCATAT<br>TCCGGCGGCCCGCTGTTTTGCCAATTCAGCGGCGATTTTGCGCAACCCACAAACT<br>CATTATGACTGGGTGCGCCCAGGGATTATTCTCTACGGCGCATCCCCCAGTGGTG<br>ATATTGCAGATATTGCCGGTCTTGGTTTGCGCCCGGTGATGAGTTTGCACAGTGA<br>GATTATCGCGCTGCAATCATTATCCGCCGGTCAAAGTGTGGGTTATGGTAGCCGT<br>TATCGCGCCACCGGTGCGCAGCGCATAGGAGTGGTCGCTTGTGGTTATGCCGATG<br>GTTATCCGCGTGTCGCACCAACGGGAACACCTATCATGGTGGATGGTATATTGAC<br>GCACACAATGGGGGCGATTTCGATGGATATGCTCACCGTGGATCTCACGCCATGT<br>CCGCAAGCGCAGATGGGCAGCAAAGTCGAAATATGGGGTGAGAATGTGCCCATT<br>GACAATGTGGCACAGGCCGCGGGAACGGTGGGGTATGAGTTGATGTGTGCGCTG<br>GCAGCCAGGGTTACCCTTCTGACTTGA-3' |
| SEQ ID No.36 | *Yersinia* dadX protein | MPRPISATLNLSALHHNLNVARSHVGTAKIWSVVKANAYGHGLARVWQGLAATDG<br>FALLDLNEAILLREQGWQGPILLLEGFFQAQDLVLLDHYRLTTAVHSDWQLEAIKAA<br>RLTAPLNIYLKFNSGMNRLGFPIGQAEAVWQWANSLSNVGEITLMSHFANADNPIGA<br>DEQFAQIQQASGHIPAARCFANSAAILRNPQTHYDWVRPGIILYGASPSGDIADIAGLG<br>LRPVMSLHSEIIALQSLSAGQSVGYGSRYRATGAQRIGVVACGYADGYPRVAPTGTPI<br>MVDGILTHTMGAISMDMLTVDLTPCPQAQMGSKVEIWGENVPIDNVAQAAGTVGY<br>ELMCALAARVTLLT |
| SEQ ID No.37 | *Haemophilus alr* gene | 5'-<br>ATGAACGTAAAACCGGCGACAGCGAAAATTAGTTCGCACGCCTTAAAACAGAAT<br>TTAGAAATAATTAAACAAAAAGCACCAAATAGCAAAATTATTGCTGTGGTTAAA<br>GCAAACGCCTATGGTCACGGCGTTGTATTCGTTGCTTCAACCTTAGAACAAAATG<br>TTGATTGCTTTGGCGTGGCGCGTTTAGAAGAGGCTTTAGCATTACGCTCCAACGG<br>CATTACTAAACCGATTTTATTGCTTGAAGGTTTTTTCAATGAACAAGATTTGCCTA<br>TTCTAGCCGTTAATAATATTGAAACCGTGGTACACAATCACGAACAGCTTGATGC<br>TTTAAAACGTGCGAATTTACCAAGTCCAATTAAAGTTTGGTTAAAAATAGATACG<br>GGAATGCATCGCTTGGGCGTTGCTCTTGATGAAGTGGATTATTTTATCAAGAAC<br>TGAAAAAACTCCCTCAAATTCAACCGCACTTAGGCTTTGTCAGCCATTTCAGCCG<br>AGCCGATGAACTAGAATCAGATTACACTCAGCTCCAAATCAATCGTTTTTTATCC<br>GCCACAAAAGATAAACAAGGTGAACGCACTATCGCAGCTTCTGGCGGCATTCTTT<br>TCTGGCCTGAATCTCATCTGGAATGTATCCGCCCAGGCATTATTATGTACGGCATT<br>TCTCCAACTGATACTATCGGTAAAGAGTTTGGCTTAACGCCAGTGATGAATTTAA<br>CCTCGTCATTAATTGCCGTTCGCCATCATAAACAAGGCGATCCTGTAGGTTACGG<br>CGGTATTTGGACAAGTCCACGAGATACTAAAATTGGCGTGGTCGCAATGGGTTAT<br>GGCGATGGTTATCCGCGCGATGTGCCAGAAGGTACACCTGTTTATTTAAATGGCC<br>GTCTTGTACCAATTGTTGGACGTGTGTCAATGGATATGCTTACTGTTGATTTAGGC<br>GCAGATAGCCAAGATTTGGTAGGCGATGAAGTAATTTTATGGGGCAAGGAATTA<br>CCTATTGAAACCGTAGCTAAATTCACAGGCATTTTAAGCTACGAGCTAATTACAA<br>AATTAACACCTCGTGTTATAACTGAATACGTTGATTAA-3' |
| SEQ ID No.38 | *Haemophilus* alr protein | MNVKPATAKISSHALKQNLEIIKQKAPNSKIIAVVKANAYGHGVVFVASTLEQNVDC<br>FGVARLEEALALRSNGITKPILLLEGFFNEQDLPILAVNNIETVVHNHEQLDALKRAN<br>LPSPIKVWLKIDTGMHRLGVALDEVDYFYQELKKLPQIQPHLGFVSHFSRADELESD<br>YTQLQINRFLSATKDKQGERTIAASGGILFWPESHLECIRPGIIMYGISPTDTIGKEFGL<br>TPVMNLTSSLIAVRHHKQGDPVGYGGIWTSPRDTKIGVVAMGYGDGYPRDVPEGTP<br>VYLNGRLVPIVGRVSMDMLTVDLGADSQDLVGDEVILWGKELPIETVAKFTGILSYE<br>LITKLTPRVITEYVD |

61

(continued)

| Sequence number | Description | Specific sequence |
|---|---|---|
| SEQ ID No.39 | *Pseudomonas alr* gene | 5'-ATGCGTCCCCTCGTTGCCACCGTCGACCTTTCCGCCATTCGCCACAACTATGCCCTGGCCAAGCGCTGCGCGCCGCAGCGCCAGGCGTTCGCGGTGGTCAAGGCGAACGCCTATGGCCACGGTGCGCGCGAAGTGGTTACCGCGTTGCACGACGATGCCGATGGCTTCGCCGTGGCCTGCCTGGAGGAGGCCGCGGAAGTCCGCGCCTTGCACGCCAGCGCGCGCATCCTGTTGCTGGAAGGCTGCTTCGAGGCGAGCGAATACGCCCTCGCCGGGCAATTGCGCCTGGACCTGGTGATCCAGGGCGCGGAACAGGGCGAGGCGTTCCTCGCCGCCGGCCTGGATATTCCACTCAACGTCTGGCTCAAGCTCGACTCCGGCATGCACCGCCTGGGCTTCGATCCTGCCGCGCTGCGCGCCTGGCATGCGCGCCTGCGCAGCCATCCCGGTGTGCGCGAGCTGAACCTGATCAGCCATTTCGCCTGCGCCGACGAACGCAACCATCCGCTCACCGAACAGCAGCTGGAGAGTTTCCTCGGCCTGCTCGACCTGGACTTCGACCAGCGCAGCCTGGCCAATTCGGCGGCGGTGCTGACTATCCCGGCCGCCCACATGGACTGGCTGCGGCCGGGAATCATGCTCTACGGGTCGACCCCGCTGGCCGACCTCAGTGCTGCCGAACTGGGCCTGAAGCCGGCCATGAGCCTGGGCGCGCAGTTGATTTCACTGCGCGAGGTGGCCGTGGGTGAGAGCGTCGGCTACGGCGCCACCTGGATCGCCGAGCGGCCGGCGCGGATCGGCACGGTCAGTTGCGGCTACGCCGACGGCTATCCGCGTACCGCGCCAGCCGGGACTCCGGTACTGGTCGGGGGGACGCCGCGCGATCCTGGCCGGGCGGGTCTCGATGGACATGCTGGCGGTCGACCTCTCCGACCTGCCGGAGGCTCGCGTTGGCGACCCGGTGGAGTTGTGGGGCGCCGGGTTGTCGGTGGACGAGGTCGCCCGTGCCTGCGGCACCCTTGGCTACGAGCTTCTGAGCAAGGTCACGGCTCGGGTTCCGCGACGCTACAGCCACTGA-3' |
| SEQ ID No.40 | *Pseudomonas* alr protein | MRPLVATVDLSAIRHNYALAKRCAPQRQAFAVVKANAYGHGAREVVTALHDDADGFAVACLEEAAEVRALHASARILLLEGCFEASEYALAGQLRLDLVIQGAEQGEAFLAAGLDIPLNVWLKLDSGMHRLGFDPAALRAWHARLRSHPGVRELNLISHFACADERNHPLTEQQLESFLGLLDLDFDQRSLANSAAVLTIPAAHMDWLRPGIMLYGSTPLADLSAAELGLKPAMSLGAQLISLREVAVGESVGYGATWIAERPARIGTVSCGYADGYPRTAPAGTPVLVGGRRAILAGRVSMDMLAVDLSDLPEARVGDPVELWGAGLSVDEVARACGTLGYELLSKVTARVPRRYSH |
| SEQ ID No.41 | *Pseudomonas dadX* gene | 5'-ATGCGCCCCGCCCGTGCCCTGATCGACCTGCAAGCCCTTCGTCACAACTATCGGCTGGCCCGCGAGGCCACCGGCGCCCGTGCGCTCGCGGTGATCAAGGCGGACGCATACGGCCACGGCGCGGTGCGCTGTGCCGAAGCGCTGGCGGCCGAGGCCGATGGCTTCGCCGTGGCCTGCATCGAGGAGGGCCTGGAGCTGCGCGAGGCCGGTATCCGCCAGCCGATCCTGCTGCTGGAGGGCTTCTTCGAGGCGTCCGAGCTGGAGCTGATCGTCGCCCACGACTTCTGGTGCGTGGTGCATTGCGCCTGGCAATTGGAGGCGATCGAACGCGCCAGCCTGGCCCGCCCGCTGAACGTCTGGCTGAAGATGGATTCGGGCATGCACCGCGTCGGCTTCTTCCCCGAGGACTTCAGCGCCGCCCACGAGCGCCTGCGGGCCAGCGGCAAGGTGGCGAAGATCGTGATGATGAGTCACTTCTCCCGCGCCGACGAACTGGATTGCCCGCGCACCGAGGAACAGCTCGCCGCCTTCGCGGCCGCGAGCCAGGGCCTGGAAGGCGAGATCAGCCTGCGCAATTCGCCAGCCGTGCTCGGCTGGCCGAAGGTGCCCAGCGATTGGGTACGTCCGGGCATCCTGCTCTACGGAGCCACGCCGTTCGAGCGCGCACATCCGCTGGCCGACCGCTTGCGCCCGGTGATGACCCTAGAA

TCGAAGGTGATCAGCGTCCGCGACCTGCCGGCCGGCGAACCGGTCGGCTACGGCGCGCGCTACAGCACCGAGCGTAGCCAGCGCATCGGCGTGGTCGCCATGGGCTACGCCGATGGCTATCCGCGCCACGCCGCCGACGGCACCCTTGTGTTCATCGACGGCAAGCCGGGGCGCCTGGTTGGCCGGGTATCGATGGACATGCTCACCGTCGACCTTACCGACCATCCCCAGGCCGGGCTGGGCAGCCGGGTCGAACTATGGGGCCCGAACGTGCCGGTCGGCGCCCTGGCGGCGCAGTTCGGCAGCATTCCCTACCAGCTGCTGTGCAACCTGAAAAGGGTGCCGCGCGTCTATTCCGGGGCTTGA-3' |
| SEQ ID No.42 | *Pseudomonas dadX* protein | MRPARALIDLQALRHNYRLAREATGARALAVIKADAYGHGAVRCAEALAAEADGFAVACIEEGLELREAGIRQPILLLEGFFEASELELIVAHDFWCVVHCAWQLEAIERASLARPLNVWLKMDSGMHRVGFFPEDFSAAHERLRASGKVAKIVMMSHFSRADELDCPRTEEQLAAFAAASQGLEGEISLRNSPAVLGWPKVPSDWVRPGILLYGATPFERAHPLADRLRPVMTLESKVISVRDLPAGEPVGYGARYSTERSQRIGVVAMGYADGYPRHAADGTLVFIDGKPGRLVGRVSMDMLTVDLTDHPQAGLGSRVELWGPNVPVGALAAQFGSIPYQLLCNLKRVPRVYSGA |

(continued)

| Sequence number | Description | Specific sequence |
|---|---|---|
| SEQ ID No.43 | Sequence in pSWT002 containing bilateral co-directional *loxP* sequences and an intervening chloramphenicol resistance gene, flanked by NotI sites | 5'-GCGGCCGCGACTAGTGAACCTCTTCGAGGGACCTAATAACTTCGTATAGCATACATTATACGAAGTTATATTAAGGGTTCCGGATCCTAAGCTTGATATCTAAGGGCACCAATAACTGCCTTAAAAAAATTACGCCCCGCCCTGCCACTCATCGCAGTACTGTTGTAATTCATTAAGCATTCTGCCGACATGGAAGCCATCACAGACGGCATGATGAACCTGAATCGCCAGCGGCATCAGCACCTTGTCGCCTTGCGTATAATATTTGCCCATGGTGAAAACGGGGGCGAAGAAGTTGTCCATATTGGCCACGTTTAAATCAAAACTGGTGAAACTCACCCAGGGATTGGCTGAGACGAAAAACATATTCTCAATAAACCCTTTAGGGAAATAGGCCAGGTTTTCACCGTAACACGCCACATCTTGCGAATATATGTGTAGAAACTGCCGGAAATCGTCGTGGTATTCACTCCAGAGCGATGAAAACGTTTCAGTTTGCTCATGGAAAACGGTGTAACAAGGGTGAACACTATCCCATATCACCAGCTCACCGTCTTTCATTGCCATACGGAATTCCGGATGAGCATTCATCAGGCGGGCAAGAATGTGAATAAAGGCCGGATAAAACTTGTGCTTATTTTTCTTTACGGTCTTTAAAAAGGCCGTAATATCCAGCTGAACGGTCTGGTTATAGGTACATTGAGCAACTGACTGAAATGCCTCAAAATGTTCTTTACGATGCCATTGGGATATATCAACGGTGGTATATCCAGTGATTTTTTTCTCCATTTTAGCTTCCTTAGCTCCTGAAAATCTCGATAACTCAAAAAATACGCCCGGTAGTGATCTTATTTCATTATGGTGAAAGTTGGAACCTCTTACGTGCCGATCAACGTCTCATTTTCGCCAAAAGTTGGCCCAGGGCTTCCCGGTATCAACAGGGACACCAGGATTTATTTATTCTGCGAAGTGATCTTCCGTCACAGGTATTTATTCGGCGCAAAGTGCGTCGGGTGATGCTGCCGATATCGGTACCCGGGATCCTAAGCTTGGCTGGACGTAAACTCCTCTTCAGACCTAATAACTTCGTATAGCATACATTATACGAAGTTATATTAAGGGTTATTGAATATGATCGGGCGGCCGC-3' |
| SEQ ID No.44 | *pepT* promoter | 5'-GTAAACGCAACGGATGGCTGACCGCTGCGGGGTTTGTGGTTAACCACCTTAATCACTCTTAATGAGGGCGGTCATTCTACGGCAAACCACCGTGATCGCCAATCCTTGTTGCGAATTACTGACTTAGCTTTATAGTCAGAAAGCGTGTCAAAGTGAAATATTCTTGTTTGCAGGGATAAAAGTGACCTGACGCAATATTTGTCTTTTCTTGCTTATTAATAATGTTGTCACGAAAAG-3' |
| SEQ ID No.45 | *asd* gene | 5'-ATGAAAAATGTTGGTTTTATCGGCTGGCGCGGAATGGTCGGCTCTGTTCTCATGCAACGCATGGTAGAGGAGCGCGATTTCGACGCTATTCGCCCTGTTTTCTTTTCTACCTCCCAGTTTGGACAGGCGGCGCCCACCTTCGGCGACACCTCCACCGGCACGCTACAGGACGCTTTTGATCTGGATGCGCTAAAAGCGCTCGATATCATCGTGACCTGCCAGGGCGGCGATTATACCAACGAAATTTATCCAAAGCTGCGCGAAAGCGGATGGCAGGGTTACTGGATTGATGCGGCTTCTACGCTGCGCATGAAAGATGATGCCATTATTATTCTCGACCCGGTCAACCAGGACGTGATTACCGACGGCCTGAACAATGGCGTGAAGACCTTTGTGGGCGGTAACTGTACCGTTAGCCTGATGTTGATGTCGCTGGGCGGTCTCTTTGCCCATAATCTCGTTGACTGGGTATCCGTCGCGACCTATCAGGCCGCCTCCGGCGGCGGCGCGCGCCATATGCGCGAGCTGTTAACCCAGATGGGTCAGTTGTATGGCCATGTCGCCGATGAACTGGCGACGCCGTCTTCCGCAATTCTTGATATTGAACGCAAAGTTACGGCATTGACCCGCAGCGGCGAGCTGCCGGTTGATAACTTTGGCGTACCGCTGGCGGGAAGCCTGATCCCCTGGATCGACAAACAGCTCGATAACGGCCAGAGCCGCGAAGAGTGGAAAGGCCAGGCGGAAACCAACAAGATTCTCAATACTGCCTCTGTGATTCCGGTTGATGGTTTGTGTGTGCGCGTCGGCGCGCTGCGCTGTCACAGCCAGGCGTTCACCATCAAGCTGAAAAAAGAGGTATCCATTCCGACGGTGGAAGAACTGCTGGCGGCACATAATCCGTGGGCGAAAGTGGTGCCGAACGATCGTGATATCACTATGCGCGAATTAACCCCGGCGGCGGTGACCGGCACGTTGACTACGCCGGTTGGTCGTCTGCGTAAGCTGAACATGGGGCCAGAGTTCTTGTCGGCGTTTACCGTAGGCGACCAGTTGTTATGGGGCGCCGCCGAGCCGCTGCGTCGAATGCTGCGCCAGTTGGCGTAG-3' |
| SEQ ID No.46 | asd protein | MKNVGFIGWRGMVGSVLMQRMVEERDFDAIRPVFFSTSQFGQAAPTFGDTSTGTLQDAFDLDALKALDIIVTCQGGDYTNEIYPKLRESGWQGYWIDAASTLRMKDDAIIILDPVNQDVITDGLNNGVKTFVGGNCTVSLMLMSLGGLFAHNLVDWVSVATYQAASGGGARHMRELLTQMGQLYGHVADELATPSSAILDIERKVTALTRSGELPVDNFGVPLAGSLIPWIDKQLDNGQSREEWKGQAETNKILNTASVIPVDGLCVRVGALRCHSQAFTIKLKKEVSIPTVEELLAAHNPWAKVVPNDRDITMRELTPAAVTGTLTTPVGRLRKLNMGPEFLSAFTVGDQLLWGAAEPLRRMLRQLA |

(continued)

| Sequence number | Description | Specific sequence |
|---|---|---|
| SEQ ID No.47 | *sodA* promoter | 5'-ACGAAAAGTACGGCATTGATAATCATTTTCAATATCATTTAATTAACTATAATGAACCAA-3' |
| SEQ ID No.48 | *Escherichia coli yhbU* promoter | 5'-CACAATACGGGAACGCAGTTTATCCAACACGAGTTTTACTCCCTGTTTCAACAATCATCCTATTTTGCCATATCAGAAAAATAACATAGCGGTATAAATCAACAATTCCATATGAAATTGCTGCTACCACCAATACAACTTTAACTGCCTTAAATCAAAAATTGTCGCAGCAAGGTTAACTAAAATCCCAGTTCGTTAACATTTTTGCGTTTTGATAGCGCAACCTTCAGGAAAAATT-3' |
| SEQ ID No.49 | *Shigella yhbU* promoter | 5'-GAGTTTTACTCCCTGTTTCAACAATCATCCTATTTTGCCATATCAGAAAAATAACATAGCGGTATAAATCAACAATTCCATATGAAATTGCTGCTACCACCAATACAACTTTAACTGCCTTAAATCAAAAATTGTCGCAGCAAGGTTAACTAAAATCCCAGTTCGTTAACATTTTTGCGTTTTGATAGCGCAACCTTCAGGAAAAATT-3' |
| SEQ ID No.50 | *Yersinia yhbU* promoter | 5'-AGGCGGTGACTCCTTGTAATCCTGAATAAAATGATAGCACTGAATAAAATGACAATGCCAAGTACCGCTATTCTGCCACATTGCTAACCTATGAAAATGGGCTATATCGACAAATTTTGCATCAGAACATCTTCAGATGTATTGCTAAATATCAGAAGAGTGTCACCAATGCGAAAGAAACTGCCTTAAATCAAGGTGCGATGTGAAGAGGTTAACTAAAATTGCCGCTCGATAAAATACCAAGATCATCTATGCCCTCACATCTGCAACTTGAATCTGCAACTTGAAGTATGGGGGGTATATCAGGGAAATTCT-3' |
| SEQ ID No.51 | *Enterobacter cloacae yhbU* promoter | 5'-GAGCTTTACTCCCTGATAGCTATAGTCCTGCTATTTTGCCATATCCGAAGCACAACATAGCGGCGTAAATCAACAAACTGTCGTCAGTTGTACCTCATTATTGGTGCCATCAATACGCCATTAGCTGCCTTAAATCAAAAATTGTCGCGTATGGGTTAATTAAAATCCCAGCTCGTTAACAAATTTGCGATCGACTACATCAGGATAAATTATGGAGCTGCTCTGCCCAGCCGGAAATCTTCCGGCGCTTAAGGCGGCCATCGAAAATGGGGCCGATGCGGTCTATATCGGGCTGAAAGATGATACCAACGCCCGCCATTTTGCTGGCCTCAACTTTACGGAGAAAAAGCTTCAGGAAGCCGTTACCTTCGTCCACCAGCACCGCCGCAAACTGCATATCGCCATCAATACCTTCGCCCATCCTGATGGCTACGCCCGCTGGCAACGCGCG-3' |
| SEQ ID No.52 | *Cronobacter yhbU* promoter | 5'-GGGCTTTTCTCCTTGCCAGATCACAATGGCCGTTATTGTGCCATATCGGCCAGGCGAGATAGCGGCTCAAATCAAGCTTTGCGCCTTCGCAGGTACCCCTTTATAGATGCCATCAATACGACATTAGCTGCCTTAAATCAAAAATTGTCGCGGACGGGTTAACTAAAATCGCCGTTCGTTAACAATTTCGCTGCCTTTATGCAGCGCGCCAGGCCGCATACGGCCTGCTTTTATTTTCAGGACACGCT-3' |
| SEQ ID No.53 | *Klebsiella yhbU* promoter | 5'-AAGCTTTACTCCATGAATGCAATAGTTCCGCTATTGTGCCATATCACATAAACAACATAGCGGCGTAAATCAACAAAAACGCGATGACGGCCGCCAGTTATTGGCGCCGCCAATACACTTTTAACTGCCTCAAATCAAAAATTGTCACCAGAAGGTTAACTAAAATCCCTGCTCGTTAACCATTCAGCGTCCCTGACGGGCCGCATTTTTCAGGAAAAATT-3' |
| SEQ ID No.54 | *Pantoea yhbU* promoter | 5'-TCCCGCCTCCTTTGAGATCAATAAAACCTATCGTAAGCGGGTTAAAAAAAAGCCAGGCTATCTCATCAATGACCGTTTCACTGCCCTAAATCAACATTTACCCAAAGCGCCATGACTAGACTCGTGCTCCATCTGCATCACGAGAGGATTGAGT-3' |
| SEQ ID No.55 | *Serratia yhbU* promoter | 5'-TCCCGCCTCCTTTGAGATCAATAAAACCTATCGTAAGCGGGTTAAAAAAAAGCCAGGCTATCTCATCAATGACCGTTTCACTGCCCTAAATCAACATTTACCCAAAGCGCCATGACTAGACTCGTGCTCCATCTGCATCACGAGAGGATTGAGT-3' |

(continued)

| Sequence number | Description | Specific sequence |
|---|---|---|
| SEQ ID No.56 | *Shimwellia yhbU* promoter | 5'-GTACTTTACTCCCTGAAATAGATAATCCTCGATATTGTGCCACAGACATGGTGCCGCCAGCTGGCTCAAAATCAACAATTGGCGAACTTAACGGCACGGGGTCATCCTGTGCTTTTCAGAGTACCAATACGCCATTAGCTGCCCCAAATCAAAAATTGCCATCAACTGGTTAATTAAAATCGCGGTTCGTTAACAATTACGCTGGCCTGCCAGGTCATGGTACAGCACGGGCGCCTGCCCGGATAACCAGGAAAAATT-3' |
| SEQ ID No.57 | *Serratia yhfK* promoter | 5'-CATCAGAACCTCTTATTAATTCCGCAAAAGATTGAGCTACCTCAAGCCCCTACGCGGCAGCATCCCCTAGAATTTTTCCGAAAGACTTCTTCGCCGCCGGTGCGGCGATACATTGTTAACGGGGCATTCTGGGGCATTTTATTC-3' |
| SEQ ID No.58 | *Shigella ynfK* promoter | 5'-CATTTTTTAACTGTTCTACCAAAATTTGCGCTATCTCAATTTGGGCCAGGAAAGCATAACTTAGACTTTCAAGGTTAATTATTTTCCTAGTTTATATTTGTGAAGCATAACGGTGGAGTTAGTG-3' |
| SEQ ID No.59 | *Enterobacter ludwigii ynfK* promoter | 5'-CTCTTTTCCGCAGATGTACGAAAAATTGCGCTATCTCAAGCTGGGTAGCGCACGCATCCCGTAGACTTCCCCCACTGAATTTTTTCTCTGGTTTATATTTTCGAAGCATACCCAAGAGGTGGAATGAGTC-3' |

(continued)

| Sequence number | Description | Specific sequence |
|---|---|---|
| SEQ ID No.60 | Sequence in pSWT001 containing Lambda-RED recombinase and Cre enzyme | 5'-GAATTCTCAGCCAAACGTCTCTTCAGGCCACTGACTAGCGATAACTTTCCCCACAACGGAACAACTCTCATTGCATGGGATCATTGGGTACTGTGGGTTTAGTGGTTGTAAAAACACCTGACCGCTATCCCTGATCAGTTTCTTGAAGGTAAACTCATCACCCCCAAGTCTGGCTATGCAGAAATCACCTGGCTCAACAGCCTGCTCAGGGTCAACGAGAATTAACATTCCGTCAGGAAAGCTTGGCTTGGAGCCTGTTGGTGCGGTCATGGAATTACCTTCAACCTCAAGCCAGAATGCAGAATCACTGGCTTTTTTTGGTTGTGCTTACCCATCTCTCCGCATCACCTTTGGTAAAGGTTCTAAGCTTAGGTGAGAACATCCCTGCCTGAACATGAGAAAAAACAGGGTACTCATACTCACTTCTAAGTGACGGCTGCATACTAACCGCTTCATACATCTCGTAGATTTCTCTGGCGATTGAAGGGCTAAATTCTTCAACGCTAACTTTGAGAATTTTTGTAAGCAATGCGGCGTTATAAGCATTTAATGCATTGATGCCATTAAATAAAGCACCAACGCCTGACTGCCCCATCCCCATCTTGTCTGCGACAGATTCCTGGGATAAGCCAAGTTCATTTTTCTTTTTTTCATAAATTGCTTTAAGGCGACGTGCGTCCTCAAGCTGCTCTTGTGTTAATGGTTTCTTTTTTGTGCTCATACGTTAAATCTATCACCGCCAAGGGATAAATATCTAACACCGTGCGTGTTGACTATTTTACCTCTGGCGGTGATAATGGTTGCATGTACTAAGGAGGTTGCGCATGCAGGTACCAATGTCCAATTTACTGACCGTACACCAAAATTTGCCTGCATTACCGGTCGATGCAACGAGTGATGAGGTTCGCAAGAACCTGATGGACATGTTCAGGGATCGCCAGGCGTTTTCTGAGCATACCTGGAAAATGCTTCTGTCCGTTTGCCGGTCGTGGGCGGCATGGTGCAAGTTGAATAACCGGAAATGGTTTCCCGCAGAACCTGAAGATGTTCGCGATTATCTTCTATATCTTCAGGCGCGCGGTCTGGCAGTAAAAACTATCCAGCAACATTTGGGCCAGCTAAACATGCTTCATCGTCGGTCCGGGCTGCCACGACCAAGTGACAGCAATGCTGTTTCACTGGTTATGCGGCGGATCCGAAAAGAAAACGTTGATGCCGGTGAACGTGCAAAACAGGCTCTAGCGTTCGAACGCACTGATTTCGACCAGGTTCGTTCACTCATGGAAAATAGCGATCGCTGCCAGGATATACGTAATCTGGCATTTCTGGGGATTGCTTATAACACCCTGTTTACGTATAGCCGAAATTGCCAGGATCAGGGTTAAAGATATCTCACGTACTGACGGTGGGAGAATGTTAATCCATATTGGCAGAACGAAAACGCTGGTTAGCACCGCAGGTGTAGAGAAGGCACTTAGCCTGGGGGTAACTAAACTGGTCGAGCGATGGATTTCCGTCTCTGGTGTAGCTGATGATCCGAATAACTACCTGTTTTGCCGGGTCAGAAAAAATGGTGTTGCCGCGCCATCTGCCACCAGCCAGCTATCAACTCGCGCCCTGGAAGGGATTTTTGAAGCAACTCATCGATTGATTTACGGCGCTAAGGATGACTCTGGTCAGAGATACCTGGCCTGGTCTGGACACAGTGCCCGTGTCGGAGCCGCGCGAGATATGGCCCGCGCTGGAGTTTCAATACCGGAGATCATGCAAGCTGGTGGCTGGACCAATGTAAATATTGTCATGAACTATATCCGTAACCTGGATAGTGAAACAGGGGCAATGGTGCGCCTGCTGGAAGATGGCGATTAGGGGTACCGAGCTCGAATTCATGAACGCTTATTACATTCAGGATCGTCTTGAGGCTCAGAGCTGGGCGCGTCACTACCAGCAGCTCGCCCGTGAAGAGAAAGAGGCAGAACTGGCAGACGACATGGAAAAAGGCCTGCCCCAGCACCTGTTTGAATCGCTATGCATCGATCATTTGCAACGCCACGGGGCCAGCAAAAAATCCATTACCCGTGCGTTTGATGACGATGTTGAGTTTCAGGAGCGCATGGCAGAACACATCCGGTACATGGTTGAAACCATTGCTCACCACCAGGTTGATATTGATTCAGAGGTATAAAACGAATGAGTACTGCACTCGCAACGCTGGCTGGGAAGCTGGCTGAACGTGTCGGCATGGATTCTGTCGACCCACAGGAACTGATCACCACTCTTCGCCAGACGGCATTTAAAGGTGATGCCAGCGATGCGCAGTTCATCGCATTACTGATCGTTGCCAACCAGTACGGCCTTAATCCGTGGACGAAAGAAATTTACGCCTTTCCTGATAAGCAGAATGGCATCGTTCCGGTGGTGGGCGTTGATGGCTGGTCCCGCATCATCAATGAAAACCAGCAGTTTGATGGCATGGACTTTGAGCAGGACAATGAATCCTGTACATGCCGGATTTACCGCAAGGACCGTAATCATCCGATCTGCGTTACCGAATGGATGGATGAATGCCGCCGCGAACCATTCAAAAACTCGCGAAGGCAGAGAAATCACGGGGCCGTGGCAGTCGCATCCCAAACGGATGTTACGTCATAAAGCCATGATTCAGTGTGCCCGTCTGGCCTTCGGATTTGCTGGTATCTATGACAAGGATGAAGCCGAGCGCATTGTCGAAAATACTGCATACACTGCAGAACGTCAGCCGGAACGCGACATCACTCCGGTTAACGATGAAACCATGCAGGAGATTAACACTCTGCTGATCGCCCTGGATAAAACATGGGATGACGACTTATTGCCGCTCTGTTCCCAGATATTTCGCCGCGACATTCGTGCATCGTCAGAACTGACACAGGCCGAAGCAGTAAAAGCTCTTGGATTCCTGAAACAGAAAGCCGCAGAGCAGAAGGTGGCAGCATGACACCGGACATTATCCTGCAGCGTACCGGGATCGATGTGAGAGCTGTCGAACAGGGGGATGATGCGTGGCACAAATTACGGCTCGGCGTCATCACCGCTTCAGAAGTTCACAACGTGATAGCAAAACCCCGCTCCGGAAAGAAGTGGCCTGACATGAAAATGTCCTACTTCCACACCCTGCTTGCTGAGGTTTGCACCGGTGTGGCTCCGGAAGTTAACGCTAAAGCACTGGCCTGGGGAAAACAGTACGAGAACGACGCCAGAACCCTGTTTGAGTTCACTTCCGGCGTGAATGTTACTGAATCCCCGATCATCTATCGCGACGAAAGTATGCGTACCGCCTGCTCTCCCGATGGTTTATGCAGTGACGGCAACGGCCTTGAACTGAAATGCCCGTTTACCTCCCGGGATTTCATGAAGTTCCGGCTCGGTGGTTTCGAGGCCATAAAGTCAGCTTACATGGCCCAGGTGCAGTACAGCATGTGGGTGACGCGAAAAAATGCCTGGTACTTTGCCAACTATGACCCGCGTATGAAGCGTGAAGGCCTGCATTATGTCGTGATTGAGCGGGATGAAAAGTACATGGCGAGTTTTGA |

(continued)

| Sequence number | Description | Specific sequence |
|---|---|---|
| | | CGAGATCGTGCCGGAGTTCATCGAAAAAATGGACGAGGCACTGGCTGAAATTGG TTTTGTATTTGGGGAGCAATGGCGATGACTCGAG-3' |
| SEQ ID No.61 | Sequence in pSWT007 containing bilateral co-directional *loxP* sequences and an intervening chloramphenicol resistance gene, flanked by SpeI and NotI sites, respectively | 5'-ACTAGTGAACCTCTTCGAGGGACCTAATAACTTCGTATAGCATACATTATACGAA GTTATATTAAGGGGTTCCGGATCCTAATTGATATCTAAGGGCACCAATAACTGCCT TAAAAAAATTATGCACCACCTTGCCATTCATCACAAATACTGTTGCAGTTCATTCA GCATACGACCAACATGAAAACCATCACAAACTGCATGATGAACCTGAATTGCCA GCGGCATCAGAACTTTATCACCCTGGGTGTAATATTTGCCCATCGTAAAAACCGG TGCGAAAAAGTTATCCATATTGGCCACATTCAGATCAAATGAGGTAAAGCTAACC CACGGATTTGCGCTAACAAAGAACATGTTTTCGATAAAGCCTTTCGGAAAATATG CCAGGTTTTCACCATAACATGCAACATCCTGGCTATAGATGTGCAGGAACTGACG AAAATCATCATGATATTCTGACCACAGGCTGCTAAAGGTTTCGGTCTGTTCATGA AAAACGGTATAACACGGATGAACGCTATCCCAAATAACCAGTTCACCATCTTTCA TTGCCATACGAAATTCCGGATGGGCATTCATCAGACGTGCCAGAATATGAATAAA GGCAGGGTAGAACTTGTGTTTGTTCTTTTTCACGGTTTTCAGAAATGCGGTTATAT CCAGCTGAACGGTCTGATTATAGGTACACTGTGCAACGCTCTGAAATGCTTCAAA GTGTTCTTTACGATGCCACTGGCTAATATCAACGGTGGTATAACCGGTGATTTTCT TTTCCATTTTAGCTTCCTTAGCTCCTGAAAATCTCGATAACTCAAAAAATACGCCC GGTAGTGATCTTATTTCATTATGGTGAAAGTTGGAACCTCTTACGTGCCGATCAA CGTCTCATTTTCGCCAAAAGTTGGCCCAGGGCTTCCCGGTATCAACAGGGACACC AGGATTTATTTATTCTGCGAAGTGATCTTCCGTCACAGGTATTTATTCGGCGCAAA GTGCGTCGGGTGATGCTGCCGATATCGGACCCGGGATCCTAATTGGCTGGACGTA AACTCCTCTTCAGACCTAATAACTTCGTATAGCATACATTATACGAAGTTATATTA AGGGTTATTGAATATGATCGGGCGGCCGC-3' |
| SEQ ID No.62 | *Escherichia coli mqo* promoter | 5'-TGCCCGGACGGCATAATATTACGACGCTGACCTGGCGGACTGCTGCCGTCAGGTC AATATGCTAAAAATCCCTTCATTTCTAATAACCCTATAATTAATCAACGAAATTA TAATGTTTCTAAAATTAGAATATAATTTATAAACATTATTTAAATGTTGTTACTTA AGTGTTACCGTTGATGCCGCGCAAATCTCACCTGCAATAAAGCGACTAAA-3' |

The specific sequences of all primers used in the present invention are shown in Table 7.

Table 7 The specific sequences of all primers used in the present invention

| Sequence Number | Code | Sequence | Purpose |
|---|---|---|---|
| SEQ ID No.63 | SWTO1 | ATGGAATCCCTGACGTTACAACCCATCGCGC GGGTCGATGGCGCCATTAACCGATCATATTC AATAACCCT | Knockout of the *aroA* gene |
| SEQ ID No.64 | SWTO2 | GAAGACTTAGGCAGGCGTACTCATTCGCGCC AGTTGTTCGAAATAATCAGGACTAGTGAACC TCTTCGAGGG | |
| SEQ ID No.65 | SWTO3 | CGGACAATACGCTGGACAAG | *aroA* identification primer, forward |
| SEQ ID No.66 | SWTO4 | GCGGCTTTGTAGGTTGACAT | *aroA* identification primer, reverse |
| SEQ ID No.67 | SWTO5 | TCCTTAGCTCCTGAAAATCTCG | Chloramphenicol resistance gene detection primer, reverse |
| SEQ ID No.68 | SWTO6 | TGTGGCGTGTTACGGTGAAA | Chloramphenicol resistance gene detection primer, forward |
| SEQ ID No.69 | SWTO7 | AAACTGCAGTGCCCGGACGGCATAATATT | *Pst*I-*mqo*-E promoter, reverse |

(continued)

| Sequence Number | Code | Sequence | Purpose |
|---|---|---|---|
| SEQ ID No.70 | SWTO8 | CCGCTCGAGTTTAGTCGCTTTATTGCAGGTGAG | *Pst*I-*mqo*-E promoter, forward |
| SEQ ID No.71 | SWTO11 | ATTTGCGGCCGCGTAAACGCAACGGATGGCTGACCGC | *Not*I-*pepT* promoter, forward |
| SEQ ID No.72 | SWTO12 | CCCAAGCTTCTTTTCGTGACAACATTATTAATAAG | *Hind*III-*pepT* promoter, reverse |
| SEQ ID No.73 | SWTO13 | CCCAAGCTTTGGAGCGAAACCGATGAAAAATGTTGGTTTTATCGGCTGGC | *Hind*III-*asd*, forward |
| SEQ ID No.74 | SWTO14 | CCGCTCGAGCTACGCCAACTGGCGCAGCATTCGA | *Xho*I-*asd*, reverse |
| SEQ ID No.75 | SWTO15 | GACGAAAAGTACGGCATTGATAATCATTTTCAATATCATTTAATTAACTATAATGAACCAAC | *Xho*I-*sodA*, forward |
| SEQ ID No.76 | SWTO16 | TCGAGTTGGTTCATTATAGTTAATTAAATGATATTGAAAATGATTATCAATGCCGTACTTTTCGTCTGCA | *Pst*I-*sodA*, reverse |
| SEQ ID No.77 | SWTO17 | CGCATACACAGCACATCTCTTTGCAGGAAAAAAACGCTGAACCTCTTCGAGGGACCTAAT | Amplification of *cm-pepT-asd-sodA* and recombination into the chromosome |
| SEQ ID No.78 | SWTO18 | GAACCACACGCAGGCCCGATAAGCGCTGCAATAGCCAACGAAAAGTACGGCATTGATAATC | |
| SEQ ID No.79 | SWTO19 | ATTTGCGGCCGCGCGTGAACGCAGTTTATCTAACACG | *Not*I-*yhbU-S* promoter, forward |
| SEQ ID No.80 | SWTO20 | CCCAAGCTTTTATCCTGAGGGTTACGTCTGAGACG | *Hind*III-*yhbU*-S promoter, reverse |
| SEQ ID No.81 | SWTO21 | ATTTGCGGCCGCCGCTGCACTGGTAAAAGACG | *Not*I-*ynfK-E* promoter, forward |
| SEQ ID No.82 | SWTO22 | CCCAAGCTTAACTCCACCGTTATGCTTCAC | *Hind*III-*ynfK*-E promoter, reverse |
| SEQ ID No.83 | SWTO23 | ATTTGCGGCCGCCTCTTCATAAAACCCGTCAGTGTAAG | *Not*I-*tdcA*-S promoter, forward |
| SEQ ID No.84 | SWTO24 | CCCAAGCTTCTGAAATAACATTGTGAACGGCA | *Hind*III-*tdcA*-S promoter, reverse |
| SEQ ID No.85 | SWTO25 | ATTTGCGGCCGCTCATTAAATTTAACTCAAATTTTGC | *Not*I-*tdcA*-E *promoter,* forward |
| SEQ ID No.86 | SWTO26 | CCCAAGCTTGACCTACGTTAATTACCTCATTGACG | *Hind*III-*tdcA*-E promoter, reverse |
| SEQ ID No.87 | SWTO27 | ATTTGCGGCCGCCGTAACGCTAAAACCAACACC | *Not*I-*yecH-E* promoter, forward |
| SEQ ID No.88 | SWTO28 | CCCAAGCTTCCCTCCTCAAAAAAAGCCTAG | *Hind*III-*yecH*-E promoter, reverse |
| SEQ ID No.89 | SWTO29 | ATTTGCGGCCGCAGACGCGATGTATAACGGCTCT | *Not*I-*ynfK-S* promoter, forward |

(continued)

| Sequence Number | Code | Sequence | Purpose |
|---|---|---|---|
| SEQ ID No.90 | SWTO30 | CCCAAGCTTCACTAACTCCACCGTTATGCTTC | *Hind*III-*ynfK*-S promoter, reverse |
| SEQ ID No.91 | SWTO31 | ATTTGCGGCCGCGTTAATATTGGCGGGGTGGAT | *Not*I-*cydA*-E promoter, forward |
| SEQ ID No.92 | SWTO32 | CCCAAGCTTGACTCCTTGCTCATCGCATGA | *Hind*III-*cydA*-E promoter, reverse |
| SEQ ID No.93 | SWTO33 | ATTTGCGGCCGCAAAGCAGCATTCTGGGCAAA | *Not*I-*focA*-E promoter, forward |
| SEQ ID No.94 | SWTO34 | CCCAAGCTTAACTCTCTCTTTATTAAGTCGGCGA | *Hind*III-*focA*-E promoter, reverse |
| SEQ ID No.95 | SWTO35 | CCCAAGCTTTGGAGCGAAACCGATGCAAGCGGCAACAGTCGTC | *Hind*III-*Alr*-S gene, forward |
| SEQ ID No.96 | SWTO36 | CCGCTCGAGTTAATCAATATACTTCATCGCCACCCTTG | *Xho*I-*Alr*-S gene, reverse |
| SEQ ID No.97 | SWTO37 | CCCAAGCTTTGGAGCGAAACCGATGCAAGCGGCAACTGTTGT | *Hind*III-*Alr*-E gene, forward |
| SEQ ID No.98 | SWTO38 | CCGCTCGAGTTAATCCACGTATTTCATCGCG | *Xho*I-*Alr*-E gene, reverse |
| SEQ ID No.99 | SWTO39 | CCCAAGCTTTGGAGCGAAACCGATGACCCGCCCTATACAGGC | *Hind*III-*dadX*-S gene, forward |
| SEQ ID No.100 | SWTO40 | CCCAAGCTTTTACGTTGTCACAAACGGCAC | *Xho*I-*dadX*-S gene, reverse |
| SEQ ID No.101 | SWTO41 | CCCAAGCTTTGGAGCGAAACCGATGACCCGTCCGATACAGGC | *Hind*III-*dadX*-E gene, forward |
| SEQ ID No.102 | SWTO42 | CCCAAGCTTTTACACCGTCACAACCGGGA | *Xho*I-*dadX*-E gene, reverse |
| SEQ ID No.103 | SWTO43 | CCGCTCGAGATTGGTTCTGTGTTACCAAGTTCTGC | *Xho*I-*cyoA*-S promoter, forward |
| SEQ ID No.104 | SWTO44 | AAAACTGCAGAACATTATTTGCCGTTGTAATAACG | *Pst*I-*cyoA*-S promoter, reverse |
| SEQ ID No.105 | SWTO45 | CCGCTCGAGATCTGAGGGTTGGCTTCCTTGTAAG | *Xho*I-*phoH*-S promoter, forward |
| SEQ ID No.106 | SWTO46 | AAAACTGCAGCCGACATGTCCGATAGCATTATTGC | PstI-phoH-S promoter, reverse |
| SEQ ID No.107 | SWTO47 | CCGCTCGAGTGCAGGTCTCCTGAGAACCACAC | *Xho*I-*lldP*-S promoter, forward |
| SEQ ID No.108 | SWTO48 | AAAACTGCAGTGAAGTAACAACACTCCCGACATC | *Pst*I-*lldP-S* promoter, reverse |
| SEQ ID No.109 | SWTO49 | CCGCTCGAGACATACCCCTCAAGTGGCGTC | *Xho*I-*argT*-S promoter, forward |
| SEQ ID No.110 | SWTO50 | AAAACTGCAGTAGCGTTGTCTGTTTAGCGTGC | *Pst*I-*argT*-S promoter, reverse |

(continued)

| Sequence Number | Code | Sequence | Purpose |
|---|---|---|---|
| SEQ ID No.111 | SWTO51 | CCGCTCGAGAAGCGATGTTAAAAACAGAGCCG | *Xho*I-*ydcI*-S promoter, forward |
| SEQ ID No.112 | SWTO52 | AAAACTGCAGAATGTCATGCCTCCAGTGAATGTTAC | *Pst*I-*ydcI*-S promoter, reverse |
| SEQ ID No.113 | SWTO53 | CCGCTCGAGCCGCGATCTCCATAGTGACT | *Xho*I-*phoH*-E promoter, forward |
| SEQ ID No.114 | SWTO54 | AAACTGCAGTGTTTTTAGGCGGATAAGGC | *Pst*I-*phoH*-E promoter, reverse |
| SEQ ID No.115 | SWTO55 | CCGCTCGAGTGCAGGTCTCCTGGAGTCCA | *Xho*I-*lldP*-E promoter, forward |
| SEQ ID No.116 | SWTO56 | AAACTGCAGGAACACAGTATCTACAGGGTGATTCTG | *Pst*I-*lldP*-E promoter, reverse |
| SEQ ID No.117 | SWTO57 | CCGCTCGAGAAGCGATGTTAAAAACGAAGCG | *Xho*I-*ydcI*-E promoter, forward |
| SEQ ID No.118 | SWTO58 | AAACTGCAGCTGACATTGCCTGCGAAAAC | *Pst*I-*ydcI*-E promoter, reverse |
| SEQ ID No.119 | SWTO59 | GGTACGCAAGATTGGGGTAC | *dadX* knockout detection reverse primer 1 |
| SEQ ID No.120 | SWTO60 | GAAAACGCTCGCCTGTTCTT | *dadX* knockout detection reverse primer 2 |
| SEQ ID No.121 | SWTO61 | TGTCAGCTCTTTACCCAGCG | For strain verification, *dadX* identification primer 1 |
| SEQ ID No.122 | SWTO70 | GCAATACGATGATGAGTAACTCTCCGTCATTCTTTTAACAAGGAATTCAACCGATCATATTCAATAACCCT | Primer for *alr* gene knockout, forward |
| SEQ ID No.123 | SWTO71 | TGCGCGATACAAGCCGGATAAGCGCAAGCGCCACCCGGCCCGCCGCGTATGACTAGTGAACCTCTTCGAGGG | Primer for *alr* gene knockout, reverse |
| SEQ ID No.124 | SWTO72 | CAGGTCTTACGCCGACAGAA | *alr* gene knockout strain verification, forward |
| SEQ ID No.125 | SWTO73 | GTTGCTGAAGAACCGGGTGA | *alr* gene knockout strain verification, reverse |
| SEQ ID No.126 | SWTO75 | CGACGGCGATGCTTAAAGGC | For strain verification, *dadX* identification primer 2 |
| SEQ ID No.127 | SWTO76 | TAAAGTTCTGATGCCGCTGG | *cm-pepT-alr-sodA*, detection reverse |
| SEQ ID No.128 | SWTO77 | CGGTGGTCTTATGGGGTGAA | *cm-pepT-alr-sodA*, detection forward |
| SEQ ID No.129 | SWTO78 | CACCCCCCCGCAACGTTTGCATAGCGCGCATAACTGATAAAGGAAGTGAACAGGAAACAGCTATGAC | Universal primer for *dadX* gene knockout, forward |
| SEQ ID No.130 | SWTO79 | ACGTCGACCCGCCCCCAAGTGGACCGGTCGACGCCTTAGCCTGAATTAGGGTAAAACGACGGCCAGT | Universal primer for *dadX* gene knockout, reverse |

(continued)

| Sequence Number | Code | Sequence | Purpose |
|---|---|---|---|
| SEQ ID No.131 | SWTO82 | ATTTGCGGCCGCCACAATACGGGAACGCAGTT | Forward primer for amplifying *Escherichia coli yhbU* promoter (*Not*I-*yhbU*-E) |
| SEQ ID No.132 | SWTO83 | CCCAAGCTTAATTTTTCCTGAAGGTTGCGC | Reverse primer for amplifying *Escherichia coli yhbU* promoter (*Hind*III-*yhbU*-E) |
| SEQ ID No.133 | SWTO84 | AAACCCGCCAGCTTACACTG | *tdcA-E* promoter detection primer, reverse |
| SEQ ID No.134 | SWTO85 | GCAATACGATGATGAGTAACTCTCCGTCATTCTTTTAACAAGGAATTCAAGAACAGGAAACAGCTATGAC | Universal primer for amplifying hypoxia expression cassette library, with *dadX* as essential gene, for *alr* gene knockout, forward |
| SEQ ID No.135 | SWTO86 | TGCGCGATACAAGCCGGATAAGCGCAAGCGCCACCCGGCCCGCCGCGTATGTAAAACGACGGCCAGT | Universal primer for amplifying hypoxia expression cassette library, with *dadX* as essential gene, for *alr* gene knockout, reverse |
| SEQ ID No.136 | SWTO87 | GCACCTTAGGCTGGACGATG | For strain verification, *dadX* identification primer 3 |
| SEQ ID No.137 | SWTO88 | CGACGCAGAGTTTCAGGATT | *cydA*-E promoter detection primer, reverse |
| SEQ ID No.138 | SWTO89 | CACCCCCCCGCAACGTTTGCATAGCGCGCATAACTGATAAAGGAAGTGAAGAACCTCTTCGAGGGACCTAAT | Amplification of *p1-cm-pepT-alr-sodA-F*, recombination into *dadX* |
| SEQ ID No.139 | SWTO90 | ACGTCGACCCGCCCCCAAGTGGACCGGTCGACGCCTTAGCCTGAATTAGGACGAAAAGTACGGCATTGATAATC | Amplification of *p1-cm-pepT-alr-sodA-R*, recombination into *dadX* |
| SEQ ID No.140 | SWTO93 | TCGCCACTGGACTTTCTGCT | *asd* gene knockout strain verification, forward |
| SEQ ID No.141 | SWTO94 | CTGCGTCGTCCTACCTTCAG | *asd* gene knockout strain verification, reverse |
| SEQ ID No.142 | SWTO95 | AAGAGGTATCCATTCCGACG | *cm-pepT-asd-sodA*, internal detection forward |
| SEQ ID No.143 | SWTO96 | AAGGGAGCATCAGGCGAATA | *focA*-E promoter detection primer, reverse |
| SEQ ID No.144 | SWTO97 | AACTGCGTTCCCGTATTGTG | *yhbU*-E promoter detection primer, reverse |
| SEQ ID No.145 | SWTO98 | TAACCGCTCCCTGGAACAAC | For strain verification, *alr* identification primer 1 |
| SEQ ID No.146 | SWTO99 | GGTAAGCAACGTAACGGTCC | For strain verification, *alr* identification primer 2 |

The tool plasmids of the present invention are shown in Table 8.

Table 8 Tool plasmids used in the present invention

| Name | Description | Source |
|---|---|---|
| pSWT001 | Temperature-inducible recombinase and CRE enzyme system | Construct |
| pSWT002 | Vector containing bilateral co-directional loxP sequences with an intervening chloramphenicol resistance gene, flanked by NotI restriction sites on both sides, used for gene knockout after amplification | Construct |
| pSWT003 | pBlueScript - Cloning backbone vector | Purchase from biofeng |
| pSWT004 | pSWT1 - For YB1-like application, containing the *cm-pepT-asd-sodA* element | Construct |
| pSWT005 | Chloramphenicol gene (*cm*) containing bilateral co-directional loxP sequences, and *pepT-alr-sodA* | Construct |
| pSWT006 | Plasmid carrying a prokaryotic promoter for strong expression of EGFP | Construct |
| pSWT007 | Vector containing bilateral co-directional loxP sequences with an intervening chloramphenicol resistance gene, flanked by SpeI and NotI restriction sites respectively, used for constructing a hypoxia-specific gene expression cassette library | Construct |

The present application has been described above in conjunction with preferred embodiments; however, these embodiments are merely exemplary and serve only for illustrative purposes. On this basis, various substitutions and improvements can be made to the present application, all of which fall within the scope of protection of the present application.

**Claims**

1.    A hypoxia-specific gene expression cassette, comprising:

a) a forward hypoxia promoter, which is a promoter comprising FNR binding site, wherein the forward hypoxia promoter is capable of being induced for expression under hypoxia;
b) a survival-essential gene; and
c) a reverse hyperoxia promoter, which is a promoter comprising FNR and ArcA binding sites, wherein the reverse hyperoxia promoter is capable of functioning under oxygen concentration condition of normal organs;

wherein the survival-essential gene is a gene encoding alanine racemase.

2.    A hypoxia-specific gene expression cassette, comprising:

a) a forward hypoxia promoter, which is a promoter comprising FNR binding site, wherein the forward hypoxia promoter is capable of being induced for expression under hypoxia;
b) a survival-essential gene; and
c) a reverse hyperoxia promoter, which is a promoter comprising two ArcA binding sites, wherein the reverse hyperoxia promoter is capable of functioning under oxygen concentration condition of normal organs;

wherein the survival-essential gene is a gene encoding alanine racemase.

3.    The hypoxia-specific gene expression cassette according to claim 1 or 2, wherein:

the FNR binding site of the forward hypoxia promoter or the reverse hyperoxia promoter conforms to the pattern of TTGATNNNNATCAA, wherein N is any base of A, T, C, and G, and wherein any base in the TTGAT and ATCAA sequences of the conserved binding site can be substituted, provided that the total number of substitutions does not exceed 3, and 3 consecutive adjacent bases cannot be substituted;
the ArcA binding site of the reverse hyperoxia promoter conforms to the pattern of GTTAATTA, wherein any base can be substituted, provided that the total number of substitutions does not exceed 2.

4. The hypoxia-specific gene expression cassette according to any one of the preceding claims, wherein:

   the forward hypoxia promoter is selected from the promoter region sequences of *yhbU*, *ynfK*, *tdcA*, *yecH* or *focA*;
   the survival-essential gene is selected from *alr* or *dadX*;
   the reverse hyperoxia promoter is selected from the promoter region sequences of *cyoA*, *ydcI*, *phoH*, *argT*, *mqo*, or *lldP*.

5. The hypoxia-specific gene expression cassette according to claim 4, wherein the expression cassette consists of the forward hypoxia promoter *yhbU*, the survival-essential gene *alr*, and the reverse hyperoxia promoter *cyoA*.

6. The hypoxia-specific gene expression cassette according to claim 4, wherein the expression cassette consists of the forward hypoxia promoter *yhbU*, the survival-essential gene *alr*, and the reverse hyperoxia promoter *ydcI*.

7. The hypoxia-specific gene expression cassette according to claim 4, wherein the expression cassette consists of the forward hypoxia promoter *yhbU*, the survival-essential gene *alr*, and the reverse hyperoxia promoter *phoH*.

8. The hypoxia-specific gene expression cassette according to claim 4, wherein the expression cassette consists of the forward hypoxia promoter *yhbU*, the survival-essential gene *alr*, and the reverse hyperoxia promoter *argT*.

9. The hypoxia-specific gene expression cassette according to claim 4, wherein the expression cassette consists of the forward hypoxia promoter *yhbU*, the survival-essential gene *alr*, and the reverse hyperoxia promoter *mqo*.

10. The hypoxia-specific gene expression cassette according to claim 4, wherein the expression cassette consists of the forward hypoxia promoter *ynfK*, the survival-essential gene *alr*, and the reverse hyperoxia promoter *cyoA*.

11. The hypoxia-specific gene expression cassette according to claim 4, wherein the expression cassette consists of the forward hypoxia promoter *ynfK*, the survival-essential gene *alr*, and the reverse hyperoxia promoter *phoH*.

12. The hypoxia-specific gene expression cassette according to claim 4, wherein the expression cassette consists of the forward hypoxia promoter *ynfK*, the survival-essential gene *alr*, and the reverse hyperoxia promoter *argT*.

13. The hypoxia-specific gene expression cassette according to claim 4, wherein the expression cassette consists of the forward hypoxia promoter *ynfK*, the survival-essential gene *alr*, and the reverse hyperoxia promoter *ydcI*.

14. The hypoxia-specific gene expression cassette according to claim 4, wherein the expression cassette consists of the forward hypoxia promoter *ynfK*, the survival-essential gene *alr*, and the reverse hyperoxia promoter *mqo*.

15. The hypoxia-specific gene expression cassette according to claim 4, wherein the expression cassette consists of the forward hypoxia promoter *tdcA*, the survival-essential gene *alr*, and the reverse hyperoxia promoter *ydcI*.

16. The hypoxia-specific gene expression cassette according to claim 4, wherein the expression cassette consists of the forward hypoxia promoter *tdcA*, the survival-essential gene *alr*, and the reverse hyperoxia promoter *cyoA*.

17. The hypoxia-specific gene expression cassette according to claim 4, wherein the expression cassette consists of the forward hypoxia promoter *yecH*, the survival-essential gene *alr*, and the reverse hyperoxia promoter *cyoA*.

18. The hypoxia-specific gene expression cassette according to claim 4, wherein the expression cassette consists of the forward hypoxia promoter *yecH*, the survival-essential gene *alr*, and the reverse hyperoxia promoter *lldP*.

19. The hypoxia-specific gene expression cassette according to claim 4, wherein the expression cassette consists of the forward hypoxia promoter *yecH*, the survival-essential gene *alr*, and the reverse hyperoxia promoter *argT*.

20. The hypoxia-specific gene expression cassette according to claim 4, wherein the expression cassette consists of the forward hypoxia promoter *yecH*, the survival-essential gene *alr*, and the reverse hyperoxia promoter *phoH*.

21. The hypoxia-specific gene expression cassette according to claim 4, wherein the expression cassette consists of the forward hypoxia promoter *yecH*, the survival-essential gene *alr*, and the reverse hyperoxia promoter *mqo*.

22. The hypoxia-specific gene expression cassette according to claim 4, wherein the expression cassette consists of the forward hypoxia promoter *focA*, the survival-essential gene *alr*, and the reverse hyperoxia promoter *lldP*.

23. The hypoxia-specific gene expression cassette according to claim 4, wherein the expression cassette consists of the forward hypoxia promoter *yhbU*, the survival-essential gene *dadX*, and the reverse hyperoxia promoter *cyoA*.

24. The hypoxia-specific gene expression cassette according to claim 4, wherein the expression cassette consists of the forward hypoxia promoter *yhbU*, the survival-essential gene *dadX*, and the reverse hyperoxia promoter *ydcI*.

25. The hypoxia-specific gene expression cassette according to claim 4, wherein the expression cassette consists of the forward hypoxia promoter *yhbU*, the survival-essential gene *dadX*, and the reverse hyperoxia promoter *phoH*.

26. The hypoxia-specific gene expression cassette according to claim 4, wherein the expression cassette consists of the forward hypoxia promoter *yhbU*, the survival-essential gene *dadX*, and the reverse hyperoxia promoter *argT*.

27. The hypoxia-specific gene expression cassette according to claim 4, wherein the expression cassette consists of the forward hypoxia promoter *yhbU*, the survival-essential gene *dadX*, and the reverse hyperoxia promoter *mqo*.

28. The hypoxia-specific gene expression cassette according to claim 4, wherein the expression cassette consists of the forward hypoxia promoter *ynfK*, the survival-essential gene *dadX*, and the reverse hyperoxia promoter *cyoA*.

29. The hypoxia-specific gene expression cassette according to claim 4, wherein the expression cassette consists of the forward hypoxia promoter *ynfK*, the survival-essential gene *dadX*, and the reverse hyperoxia promoter *phoH*.

30. The hypoxia-specific gene expression cassette according to claim 4, wherein the expression cassette consists of the forward hypoxia promoter *ynfK*, the survival-essential gene *dadX*, and the reverse hyperoxia promoter *argT*.

31. The hypoxia-specific gene expression cassette according to claim 4, wherein the expression cassette consists of the forward hypoxia promoter *ynfK*, the survival-essential gene *dadX*, and the reverse hyperoxia promoter *ydcI*.

32. The hypoxia-specific gene expression cassette according to claim 4, wherein the expression cassette consists of the forward hypoxia promoter *ynfK*, the survival-essential gene *dadX*, and the reverse hyperoxia promoter *mqo*.

33. The hypoxia-specific gene expression cassette according to claim 4, wherein the expression cassette consists of the forward hypoxia promoter *tdcA*, the survival-essential gene *dadX*, and the reverse hyperoxia promoter *ydcI*.

34. The hypoxia-specific gene expression cassette according to claim 4, wherein the expression cassette consists of the forward hypoxia promoter *tdcA*, the survival-essential gene *dadX*, and the reverse hyperoxia promoter *cyoA*.

35. The hypoxia-specific gene expression cassette according to claim 4, wherein the expression cassette consists of the forward hypoxia promoter *yecH*, the survival-essential gene *dadX*, and the reverse hyperoxia promoter *cyoA*.

36. The hypoxia-specific gene expression cassette according to claim 4, wherein the expression cassette consists of the forward hypoxia promoter *yecH*, the survival-essential gene *dadX*, and the reverse hyperoxia promoter *lldP*.

37. The hypoxia-specific gene expression cassette according to claim 4, wherein the expression cassette consists of the forward hypoxia promoter *yecH*, the survival-essential gene *dadX*, and the reverse hyperoxia promoter *argT*.

38. The hypoxia-specific gene expression cassette according to claim 4, wherein the expression cassette consists of the forward hypoxia promoter *yecH*, the survival-essential gene *dadX*, and the reverse hyperoxia promoter *phoH*.

39. The hypoxia-specific gene expression cassette according to claim 4, wherein the expression cassette consists of the forward hypoxia promoter *yecH*, the survival-essential gene *dadX*, and the reverse hyperoxia promoter *mqo*.

40. The hypoxia-specific gene expression cassette according to claim 4, wherein the expression cassette consists of the forward hypoxia promoter *focA*, the survival-essential gene *dadX*, and the reverse hyperoxia promoter *lldP*.

41. The hypoxia-specific gene expression cassette according to any one of the preceding claims, wherein the forward hypoxia promoter and/or the reverse hyperoxia promoter are promoters derived from Gram-negative bacteria.

42. The hypoxia-specific gene expression cassette according to claim 41, wherein the Gram-negative bacteria are selected from the group consisting of *Salmonella*, *Escherichia coli*, *Shigella*, *Yersinia*, *Enterobacter cloacae*, *Cronobacter*, *Klebsiella*, *Pantoea*, *Serratia*, *Shimwellia*, *Enterobacter ludwigii*, *Haemophilus*, *Vibrio*, *Pseudomonas*, *Pasteurella*, *Bordetella*, *Bordetella pertussis*, *Acinetobacter baumannii*, *Burkholderia*, *Vibrio vulnificus*, *Bacteroides fragilis*, *Pseudomonas syringae*, *Pseudomonas putida*, *Legionella*, *Klebsiella pneumoniae*, *Vibrio parahaemolyticus*, *Vibrio cholerae*, *Yersinia pestis*, *Catarrhalis coccus*, *Moraxella catarrhalis*, *Campylobacter jejuni*, *Shigella dysenteriae*, *Neisseria gonorrhoeae*, *Haemophilus influenzae*, *Moraxella*, *Neisseria meningitidis*, *Proteus vulgaris*, *Proteus mirabilis*, *Pasteurella haemolytica*, *Salmonella enterica*, *Salmonella bongori*, *Salmonella paratyphi*, *Salmonella typhi*, *Legionella pneumophila*, *Yersinia pestis*, *Shigella sonnei*, *Pseudomonas aeruginosa*, *Yersinia enterocolitica*, *Cryptococcus neoformans*, *Burkholderia cenocepacia*, and *Helicobacter pylori*.

43. The hypoxia-specific gene expression cassette according to any one of the preceding claims, wherein the forward hypoxia promoter is *yhbU*, which is selected from the group consisting of *Salmonella*, *Escherichia coli*, *Shigella*, *Yersinia*, *Enterobacter cloacae*, *Cronobacter*, *Klebsiella*, *Pantoea*, *Serratia*, and *Shimwellia*.

44. The hypoxia-specific gene expression cassette according to any one of the preceding claims, wherein the forward hypoxia promoter is *ynfK*, which is selected from the group consisting of *Salmonella*, *Escherichia coli*, *Serratia*, *Shigella*, and *Enterobacter ludwigii*.

45. The hypoxia-specific gene expression cassette according to any one of the preceding claims, wherein the alanine racemase gene *alr* and/or *dadX* is selected from the group consisting of *Salmonella, Escherichia coli*, *Shigella*, *Klebsiella*, *Yersinia*, *Haemophilus*, and *Pseudomonas*.

46. The hypoxia-specific gene expression cassette according to any one of the preceding claims, which is regulated by oxygen concentration.

47. The hypoxia-specific gene expression cassette according to claim 46, wherein the forward hypoxia promoter functions when the oxygen concentration is lower than 1%, and does not function when the oxygen concentration is higher than 1%; and/or the reverse hyperoxia promoter functions when the oxygen concentration is higher than 1%, and does not function when the oxygen concentration is lower than 1%.

48. The hypoxia-specific gene expression cassette according to claim 47, wherein the forward hypoxia promoter functions when the oxygen concentration is lower than 0.8%, and does not function when the oxygen concentration is higher than 0.8%; and/or the reverse hyperoxia promoter functions when the oxygen concentration is higher than 0.8%, and does not function when the oxygen concentration is lower than 0.8%.

49. The hypoxia-specific gene expression cassette according to any one of the preceding claims, which is integrated into the chromosome of host bacterium.

50. A modified Gram-negative bacterium, which is modified by the hypoxia-specific gene expression cassette according to any one of the preceding claims.

51. The Gram-negative bacterium according to claim 50, which is *Salmonella*, *Escherichia coli*, *Shigella*, *Yersinia*, *Enterobacter cloacae*, *Cronobacter*, *Klebsiella*, *Pantoea*, *Serratia*, *Shimwellia*, *Enterobacter ludwigii*, *Haemophilus*, *Vibrio*, *Pseudomonas*, *Pasteurella*, *Bordetella*, *Bordetella pertussis*, *Acinetobacter baumannii*, *Burkholderia*, *Vibrio vulnificus*, *Bacteroides fragilis*, *Pseudomonas syringae*, *Pseudomonas putida*, *Legionella*, *Klebsiella pneumoniae*, *Vibrio parahaemolyticus*, *Vibrio cholerae*, *Yersinia pestis*, *Catarrhalis coccus*, *Moraxella catarrhalis*, *Campylobacter jejuni*, *Shigella dysenteriae*, *Neisseria gonorrhoeae*, *Haemophilus influenzae*, *Moraxella*, *Neisseria meningitidis*, *Proteus vulgaris*, *Proteus mirabilis*, *Pasteurella haemolytica*, *Salmonella enterica*, *Salmonella bongori*, *Salmonella paratyphi*, *Salmonella typhi*, *Legionella pneumophila*, *Yersinia pestis*, *Shigella sonnei*, *Pseudomonas aeruginosa*, *Yersinia enterocolitica*, *Cryptococcus neoformans*, *Burkholderia cenocepacia*, or *Helicobacter pylori*.

52. The Gram-negative bacterium according to claim 51, which is *Salmonella*.

53. A pharmaceutical composition, comprising the hypoxia-specific gene expression cassette according to any one of claims 1-49 or the Gram-negative bacterium according to any one of claims 50-52.

**54.** The pharmaceutical composition according to claim 53, further comprising a pharmaceutically acceptable carrier.

**55.** The pharmaceutical composition according to claim 54, wherein the pharmaceutically acceptable carrier is selected from the group consisting of disintegrants, binders, fillers, buffers, tonicity agents, stabilizers, antioxidants, surfactants, and lubricants.

**56.** The pharmaceutical composition according to any one of claims 53-55, which is used for treating solid tumors.

**57.** Use of the hypoxia-specific gene expression cassette according to any one of claims 1-49, the Gram-negative bacterium according to any one of claims 50-52, or the pharmaceutical composition according to any one of claims 53-56 in the preparation of a medicament for treating tumor.

**58.** The use according to claim 57, wherein the tumor is a solid tumor.

**59.** The pharmaceutical composition according to claim 56 or the use according to claim 58, wherein the solid tumor is selected from the group consisting of tumors/cancers of the breast, bone, liver, lung, skin, kidney, stomach, pancreas, prostate, lymph (non-Hodgkin's lymphoma, Hodgkin's lymphoma), intestines (colon cancer, rectal cancer), pelvis (cervical cancer, ovarian malignancy, endometrial cancer, ovarian cancer), nervous system, head and neck, and bladder, etc.

**60.** The pharmaceutical composition according to claim 56 or the use according to claim 58, wherein the solid tumor is selected from the group consisting of breast cancer, osteosarcoma, liver cancer, lung cancer, melanoma, kidney cancer, stomach cancer, pancreatic cancer, prostate cancer, colon cancer, ovarian cancer, neuroblastoma, squamous cell carcinoma, and bladder cancer.

**61.** A method of treating tumor, comprising administering to a subject the hypoxia-specific gene expression cassette according to any one of claims 1-49, the Gram-negative bacterium according to any one of claims 50-52, or the pharmaceutical composition according to any one of claims 53-56.

**62.** The method according to claim 61, wherein the tumor is a solid tumor.

**63.** The method according to claim 62, wherein the solid tumor is selected from the group consisting of tumors/cancers of the breast, bone, liver, lung, skin, kidney, stomach, pancreas, prostate, lymph (non-Hodgkin's lymphoma, Hodgkin's lymphoma), intestines (colon cancer, rectal cancer), pelvis (cervical cancer, ovarian malignancy, endometrial cancer, ovarian cancer), nervous system, head and neck, and bladder, etc.

**64.** The method according to claim 63, wherein the solid tumor is selected from the group consisting of breast cancer, osteosarcoma, liver cancer, lung cancer, melanoma, kidney cancer, stomach cancer, pancreatic cancer, prostate cancer, colon cancer, ovarian cancer, neuroblastoma, squamous cell carcinoma, and bladder cancer.

Figure 1

Figure 2

Figure 3

Figure 4

Figure 5

Figure 6

Figure 7

Figure 8

Figure 9

Figure 10

Figure 11

(A) O₂-     (B) O₂+

Figure 12

1000bp

Figure 13

1000bp

Figure 14

(A) O₂-     (B) O₂+

Figure 15

Figure 16

Figure 17

Figure 18

Figure 19

Figure 20

Figure 21

Figure 22

Figure 23

Figure 24

Figure 25

Figure 26

(A)  O₂-    (B)  O₂+

Figure 27

(A)  O₂-    (B)  O₂+

Figure 28

(A)  O₂-    (B)  O₂+

Figure 29

(A)  O₂-    (B)  O₂+

Figure 30

(A) O₂⁻    (B) O₂⁺

Figure 31

(A)    (B)

(C)    (D)

Figure 32

(A)    (B)

*Salmonella* antibody    HIF1α antibody

Figure 33

## Invasion effect of SWT1001 on cancer cells

Figure 34

## Invasion effect of SWT1001 on cancer cells

Figure 35

## Invasion effect of SWT1002 on cancer cells

Figure 36

## Invasion effect of SWT1002 on cancer cells

Figure 37

## Invasion effect of SWT1004 on cancer cells

Figure 38

## Invasion effect of SWT1004 on cancer cells

Figure 39

## Invasion effect of SWT1005 on cancer cells

Figure 40

## Invasion effect of SWT1005 on cancer cells

Figure 41

## Invasion effect of SWT1009 on cancer cells

Figure 42

**Invasion effect of SWT1009 on cancer cells**

Figure 43

**Invasion effect of SWT1010 on cancer cells**

Figure 44

**Invasion effect of SWT1010 on cancer cells**

Figure 45

## Invasion effect of SWT1012 on cancer cells

Figure 46

## Invasion effect of SWT1012 on cancer cells

Figure 47

## Invasion effect of SWT1013 on cancer cells

Figure 48

**Invasion effect of SWT1013 on cancer cells**

Figure 49

**Invasion effect of SWT1021 on cancer cells**

Figure 50

**Invasion effect of SWT1021 on cancer cells**

Figure 51

**Invasion effect of SWT1025 on cancer cells**

Figure 52

**Invasion effect of SWT1025 on cancer cells**

Figure 53

**Invasion effect of SWT1033 on cancer cells**

Figure 54

**Invasion effect of SWT1033 on cancer cells**

Figure 55

**Invasion effect of SWT1035 on cancer cells**

Figure 56

**Invasion effect of SWT1035 on cancer cells**

Figure 57

Invasion effect of SWT1036 on cancer cells

Figure 58

Invasion effect of SWT1036 on cancer cells

Figure 59

Invasion effect of SWT1038 on cancer cells

Figure 60

## Invasion effect of SWT1038 on cancer cells

Figure 61

## Invasion effect of SWT1063 on cancer cells

Figure 62

## Invasion effect of SWT1063 on cancer cells

Figure 63

(A)

Figure 64

(A)

(B)

Figure 65

Figure 66

Figure 67

Figure 68

Figure 69

(A)

(B)

Figure 70

Figure 71

SWT005

Figure 72

Figure 73

Figure 74

(A)

(C)

(B)

(D)

Figure 75

(A)

(C)

(B)

Figure 76

Figure 77

Figure 78

(A) **Inhibitory effect of SWT1021 on tumors**

(C) **Inhibitory effect of SWT1033 on tumors**

(B) **Inhibitory effect of SWT1025 on tumors**

(D) **Inhibitory effect of SWT1035 on tumors**

Figure 79

(A) **Inhibitory effect of SWT1036 on tumors**

(C) **Inhibitory effect of SWT1063 on tumors**

(B) **Inhibitory effect of SWT1038 on tumors**

Figure 80

Figure 81

Figure 82

Figure 83

Figure 84

Figure 85

Figure 86

Figure 87

Figure 88

Figure 89

Figure 90

## INTERNATIONAL SEARCH REPORT

| International application No. |
|---|
| **PCT/CN2024/117998** |

| | |
|---|---|
| **A.** | **CLASSIFICATION OF SUBJECT MATTER** |

C12N 15/74(2006.01)i; C12N 15/61(2006.01)i; C12N 15/113(2010.01)i; C12N 1/21(2006.01)i; A61K 48/00(2006.01)i; A61K 35/74(2015.01)i; A61P 35/00(2006.01)i

According to International Patent Classification (IPC) or to both national classification and IPC

| | |
|---|---|
| **B.** | **FIELDS SEARCHED** |

Minimum documentation searched (classification system followed by classification symbols)

IPC:C12N,A61K,A61P

Documentation searched other than minimum documentation to the extent that such documents are included in the fields searched

Electronic data base consulted during the international search (name of data base and, where practicable, search terms used)

CJFD, CNTXT, ENTXT, ENTXTC, PUBMED, ISI Web of Knowledge, Elsevier Science Direct, Wiley InterScience, Nature, Science, CNKI, 万方, Wanfang, 读秀, Duxiu, 中国专利生物序列库, China Patent Biological Sequence Library: (alr or dadx or 丙氨酸消旋酶) and (yhbU or ynfK or tdcA or yecH or focA) and (cyoA or ydcI or phoH or argT or mqo or lldP), biosynthe ticalanineracemase, catabolicalanineracemase, Alanineracemase, alr, dadx/b, cassette, anaerobic, aerobic, 表达盒, 肿瘤, 启动子, arcA, aeobic respiratory control, 有氧呼吸控制转录调控因子, FNR, fumarate nitrate reduction regulator, 富马酸盐硝酸盐还原转录调控因子

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | CN 104471057 A (THE UNIVERSITY OF HONG KONG) 25 March 2015 (2015-03-25) claims 1-58, description, paragraphs 12-21 and 84-87, and figures 8A-C | 1-3, 41-64 |
| X | CN 114438111 A (SHENZHEN INSTITUTES OF ADVANCED TECHNOLOGY) 06 May 2022 (2022-05-06) claims 1-10, and description, paragraphs 13-36 and 51-74 | 1-3, 41-64 |
| A | US 2019160115 A1 (SYNLOGIC, INC.) 30 May 2019 (2019-05-30) claims 1-63 | 1-64 |
| A | WO 2008025744 A1 (NOVO NORDISK AS) 06 March 2008 (2008-03-06) description, page 7, line 20 to page 10, line 10 | 1-64 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **16 December 2024** | **23 December 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/117998** |

**C. DOCUMENTS CONSIDERED TO BE RELEVANT**

| Category* | Citation of document, with indication, where appropriate, of the relevant passages | Relevant to claim No. |
| --- | --- | --- |
| A | 杨金茹等 (YANG, Jinru et al.). "抗菌药物靶标丙氨酸消旋酶及其抑制剂研究进展 (Research Progress of Antibacterial Drug Target Alanine Racemase and its Inhibitors)" 微生物学通报 (*Microbiology China*), Vol. 48, No. (12), 20 December 2021 (2021-12-20), 4894-4903 abstract, sections 1.1-1.2 and 4.4 | 1-64 |
| A | SAWERS, G. et al. "Anaerobic Induction of Pyruvate Formate-Lyase Gene Expression is Mediated by the ArcA and FNR Proteins" *Journal of Bacteriology,* Vol. 174, No. (11), 30 June 1992 (1992-06-30), pp. 3474-3477 | 1-64 |

Form PCT/ISA/210 (second sheet) (July 2022)

**INTERNATIONAL SEARCH REPORT**

| International application No. |
|---|
| **PCT/CN2024/117998** |

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

International application No.

**PCT/CN2024/117998**

| Box No. II    Observations where certain claims were found unsearchable (Continuation of item 2 of first sheet) |
|---|

This international search report has not been established in respect of certain claims under Article 17(2)(a) for the following reasons:

1. ☑ Claims Nos.: **61-64**
   because they relate to subject matter not required to be searched by this Authority, namely:

   Claims 61-64 relate to a tumor treatment method, which falls within disease treatment methods as defined in PCT Rule 39.1(iv). However, a search is still carried out on the basis of the pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2024/117998**

| Patent document cited in search report | | | Publication date (day/month/year) | Patent family member(s) | | | Publication date (day/month/year) |
|---|---|---|---|---|---|---|---|
| CN | 104471057 | A | 25 March 2015 | US | 2013295054 | A1 | 07 November 2013 |
| | | | | US | 9127284 | B2 | 08 September 2015 |
| | | | | EP | 2844736 | A1 | 11 March 2015 |
| | | | | EP | 2844736 | A4 | 11 November 2015 |
| | | | | EP | 2844736 | B1 | 01 March 2017 |
| | | | | WO | 2013163893 | A1 | 07 November 2013 |
| CN | 114438111 | A | 06 May 2022 | | None | | |
| US | 2019160115 | A1 | 30 May 2019 | CA | 3011283 | A1 | 20 July 2017 |
| | | | | WO | 2017123675 | A1 | 20 July 2017 |
| | | | | WO | 2017123675 | A8 | 24 August 2017 |
| | | | | US | 11723932 | B2 | 15 August 2023 |
| | | | | EP | 3402498 | A1 | 21 November 2018 |
| WO | 2008025744 | A1 | 06 March 2008 | | None | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023112106010
- CN 104471057 B